# EUROPÄISCHE PATENTSCHRIFT

(11) **EP 0 854 852 B1**
(45) Veröffentlichungstag und Bekanntmachung des Hinweises auf die Patenterteilung: **17.10.2001**
(21) Anmeldenummer: 96934507.3
(22) Anmeldetag: 01.10.1996
(51) Int. Cl.: C07C 49/747, C07C 49/697, C07C 49/753, C07C 69/013, C07C 69/96, C07C 309/65, C07C 329/06, A01N 35/06, A01N 37/02, C07C 69/757

(54) **CYCLOPENTAN-1,3-DION-DERIVATE**
CYCLOPENTANE-1,3-DIONE DERIVATIVES
DERIVES DE CYCLOPENTANE-1,3-DIONE

(30) Priorität: 13.10.1995 DE 19538218
(43) Veröffentlichungstag der Anmeldung: 29.07.1998
(73) Patentinhaber: BAYER AG, 51368 Leverkusen (DE)
(72) Erfinder: FISCHER, Reiner, D-40789 Monheim (DE); RUTHER, Michael, D-40789 Monheim (DE); GRAFF, Alan, D-51427 Bergisch Gladbach (DE); WIDDIG, Arno, D-51519 Odenthal (DE); DUMAS, Jacques, D-51061 Köln (DE); ERDELEN, Christoph, D-42799 Leichlingen (DE); DAHMEN, Peter, D-41470 Neuss (DE); DOLLINGER, Markus, D-51381 Leverkusen (DE); WACHENDORFF-NEUMANN, Ulrike, D-56566 Neuwied (DE)
(86) Internationale Anmeldenummer: EP9604283
(87) Internationale Veröffentlichungsnummer: WO9714667

(56) Entgegenhaltungen:
- WO-A-96/01798
- WO-A-96/03366
- US-A- 4 283 348
- US-A- 4 551 547

## Beschreibung

Die vorliegende Erfindung betrifft neue Cyclopentan-1,3-dion-Derivate, Verfahren zu ihrer Herstellung und ihre Verwendung als Herbizide und Schädlingsbekämpfungsmittel.

Es ist bekannt, daß bestimmte substituierte 2-Arylcyclopentandione, wie z.B. 2-(2',4'-Dimethylphenyl)-4,5,6,7,8,9-hexahydro-1,3-indandion, herbizide und akarizide Eigenschaften besitzen (vgl. z.B. US 4 283 348; 4 338 122; 4 436 666; 4 526 723; 4 551 547 und 4 626 698). Außerdem ist 2-(2,4,6-Trimethylphenyl)-1,3-indandion aus der Publikation J. Economic Entomology, 66, (1973), 584 und der Offenlegungsschrift DE 2 361 084 bekannt, mit Angabe von herbiziden und akariziden Wirkungen.

Ferner werden 2-Aryl-cyclopentan-1,3-dion-Derivate als Herbizide und Schädlingsbekämpfungsmittel in der WO 96/01 798 und WO 96/03 366 beschrieben.

Die Wirksamkeit dieser bekannten Verbindungen ist jedoch, insbesondere bei niedrigen Aufwandmengen und -konzentrationen, nicht in allen Anwendungsgebieten völlig zufriedenstellend. Weiterhin ist die Pflanzenverträglichkeit der bekannten Verbindungen für Kulturpflanzen nicht immer ausreichend.

Es wurden nun neue bicyclische Cyclopentan-1,3-dion-Derivate der Formel (I) 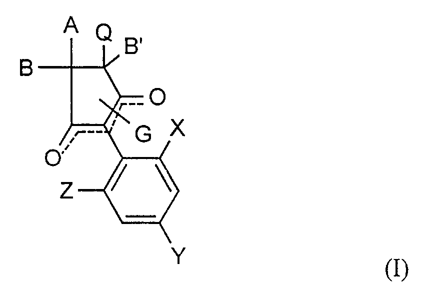 gefunden,
in welcher
- X: für Halogen, C₁-C₆-Alkyl, C₂-C₆-Alkenyl, C₁-C₆-Alkoxy, C₂-C₆-Alkenyloxy, C₁-C₆-Alkylthio, C₁-C₆-Alkylsulfinyl, C₁-C₆-Alkylsulfonyl, C₁-C₆-Halogenalkyl, C₁-C₆-Halogenalkoxy, C₂-C₆-Halogenalkenyloxy, Nitro, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Benzyloxy oder Benzylthio steht,
- Y: für Wasserstoff, Halogen, C₁-C₆-Alkyl, C₂-C₆-Alkenyl, C₁-C₆-Alkoxy, C₂-C₆-Alkenyloxy, C₁-C₆-Alkylthio, C₁-C₆-Alkylsulfinyl, C₁-C₆-Alkylsulfonyl, C₁-C₆-Halogenalkyl, C₁-C₆-Halogenalkoxy, C₂-C₆-Halogenalkenyloxy, Nitro oder Cyano steht,
- Z: für Halogen, C₁-C₆-Alkyl, C₂-C₆-Alkenyl, C₁-C₆-Alkoxy, C₂-C₆-Alkenyloxy, C₁-C₆-Halogenalkyl, C₁-C₆-Halogenalkoxy, C₂-C₆-Halogenalkenyloxy, Nitro oder Cyano steht,
wobei X, Y und Z nicht gleichzeitig für Methyl stehen,
- A und Q: gemeinsam für jeweils gegebenenfalls einfach bis dreifach, gleich oder verschieden durch Halogen, Hydroxy, Mercapto, durch jeweils gegebenenfalls einfach bis neunfach, gleich oder verschieden durch Halogen substituiertes C₁-C₁₀-Alkyl, C₁-C₆-Alkoxy, C₁-C₆-Alkylthio, C₃-C₇-Cycloalkyl oder durch jeweils gegebenenfalls einfach bis fünffach, gleich oder verschieden durch Halogen, C₁-C₆-Alkyl oder C₁-C₆-Alkoxy substituiertes Benzyloxy oder Phenyl substituiertes C₁-C₆-Alkandiyl oder C₂-C₆-Alkendiyl stehen, welches außerdem gegebenenfalls eine der nachstehenden Gruppen 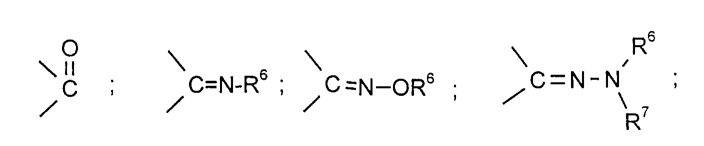 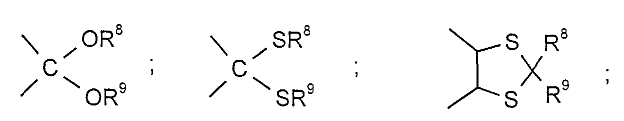 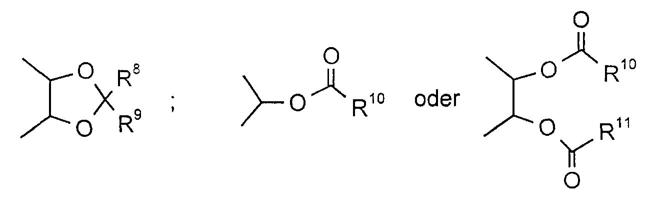 enthält oder durch eine C₁-C₂-Alkandiylgruppe überbrückt ist,
- B und B': unabhängig voneinander bevorzugt für Wasserstoff, Halogen oder C₁-C₆-Alkyl oder gemeinsam für jeweils gegebenenfalls durch C₁-C₆-Alkyl substituiertes C₁-C₆-Alkandiyl oder C₂-C₄-Alkendiyl stehen,
- G: für Wasserstoff (a) oder für eine der Gruppen 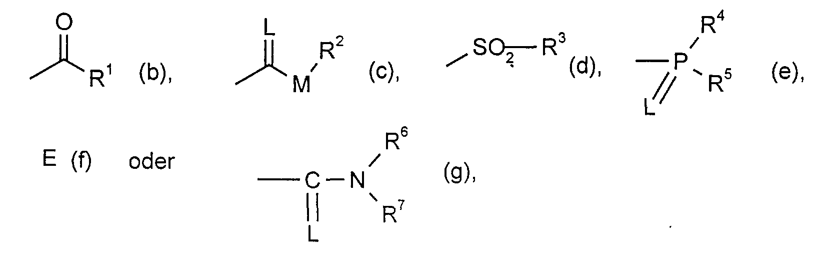 steht, in welchen
- E: für ein Metallion oder ein Ammoniumion steht,
- L: für Sauerstoff oder Schwefel steht und
- M: für Sauerstoff oder Schwefel steht,
- R¹: für jeweils gegebenenfalls einfach oder mehrfach, gleich oder verschieden durch Halogen substituiertes C₁-C₂₀-Alkyl, C₂-C₁₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder für gegebenenfalls einfach oder mehrfach, gleich oder verschieden durch Halogen, C₁-C₆-Alkyl oder C₁-C₆-Alkoxy substituiertes C₃-C₈-Cycloalkyl, in welchem mindestens eine Methylengruppe durch ein Sauerstoff- und/oder Schwefelatom ersetzt sein kann,
für gegebenenfalls einfach oder mehrfach, gleich oder verschieden durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl,
für gegebenenfalls einfach oder mehrfach, gleich oder verschieden durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls einfach oder mehrfach, gleich oder verschieden durch Halogen oder C₁-C₆-Alkyl substituiertes Hetaryl mit 5 oder 6 Ringatomen und ein bis drei Heteroatomen aus der Reihe Sauerstoff, Schwefel und Stickstoff,
für gegebenenfalls einfach oder mehrfach, gleich oder verschieden durch Halogen oder C₁-C₆-Alkyl substituiertes Phenoxy-C₁-C₆-alkyl oder
für gegebenenfalls einfach oder mehrfach, gleich oder verschieden durch Halogen, Amino oder C₁-C₆-Alkyl substituiertes Hetaryloxy-C₁-C₆-alkyl mit 5 oder 6 Ringatomen und ein bis drei Heteroatomen aus der Reihe Sauerstoff, Schwefel und Stickstoff steht,
- R²: für jeweils gegebenenfalls einfach oder mehrfach, gleich oder verschieden durch Halogen substituiertes C₁-C₂₀-Alkyl, C₂-C₁₀-Alkenyl, C₁-C₈-Alkoxy-C₂-C₈-alkyl oder Poly-C₁-C₈-alkoxy-C₂-C₈-alkyl,
für gegebenenfalls einfach oder mehrfach, gleich oder verschieden durch Halogen, C₁-C₆-Alkyl oder C₁-C₆-Alkoxy substituiertes C₃-C₆-Cycloalkyl oder
für jeweils gegebenenfalls einfach oder mehrfach, gleich oder verschieden durch Halogen, Nitro, Cyano, C₁-C₆-Alkyl, C₁-C₆-Alkoxy, C₁-C₃-Halogenalkoxy oder C₁-C₃-Halogenalkyl substituiertes Phenyl oder Benzyl steht,
- R³: für jeweils gegebenenfalls einfach oder mehrfach, gleich oder verschieden durch Halogen substituiertes C₁-C₁₂-Alkyl oder für jeweils gegebenenfalls einfach oder mehrfach, gleich oder verschieden durch Halogen, C₁-C₆-Alkyl, C₁-C₆-Alkoxy, C₁-C₃-Halogenalkyl, C₁-C₃-Halogenalkoxy, Cyano oder Nitro substituiertes Phenyl oder Phenyl-C₁-C₄-alkyl steht,
- R⁴ und R⁵: unabhängig voneinander für jeweils gegebenenfalls einfach oder mehrfach, gleich oder verschieden durch Halogen substituiertes C₁-C₈-Alkyl, C₁-C₈-Alkoxy, C₁-C₈-Alkylamino, Di-(C₁-C₈-alkyl)-amino, C₁-C₈-Alkylthio, C₃-C₅-Alkenylthio, C₃-C₇-Cycloalkylthio oder für jeweils gegebenenfalls einfach oder mehrfach, gleich oder verschieden durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
- R⁶: für Wasserstoff, jeweils gegebenenfalls einfach oder mehrfach, gleich oder verschieden durch Halogen substituiertes C₁-C₁₀-Alkyl, C₃-C₈-Alkenyl, C₁-C₈-Alkoxy-C₂-C₈-alkyl, für gegebenenfalls einfach oder mehrfach, gleich oder verschieden durch Halogen, C₁-C₆-Alkyl, C₁-C₆-Alkoxy, C₁-C₃-Halogenalkyl oder C₁-C₃-Halogenalkoxy substituiertes C₃-C₁₀-Cycloalkyl, für gegebenenfalls einfach oder mehrfach, gleich oder verschieden durch Halogen, C₁-C₃-Halogenalkyl, C₁-C₈-Alkyl, C₁-C₃-Halogenalkoxy oder C₁-C₃-Alkoxy substituiertes Phenyl oder für gegebenenfalls einfach oder mehrfach, gleich oder verschieden durch Halogen, C₁-C₈-Alkyl, C₁-C₃-Halogenalkyl, C₁-C₃-Halogenalkoxy oder C₁-C₈-Alkoxy substituiertes Benzyl steht,
- R⁷: für Wasserstoff oder für jeweils gegebenenfalls einfach oder mehrfach, gleich oder verschieden durch Halogen substituiertes C₁-C₁₀-Alkyl oder C₃-C₁₀-Alkenyl steht oder
- R⁶ und R⁷: gemeinsam mit dem N-Atom, an das sie gebunden sind, für einen gegebenenfalls Sauerstoff oder Schwefel enthaltenden und gegebenenfalls durch C₁-C₆-Alkyl substituierten 3- bis 7-gliedrigen Ring stehen,
- R⁸ und R⁹: unabhängig voneinander für Wasserstoff, gegebenenfalls einfach oder mehrfach, gleich oder verschieden durch Halogen substituiertes C₁-C₆-Alkyl oder für jeweils gegebenenfalls einfach oder mehrfach, gleich oder verschieden durch Halogen, C₁-C₆-Alkyl, C₁-C₆-Alkoxy, C₁-C₆-Halogenalkyl, C₁-C₆-Halogenalkoxy, Nitro oder Cyano substituiertes Phenyl oder Phenyl-C₁-C₄-alkyl, oder zusammen für gegebenenfalls einfach oder mehrfach, gleich oder verschieden durch Halogen, C₁-C₆-Alkyl, C₁-C₆-Alkoxy oder C₁-C₃-Halogenalkyl substituiertes C₂-C₆-Alkandiyl stehen und
- R¹⁰ und R¹¹: unabhängig voneinander für jeweils gegebenenfalls einfach oder mehrfach, gleich oder verschieden durch Halogen substituiertes C₁-C₁₀-Alkyl, C₂-C₁₀-Alkenyl, C₁-C₁₀-Alkoxy, C₁-C₁₀-Alkylamino, Di-(C₁-C₁₀-alkyl)-amino, C₃-C₁₀-Alkenylamino, Di-(C₃-C₁₀-alkenyl)-amino oder jeweils einfach oder mehrfach, gleich oder verschieden durch Halogen, C₁-C₄-Alkyl, C₁-C₄-Alkoxy, C₁-C₄-Halogenalkyl, Nitro oder Cyano substituiertes Phenyl oder Benzyl stehen.

Die Verbindungen der Formel (I) können in Abhängigkeit von der Stellung des Substituenten G in den zwei isomeren Formen der Formeln (I-A) bzw. (I-B) vorliegen, was durch die gestrichelte Linie in der Formel (I) zum Ausdruck gebracht werden soll:

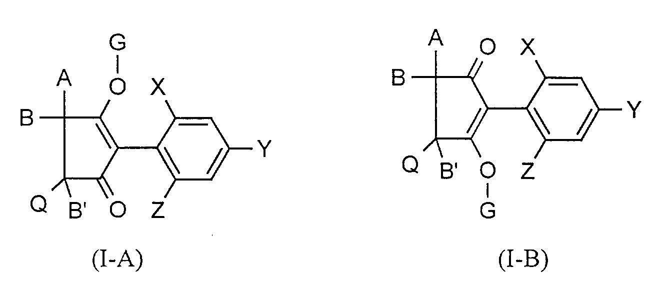

Die Verbindungen der Formeln (I-A) bzw. (I-B) können sowohl als Gemische als auch in Form ihrer reinen Isomeren vorliegen. Gemische der Verbindungen der Formeln (I-A) und (I-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt ein, daß die betreffende Verbindung gegebenenfalls als Isomerengemisch oder in der jeweils anderen isomeren Form vorliegen kann.

Unter Einbeziehung der verschiedenen Bedeutungen (a), (b), (c), (d), (e), (f) und (g) der Gruppe G ergeben sich folgende hauptsächlichen Strukturen (Ia) bis (Ig): 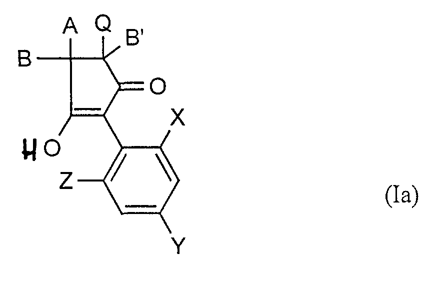 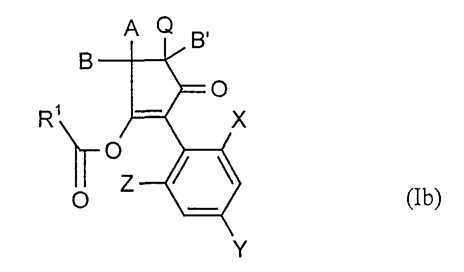 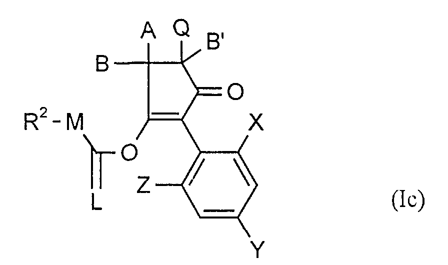 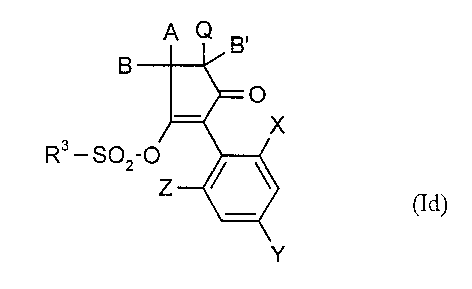 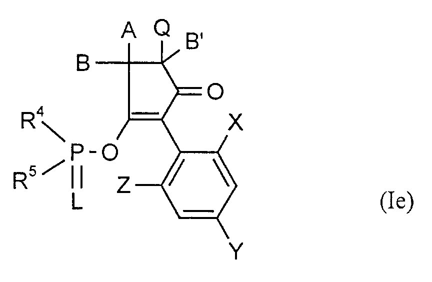 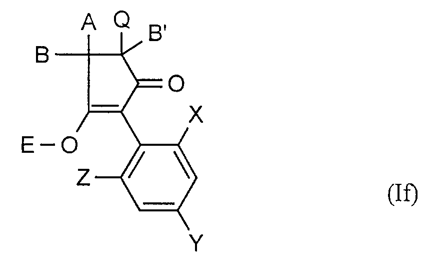 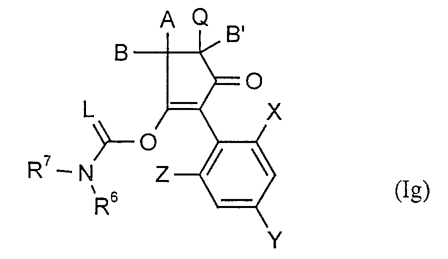 worin
A, B, B', E, L, M, Q, X, Y, Z, R¹, R², R³, R⁴, R⁵, R⁶ und R⁷ die oben angegebenen Bedeutungen besitzen.

Aufgrund eines oder mehrerer Chiralitätszentren, fallen die Verbindungen der Formel (Ia) - (Ig) im allgemeinen als Stereoisomerengemisch an. Sie können sowohl in Form ihrer Diastereomerengemische als auch als reine Diastereomere oder Enantiomere vorliegen und verwendet werden.

Weiterhin wurde gefunden, daß man die neuen substituierten Cyclopentan-1,3-dion-Derivate der Formel (I) nach einem der im folgenden beschriebenen Verfahren erhält.
(A) Man erhält Cyclopentan-1,3-dione bzw. deren Enole der Formel (Ia) 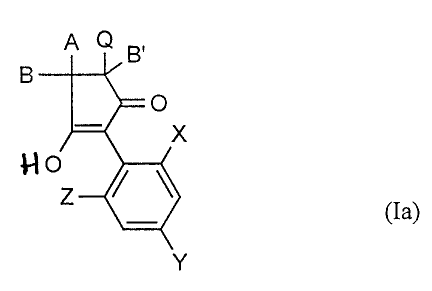 in welcher
   - A, B, B', Q, X, Y und Z: die oben angegebene Bedeutung haben,
   wenn man
   5-Aryl-4-keto-valeriansäureester der Formel (II) 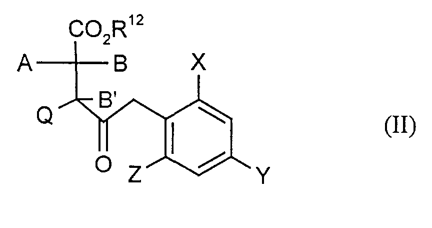 in welcher
   - A, B, B' Q, X, Y und Z: die oben angegebene Bedeutung haben, und
   - R¹²: für Alkyl (bevorzugt C₁-C₆-Alkyl) steht,
   in Gegenwart eines Verdünnungsmittels und in Gegenwart einer Base intramolekular kondensiert; und
(B) man erhält Verbindungen der Formel (Ib) 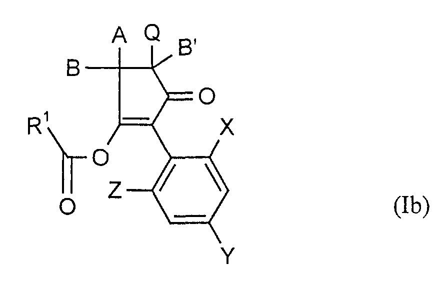 in welcher
   - A, B, B', Q, X, Y, Z und R¹: die oben angegebene Bedeutung haben,
   wenn man Verbindungen der Formel (Ia), 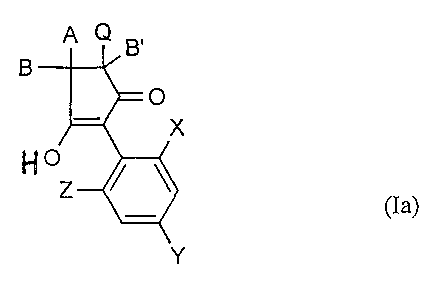 in welcher
   - A, B, B', X, Y, Z und Q: die oben angegebene Bedeutung haben,

   α) mit Säurehalogeniden der Formel (III) 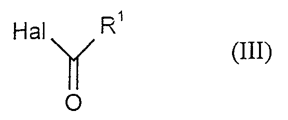 in welcher
      - R¹: die oben angegebene Bedeutung hat und
      - Hal: für Halogen (insbesondere Chlor und Brom) steht,
      gegebenenfalls in Gegenwart eines Verdünnungsmittels und gegebenenfalls in Gegenwart eines Säurebindemittels umsetzt
      oder
   β) mit Carbonsäureanhydriden der Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
      und
(C) man erhält Verbindungen der Formel (Ic-1) 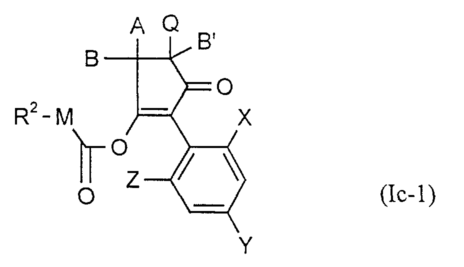 in welcher
   - A, B, B', Q, X, Y, Z und R²: die oben angegebene Bedeutung haben,
   und
   - M: für Sauerstoff oder Schwefel steht,
   wenn man Verbindungen der Formel (la) 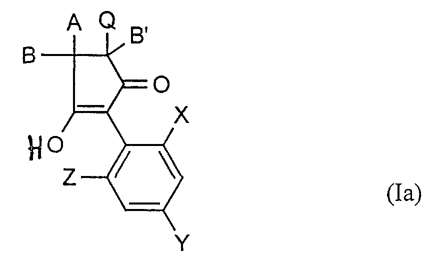 in welcher
   - A, B, B', Q, X, Y und Z: die oben angegebene Bedeutung haben,
   mit Chlorameisensäureestem oder Chlorameisensäurethioestern der Formel (V)

   R²-M-CO-Cl (V)

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt;
   und
(D) man erhält Verbindungen der Formel (Ic-2) 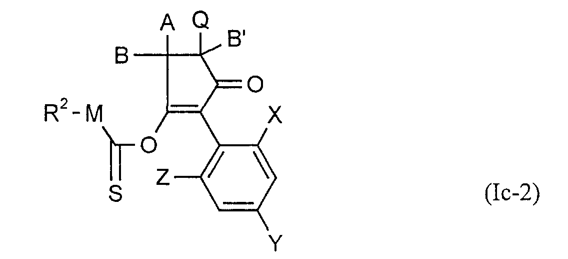 in welcher
   - A, B, B', Q, X, Y, Z und R²: die oben angegebene Bedeutung haben
   und
   - M: für Sauerstoff oder Schwefel steht,
   wenn man Verbindungen der Formel (Ia) 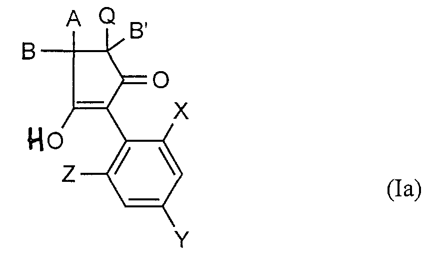 in welcher
   - A, B, B', Q, X, Y und Z: die oben angegebene Bedeutung haben,

   α) mit Chlormonothioameisensäureestern oder Chlordithioameisensäureestern der Formel (VI) 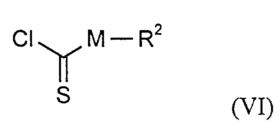 in welcher
      - M und R²: die oben angegebene Bedeutung haben,
      gegebenenfalls in Gegenwart eines Verdünnungsmittels und gegebenenfalls in Gegenwart eines Säurebindemittels umsetzt,
      oder
   β) mit Schwefelkohlenstoff und anschließend mit Alkylhalogeniden der allgemeinen Formel (VII)

      R²-Hal (VII)

      in welcher
      - R²: die oben angegebene Bedeutung hat
      und
      - Hal: für Chlor, Brom oder Iod steht,
      gegebenenfalls in Gegenwart eines Verdünnungsmittels und gegebenenfalls in Gegenwart einer Base umsetzt;
      und
(E) man erhält Verbindungen der Formel (Id) 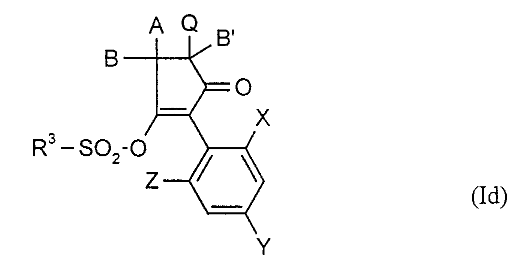 in welcher
   - A, B, B', Q, X, Y, Z und R³: die oben angegebene Bedeutung haben,
   wenn man Verbindungen der Formel (Ia) 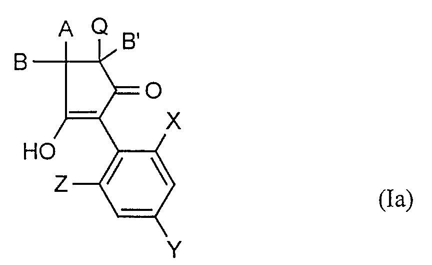 in welcher
   - A, B, B', Q, X, Y und Z: die oben angegebene Bedeutung haben,
   mit Sulfonsäurechloriden der Formel (VIII)

   R³-SO₂-Cl (VIII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt;
   und
(F) man erhält Verbindungen der Formel (Ie) 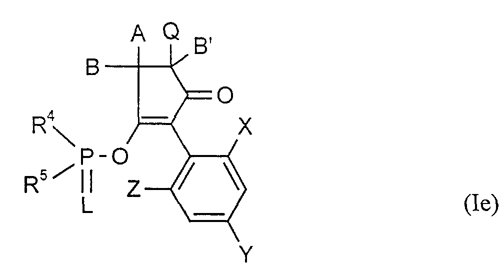 in welcher
   - A, B, L, B', Q, X, Y, Z, R⁴ und R⁵: die oben angegebene Bedeutung haben,
   wenn man
   Verbindungen der Formel (Ia) bzw. deren Enole 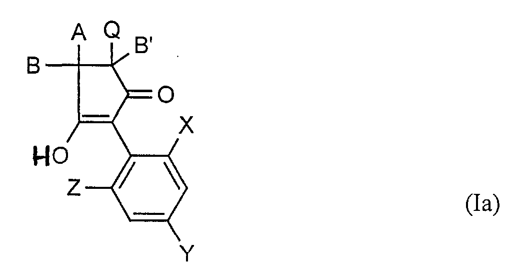 in welcher
   - A, B, B' Q, X, Y und Z: die oben angegebene Bedeutung haben,
   mit Phosphorverbindungen der Formel (IX) 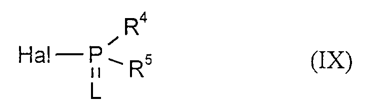 in welcher
   - L, R⁴ und R⁵: die oben angegebene Bedeutung haben
   und
   - Hal: für Halogen (insbesondere Chlor und Brom) steht,
   gegebenenfalls in Gegenwart eines Verdünnungsmittels und gegebenenfalls in Gegenwart eines Säurebindemittels umsetzt;
   und
(G) man erhält Verbindungen der Formel (If) 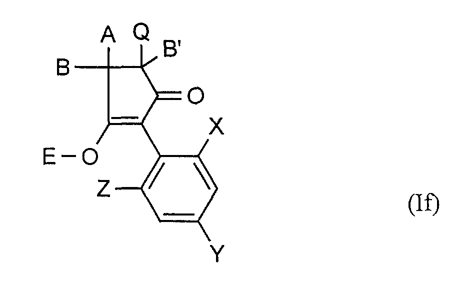 in welcher
   - A, B, B' Q, X, Y und Z: die oben angegebene Bedeutung haben,
   und
   - E: für ein Metallionäquivalent oder für ein Ammoniumion steht,
   wenn man Verbindungen der Formel (Ia) 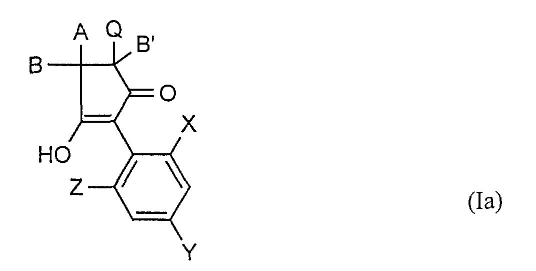 in welcher
   - A, B, B', Q, X, Y und Z: die oben angegebene Bedeutung haben,
   mit Metall-Verbindungen oder Aminen der Formeln (X) bzw. (XI)

   Me Rₜ ¹⁶ (X)

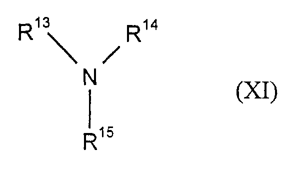

   in welchen
   - Me: für ein- oder zweiwertige Metallionen (insbesondere des Lithiums, Natriums, Kaliums, Magnesiums oder Calciums) steht,
   - t: für die Zahl 1 oder 2 steht,
   - R¹³, R¹⁴ und R¹⁵: unabhängig voneinander für Wasserstoff oder Alkyl (insbesondere C₁-C₈-Alkyl) stehen und
   - R¹⁶: für Wasserstoff, Hydroxy oder C₁-C₄-Alkoxy steht,
   gegebenenfalls in Gegenwart eines Verdünnungsmittels umsetzt.
(H) Ferner wurde gefunden, daß man Verbindungen der Formel (Ig) 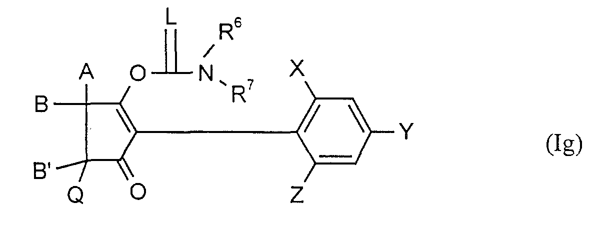 in welcher
   - A, B, L, B', Q, X, Y, Z, R⁶ und R⁷: die oben angegebene Bedeutung haben,
   erhält, wenn man Verbindungen der Formel (Ia) 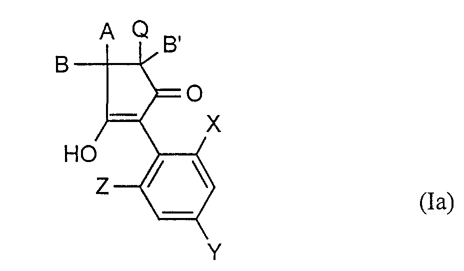 in welcher
   - A, B, B', Q, X, Y und Z: die oben angegebene Bedeutung haben,

   α) mit Verbindungen der Formel (XII)

      R⁶-N=C=L (XII)

      in welcher
      - L und R⁶: die oben angegebene Bedeutung haben,
      gegebenenfalls in Gegenwart eines Verdünnungsmittels und gegebenenfalls in Gegenwart eines Katalysators
      oder
   β) mit Carbamidsäurechloriden oder Thiocarbamidsäurechloriden der Formel (XIII) 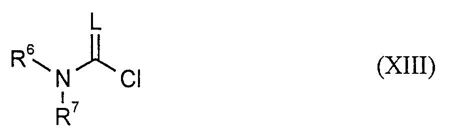 in welcher
      - L, R⁶ und R⁷: die oben angegebene Bedeutung haben
      gegebenenfalls in Gegenwart eines Verdünnungsmittels und gegebenenfalls in Gegenwart eines Säurebindemittels umsetzt.

Weiterhin wurde gefunden, daß sich die neuen Verbindungen der Formel (I) durch hervorragende insektizide, akarizide und herbizide Wirkungen auszeichnen.

Die erfindungsgemäßen Verbindungen sind durch die Formel (I) definiert.

Bevorzugte Substituenten bzw. Bereiche der in den oben und nachstehend erwähnten Formeln aufgeführten Reste werden im folgenden erläutert.
- X: steht bevorzugt für Fluor, Chlor, Brom, C₁-C₄-Alkyl, C₂-C₄-Alkenyl, C₁-C₄-Alkoxy, C₂-C₄-Alkenyloxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₂-C₄-Halogenalkenyloxy, Nitro, Cyano oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Phenylthio, Benzyloxy oder Benzylthio.
- Y: steht bevorzugt für Wasserstoff, Fluor, Chlor, Brom, C₁-C₄-Alkyl, C₂-C₄-Alkenyl, C₁-C₄-Alkoxy, C₂-C₄-Alkenyloxy, C₁-C₄-Alkylthio, C₁-C-Alkylsulfinyl, C₁-C₄-Alkylsulfonyl, C₁-C₄-Halogenalkyl, C₁-C₄-Halogenalkoxy, C₂-C₄-Halogenalkenyloxy, Nitro oder Cyano.
- Z: steht bevorzugt für Fluor, Chlor, Brom, C₁-C₄-Alkyl, C₂-C₄-Alkenyl, C₁-C₄-Alkoxy, C₂-C₄-Alkenyloxy, C₁-C₄-Halogenalkyl, C₁-C₄-Halogenalkoxy, C₂-C₄-Halogenalkenyloxy, Nitro oder Cyano.
- A und Q: stehen gemeinsam bevorzugt für jeweils gegebenenfalls einfach oder zweifach, gleich oder verschieden durch Fluor, Chlor, Brom, Hydroxy, Mercapto, durch jeweils gegebenenfalls einfach bis fünffach, gleich oder verschieden durch Fluor oder Chlor substituiertes C₁-C₈-Alkyl, C₁-C₄-Alkoxy, C₁-C₄-Alkylthio, C₅-C₇-Cycloalkyl oder Phenyl substituiertes C₁-C₅-Alkandiyl oder C₂-C₅-Alkendiyl, welches außerdem gegebenenfalls eine der nachstehenden Gruppierungen 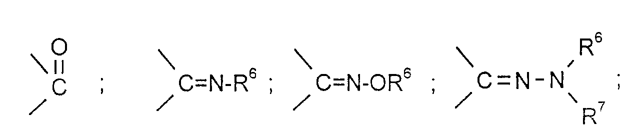 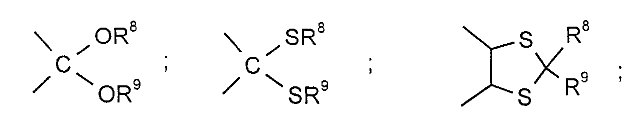 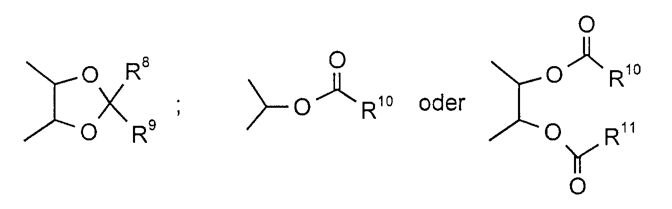 enthält oder durch eine C₁-C₂-Alkandiylgruppe überbrückt ist.
- B und B': stehen unabhängig voneinander bevorzugt für Wasserstoff, Fluor, Chlor oder C₁-C₄-Alkyl oder gemeinsam für jeweils gegebenenfalls durch C₁-C₄-Alkyl substituiertes C₁-C₅-Alkandiyl oder C₂-C₄-Alkendiyl.
- G: steht bevorzugt für Wasserstoff (a) oder für eine der Gruppen 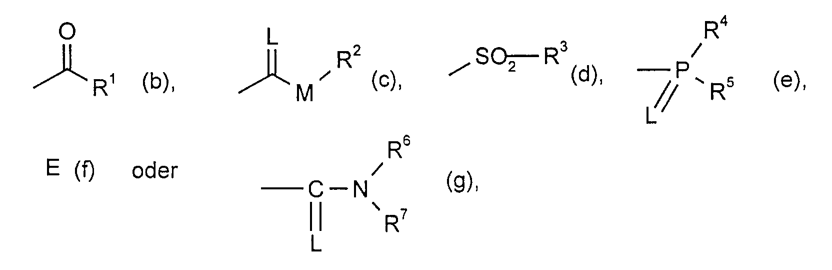 in welchen
E für ein Metallion oder ein Ammoniumion steht,
L für Sauerstoff oder Schwefel steht und
M für Sauerstoff oder Schwefel steht.
- R¹: steht bevorzugt für jeweils gegebenenfalls einfach bis neunfach, gleich oder verschieden durch Fluor oder Chlor substituiertes C₁-C₁₆-Alkyl, C₂-C₈-Alkenyl, C₁-C₆-Alkoxy-C₁-C₆-alkyl, C₁-C₆-Alkylthio-C₁-C₆-alkyl, Poly-C₁-C₆-alkoxy-C₁-C₆-alkyl oder für gegebenenfalls einfach bis dreifach, gleich oder verschieden durch Fluor, Chlor, C₁-C₄-Alkyl oder C₁-C₄-Alkoxy substituiertes C₃-C₇-Cycloalkyl, in welchem eine oder zwei nicht direkt benachbarte Methylengruppen durch Sauerstoff- und/oder Schwefelatome ersetzt sein können,
für gegebenenfalls einfach bis dreifach, gleich oder verschieden durch Fluor, Chlor, Brom, Cyano, Nitro, C₁-C₄-Alkyl, C₁-C₄-Alkoxy, C₁-C₃-Halogenalkyl oder C₁-C₃-Halogenalkoxy substituiertes Phenyl,
für gegebenenfalls einfach bis dreifach, gleich oder verschieden durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach oder zweifach, gleich oder verschieden durch Fluor, Chlor, Brom oder C₁-C₄-Alkyl substituiertes Furanyl, Thienyl, Pyridyl, Pyrimidyl, Thiazolyl oder Pyrazolyl,
für gegebenenfalls einfach bis dreifach, gleich oder verschieden durch Fluor, Chlor oder C₁-C₄-Alkyl substituiertes Phenoxy-C₁-C₅-alkyl oder
für jeweils gegebenenfalls einfach oder zweifach, gleich oder verschieden durch Fluor, Chlor, Amino oder C₁-C₄-Alkyl substituiertes Pyridyloxy-C₁-C₆-alkyl, Pyrimidinyloxy-C₁-C₆-alkyl oder Thiazolyloxy-C₁-C₆-alkyl.
- R²: steht besonders bevorzugt für jeweils gegebenenfalls einfach bis siebenfach, gleich oder verschieden durch Fluor oder Chlor substituiertes C₁-C₁₆-Alkyl, C₂-C₈-Alkenyl, C₁-C₆-Alkoxy-C₂-C₆-alkyl oder Poly-C₁-C₆-alkoxy-C₂-C₆-alkyl,
für gegebenenfalls einfach bis dreifach, gleich oder verschieden durch Fluor, Chlor, C₁-C₄-Alkyl oder C₁-C₄-Alkoxy substituiertes C₃-C₆-Cycloalkyl oder
für jeweils gegebenenfalls einfach bis dreifach, gleich oder verschieden durch Fluor, Chlor, Brom, Nitro, Cyano, C₁-C₄-Alkyl, C₁-C₄-Alkoxy, C₁-C₂-Halogenalkoxy oder C₁-C₂-Halogenalkyl substituiertes Phenyl oder Benzyl.
- R³: steht bevorzugt für gegebenenfalls einfach bis fünffach, gleich oder verschieden durch Fluor oder Chlor substituiertes C₁-C₉-Alkyl oder für jeweils gegebenenfalls einfach bis dreifach, gleich oder verschieden durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl oder Phenyl-C₁-C₂-alkyl.
- R⁴ und R⁵: stehen unabhängig voneinander für gegebenenfalls einfach bis fünffach, gleich oder verschieden durch Fluor oder Chlor substituiertes C₁-C₆-Alkyl, C₁-C₆-Alkoxy, C₁-C₆-Alkylamino, Di-(C₁-C₆-alkyl)-amino, C₁-C₆-Alkylthio, C₃-C₄-Alkenylthio, C₃-C₆-Cycloalkylthio oder für jeweils gegebenenfalls einfach bis dreifach, gleich oder verschieden durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶: steht bevorzugt für Wasserstoff, jeweils gegebenenfalls einfach bis fünffach, gleich oder verschieden durch Fluor oder Chlor substituiertes C₁-C₈-Alkyl, C₃-C₆-Alkenyl, C₁-C₆-Alkoxy-C₂-C₆-alkyl, für gegebenenfalls einfach bis dreifach, gleich oder verschieden durch Fluor, Chlor, C₁-C₄-Alkyl, C₁-C₄-Alkoxy, C₁-C₂-Halogenalkyl oder C₁-C₂-Halogenalkoxy substituiertes C₃-C₈-Cycloalkyl, für gegebenenfalls einfach bis dreifach, gleich oder verschieden durch Fluor, Chlor, Brom, C₁-C₂-Halogenalkyl, C₁-C₅-Alkyl, C₁-C₂-Halogenalkoxy oder C₁-C₅-Alkoxy substituiertes Phenyl oder für gegebenenfalls einfach bis dreifach, gleich oder verschieden durch Fluor, Chlor, Brom, C₁-C₅-Alkyl, C₁-C₂-Halogenalkyl, C₁-C₂-Halogenalkoxy oder C₁-C₅-Alkoxy substituiertes Benzyl.
- R⁷: steht bevorzugt für Wasserstoff oder für jeweils gegebenenfalls einfach bis fünffach, gleich oder verschieden durch Fluor oder Chlor substituiertes C₁-C₈-Alkyl oder C₃-C₈-Alkenyl oder
- R⁶ und R⁷: stehen bevorzugt gemeinsam mit dem N-Atom, an das sie gebunden sind, für einen gegebenenfalls Sauerstoff oder Schwefel enthaltenden und gegebenenfalls durch C₁-C₄-Alkyl substituierten 4- bis 7-gliedrigen Ring.
- R⁸ und R⁹: stehen unabhängig voneinander bevorzugt für Wasserstoff, gegebenenfalls einfach bis fünffach, gleich oder verschieden durch Fluor oder Chlor substituiertes C₁-C₄-Alkyl oder für gegebenenfalls einfach bis dreifach, gleich oder verschieden durch Fluor, Chlor, C₁-C₄-Alkyl, C₁-C₄-Alkoxy, C₁-C₂-Halogenalkyl, C₁-C₂-Halogenalkoxy, Nitro oder Cyano substituiertes Phenyl
oder zusammen für gegebenenfalls einfach bis dreifach, gleich oder verschieden durch Fluor, Chlor, C₁-C₄-Alkyl, C₁-C₄-Alkoxy oder C₁-C₂-Halogenalkyl substituiertes C₂-C₅-Alkandiyl.
- R¹⁰ und R¹¹: stehen unabhängig voneinander bevorzugt für jeweils gegebenenfalls einfach bis fünffach, gleich oder verschieden durch Fluor oder Chlor substituiertes C₁-C₈-Alkyl, C₂-C₈-Alkenyl, C₁-C₈-Alkoxy, C₁-C₈-Alkylamino, C₃-C₈-Alkenylamino, Di-(C₁-C₈-alkyl)-amino oder Di-(C₃-C₈-alkenyl)-amino.
- X: steht besonders bevorzugt für Fluor, Chlor, Brom, Methyl, Ethyl, Propyl, iso-Propyl, Ethenyl, 1-Propenyl, Methoxy, Ethoxy, Propoxy, iso-Propoxy, Allyloxy, Methallyloxy, Trifluormethyl, Difluormethoxy, Trifluormethoxy, Trifluorethoxy, Methylthio, Methylsulfinyl, Methylsulfonyl, Nitro, Cyano, oder jeweils gegebenenfalls durch Fluor, Chlor, Brom, Methyl, Ethyl, Propyl, iso-Propyl, tert.-Butyl, Methoxy, Ethoxy, Propoxy, tert.-Butoxy, Trifluormethyl, Trifluormethoxy, Nitro oder Cyano substituiertes Phenyl, Phenoxy, Phenylthio, Benzyloxy oder Benzylthio.
- Y: steht besonders bevorzugt für Wasserstoff, Fluor, Chlor, Brom, Methyl, Ethyl, n-Propyl, i-Propyl, n-Butyl, i-Butyl, tert.-Butyl, Ethenyl, 1-Propenyl, Methoxy, Ethoxy, Propoxy, iso-Propoxy, Allyloxy, Methallyloxy, Trifluormethyl, Methylthio, Methylsulfinyl, Methylsulfonyl, Difluormethoxy, Trifluormethoxy, Trifluorethoxy, Nitro oder Cyano.
- Z: steht besonders bevorzugt für Fluor, Chlor, Brom, Methyl, Ethyl, n-Propyl, i-Propyl, n-Butyl, i-Butyl, tert.-Butyl, Ethenyl, 1-Propenyl, Methoxy, Ethoxy, Propoxy, iso-Propoxy, Allyloxy, Methallyloxy, Trifluormethyl, Difluormethoxy, Trifluormethoxy, Trifluorethoxy, Nitro oder Cyano.
- A und Q: stehen gemeinsam besonders bevorzugt für jeweils gegebenenfalls einfach oder zweifach, gleich oder verschieden durch Fluor, Chlor, Hydroxy, durch jeweils gegebenenfalls einfach bis dreifach, gleich oder verschieden durch Fluor oder Chlor substituiertes C₁-C₆-Alkyl oder C₁-C₂-Alkoxy substituiertes C₁-C₄-Alkandiyl oder C₂-C₄-Alkendiyl.
- B und B': stehen unabhängig voneinander besonders bevorzugt für Wasserstoff, Methyl oder Ethyl.
- G: steht besonders bevorzugt für Wasserstoff (a) oder für eine der Gruppen 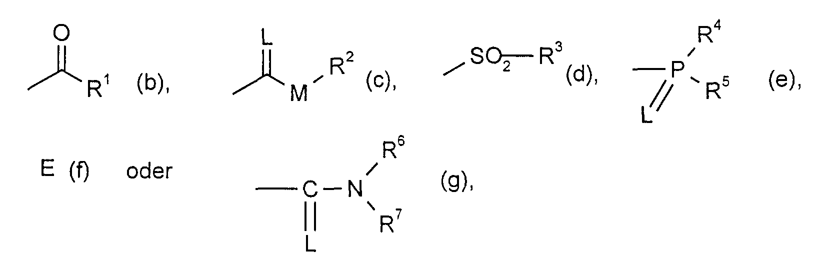 in welchen
E für ein Metallion oder ein Ammoniumion steht,
L für Sauerstoff oder Schwefel steht und
M für Sauerstoff oder Schwefel steht.
- R¹: steht besonders bevorzugt für jeweils gegebenenfalls einfach bis fünffach, gleich oder verschieden durch Fluor oder Chlor substituiertes C₁-C₁₄-Alkyl, C₂-C₆-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls einfach oder zweifach, gleich oder verschieden durch Fluor, Chlor, Methyl, Ethyl, Methoxy oder Ethoxy substituiertes C₃-C₆-Cycloalkyl, in welchem eine oder zwei nicht direkt benachbarte Methylengruppen durch Sauerstoff- und/oder Schwefelatome ersetzt sein können,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl, Trifluormethoxy, Cyano oder Nitro substituiertes Phenyl,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl oder Trifluormethoxy substituiertes Benzyl,
für jeweils gegebenenfalls einfach oder zweifach, gleich oder verschieden durch Fluor, Chlor, Brom, Methyl oder Ethyl substituiertes Thienyl, Furanyl oder Pyridyl,
für gegebenenfalls einfach oder zweifach, gleich oder verschieden durch Fluor, Chlor, Methyl oder Ethyl substituiertes Phenoxy-C₁-C₄-alkyl oder
für jeweils gegebenenfalls einfach oder zweifach, gleich oder verschieden durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht besonders bevorzugt für jeweils gegebenenfalls einfach bis fünffach, gleich oder verschieden durch Fluor oder Chlor substituiertes C₁-C₁₄-Alkyl, C₂-C₆-Alkenyl, C₁-C₄-Alkoxy-C₂-C₆-alkyl oder Poly-C₁-C₄-alkoxy-C₂-C₆-alkyl, für gegebenenfalls einfach bis dreifach, gleich oder verschieden durch Fluor, Chlor, Methyl, Ethyl, Methoxy oder Ethoxy substituiertes C₃-C₆-Cycloalkyl
oder für jeweils gegebenenfalls einfach oder zweifach, gleich oder verschieden durch Fluor, Chlor, Nitro, Cyano, Methyl, Ethyl, Propyl, i-Propyl, Methoxy, Ethoxy, Trifluormethoxy oder Trifluormethyl substituiertes Phenyl oder Benzyl.
- R³: steht besonders bevorzugt für gegebenenfalls einfach bis dreifach, gleich oder verschieden durch Fluor oder Chlor substituiertes C₁-C₆-Alkyl oder für jeweils gegebenenfalls einfach oder zweifach durch Fluor, Chlor, Brom, Methyl, Ethyl, Methoxy, Ethoxy, Trifluormethyl, Trifluormethoxy, Cyano oder Nitro substituiertes Phenyl oder Benzyl.
- R⁴ und R⁵: stehen unabhängig voneinander besonders bevorzugt für jeweils gegebenenfalls einfach bis dreifach, gleich oder verschieden durch Fluor oder Chlor substituiertes C₁-C₄-Alkyl, C₁-C₄-Alkoxy, C₁-C₄-Alkylamino, Di-(C₁-C₄-alkyl)-amino, C₁-C₄-Alkylthio oder für jeweils gegebenenfalls einfach oder zweifach, gleich oder verschieden durch Fluor, Chlor, Brom, Nitro, Cyano, C₁-C₂-Alkoxy, Trifluormethoxy, C₁-C₂-Alkylthio, Trifluormethyl oder C₁-C₃-Alkyl substituiertes Phenyl, Phenoxy oder Phenylthio.
- R⁶: steht besonders bevorzugt für Wasserstoff, jeweils gegebenenfalls einfach bis dreifach, gleich oder verschieden durch Fluor oder Chlor substituiertes C₁-C₆-Alkyl, C₁-C₆-Alkoxy-C₂-C₄-alkyl, für gegebenenfalls einfach oder zweifach, gleich oder verschieden durch Fluor, Chlor, Methyl, Methoxy, Trifluormethyl oder Trifluormethoxy substituiertes C₃-C₆-Cycloalkyl, für gegebenenfalls einfach oder zweifach, gleich oder verschieden durch Fluor, Chlor, Trifluormethyl, C₁-C₄-Alkyl, Trifluormethoxy oder C₁-C₄-Alkoxy substituiertes Phenyl oder für gegebenenfalls einfach oder zweifach, gleich oder verschieden durch Fluor, Chlor, C₁-C₄-Alkyl, Trifluormethyl, Trifluormethoxy oder C₁-C₄-Alkoxy substituiertes Benzyl.
- R⁷: steht besonders bevorzugt für Wasserstoff, jeweils gegebenenfalls einfach bis dreifach, gleich oder verschieden durch Fluor oder Chlor substituiertes C₁-C₆-Alkyl oder C₃-C₆-Alkenyl oder
- R⁶ und R⁷: stehen besonders bevorzugt gemeinsam mit dem N-Atom, an das sie gebunden sind, für einen gegebenenfalls Sauerstoff oder Schwefel enthaltenden, gegebenenfalls durch Methyl substituierten 5- bis 7-gliedrigen Ring.

Dabei gilt jeweils, daß X, Y und Z nicht gleichzeitig für Methyl stehen.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

In den oben aufgeführten Definitionen können gesättigte oder ungesättigte Kohlenwasserstoffreste, auch in Verbindung mit Heteroatomen (beispielsweise Alkoxy oder Alkenylthio) jeweils, soweit möglich, geradkettig oder verzweigt sein.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Substituierte Reste können, wo nichts anderes angegeben ist, einfach oder mehrfach, gleich oder verschieden durch die in Frage kommenden Substituenten substituiert sein.

Im einzelnen seien außer den bei den Herstellungsbeispielen genannten Verbindungen die folgenden 2-Phenyl-substituierten 3-Hydroxy-Δ²-cyclopentenon-Derivate der Formel (Ia) genannt.

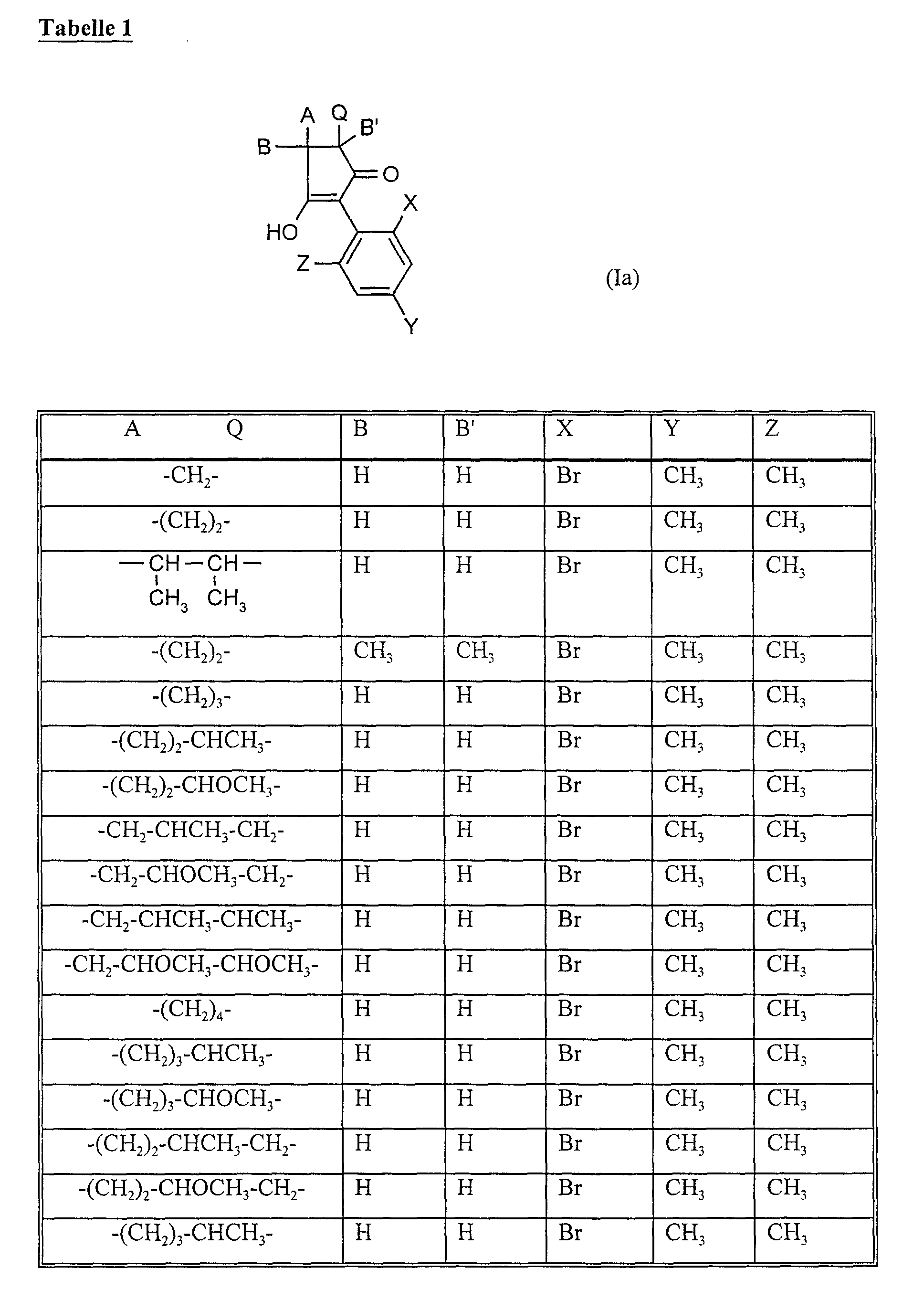

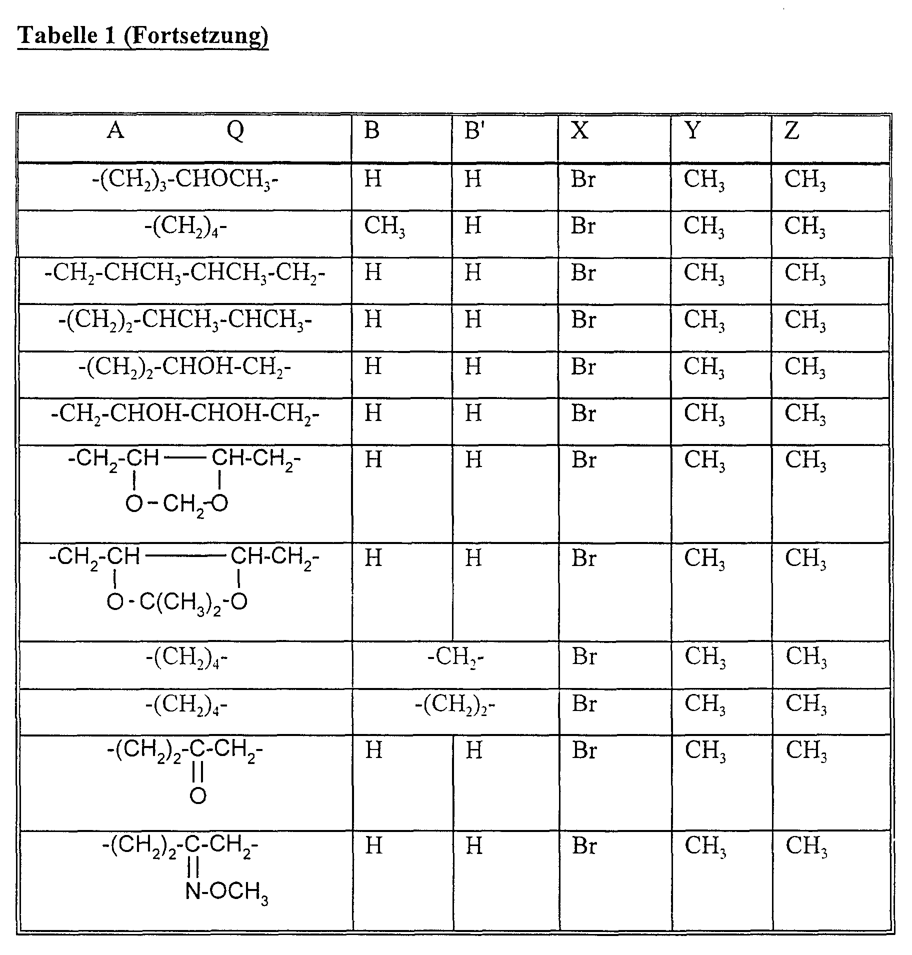

**Tabelle 2**

| | | |
|---|---|---|
| A, Q, B und B' wie in Tabelle 1 | | |
| X = CH₃ | Y = Br | Z = CH₃ |

**Tabelle 3**

| | | |
|---|---|---|
| A, Q, B und B' wie in Tabelle 1 | | |
| X = C₂H₅ | Y = Br | Z = CH₃ |

**Tabelle 4**

| | | |
|---|---|---|
| A, Q, B und B' wie in Tabelle 1 | | |
| X = Cl | Y = CH₃ | Z = CH₃ |

**Tabelle 5**

| | | |
|---|---|---|
| A, Q, B und B' wie in Tabelle 1 | | |
| X = CH₃ | Y = Cl | Z = CH₃ |

**Tabelle 6**

| | | |
|---|---|---|
| A, Q, B und B' wie in Tabelle 1 | | |
| X = OCH₃ | Y = CH₃ | Z = CH₃ |

**Tabelle 7**

| | | |
|---|---|---|
| A, Q, B und B' wie in Tabelle 1 | | |
| X = CH₃ | Y = OCH₃ | Z = CH₃ |

**Tabelle 8**

| | | |
|---|---|---|
| A, Q, B und B' wie in Tabelle 1 | | |
| X = OCH₃ | Y = H | Z = CH₃ |

**Tabelle 9**

| | | |
|---|---|---|
| A, Q, B und B' wie in Tabelle 1 | | |
| X = Cl | Y = H | Z = CH₃ |

**Tabelle 10**

| | | |
|---|---|---|
| A, Q, B und B' wie in Tabelle 1 | | |
| X = Cl | Y = H | Z = Cl |

**Tabelle 11**

| | | |
|---|---|---|
| A, Q, B und B' wie in Tabelle 1 | | |
| X = CH₃ | Y = H | Z = CH₃ |

**Tabelle 12**

| | | |
|---|---|---|
| A, Q, B und B' wie in Tabelle 1 | | |
| X = Cl | Y = Cl | Z = CH₃ |

**Tabelle 13**

| | | |
|---|---|---|
| A, Q, B und B' wie in Tabelle 1 | | |
| X = Cl | Y = CH₃ | Z = Cl |

**Tabelle 14**

| | | |
|---|---|---|
| A, Q, B und B' wie in Tabelle 1 | | |
| X = Cl | Y = H | Z = OCH₃ |

**Tabelle 15**

| | | |
|---|---|---|
| A, Q, B und B' wie in Tabelle 1 | | |
| X = CH₃ | Y = CN | Z = CH₃ |

**Tabelle 16**

| | | |
|---|---|---|
| A, Q, B und B' wie in Tabelle 1 | | |
| X = CN | Y = CH₃ | Z = CH₃ |

Verwendet man gemäß Verfahren (A) 5-(2-Chlor-6-methylphenyl)-2,3-tetramethylen-4-oxo-valeriansäureethylester, so kann der Verlauf des erfindungsgemäßen Verfahrens durch folgendes Reaktionsschema wiedergegeben werden:

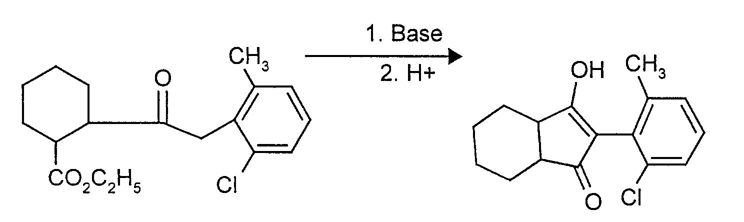

Verwendet man gemäß Verfahren (B) (Variante α) 2-(2,4-Dichlor-6-methylphenyl)-4,5-(2,3-dimethyl)-tetramethylen-3-hydroxy-2-cyclopenten-1-on und Pivaloylchlorid als Ausgangsstoffe, so kann der Verlauf des erfindungsgemäßen Verfahrens durch folgendes Reaktionsschema wiedergegeben werden:

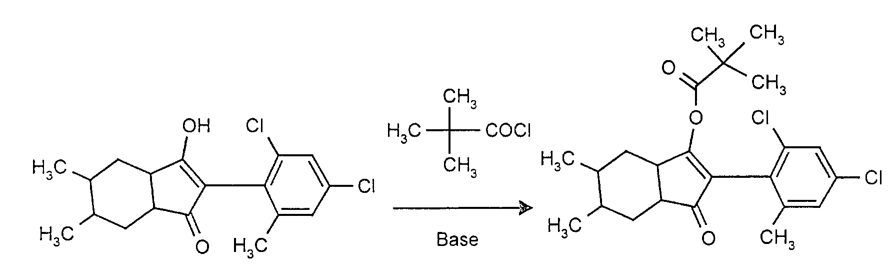

Verwendet man gemäß Verfahren B (Variante β) 2-(2-Chlor-6-methoxyphenyl)-4,5-methylen-3-hydroxy-2-cyclopenten-1-on und Acetanhydrid als Ausgangsverbindungen, so kann der Verlauf des erfindungsgemäßen Verfahrens durch folgendes Reaktionsschema wiedergegeben werden:

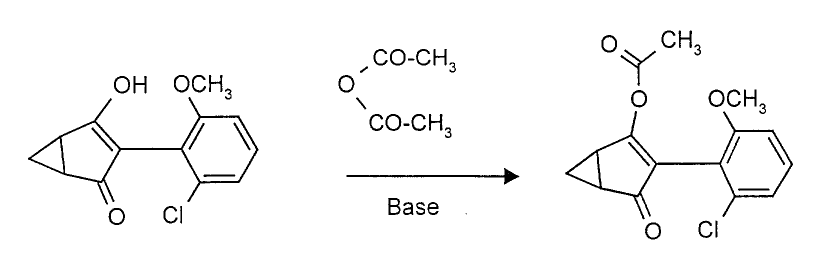

Verwendet man gemäß Verfahren (C) 2-(2-Chlor-4,6-dimethylphenyl)-4,5-(3-oxo)-tetramethylen-3-hydroxy-2-cyclopenten-1-on und Chlorameisensäureethoxyethylester als Ausgangsverbindungen, so kann der Verlauf des erfindungsgemäßen Verfahrens durch folgendes Reaktionsschema wiedergegeben werden.

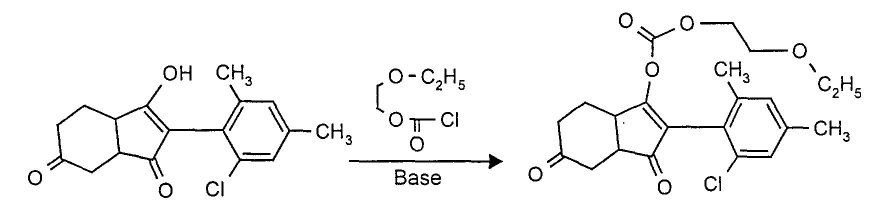

Verwendet man gemäß Verfahren (D_{α}) 2-(4-Chlor-2,6-dimethylphenyl)-4,5-(3-methyl)-tetramethylen-3-hydroxy-2-cyclopenten-1-on und Chlormonothioameisensäuremethylester als Ausgangsprodukte, so kann der Reaktionsverlauf wie folgt wiedergegeben werden:

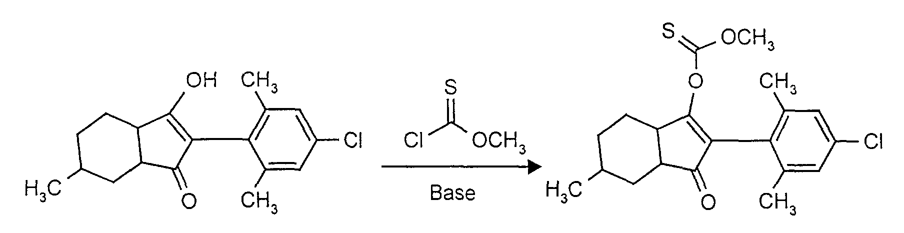

Verwendet man gemäß Verfahren (D_{β}) 2-(2,6-Dichlorphenyl)-4,5-trimethylen-3-hydroxy-2-cyclopenten-1-on, Schwefelkohlenstoff und Methyliodid als Ausgangskomponenten, so kann der Reaktionsverlauf wie folgt wiedergegeben werden:

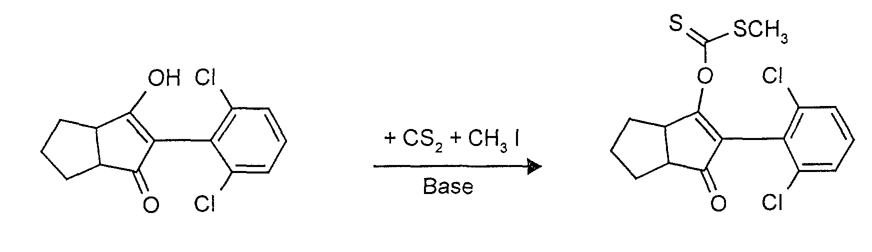

Verwendet man gemäß Verfahren (E) 2-(4-Brom-2,6-dimethylphenyl)-4,5-(3-methoxy)-tetramethylen-3-hydroxy-2-cyclopenten-1-on und Methansulfonsäurechlorid als Ausgangsprodukt, so kann der Reaktionsverlauf durch folgendes Reaktionsschema wiedergegeben werden:

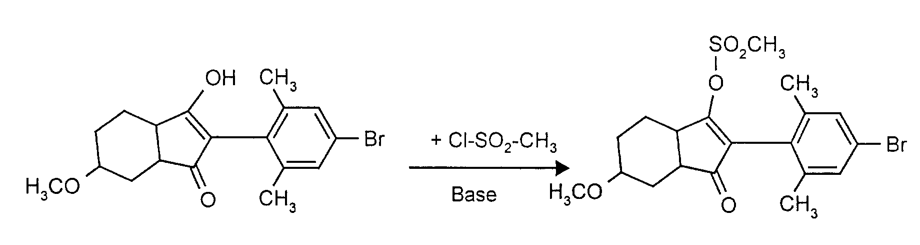

Verwendet man gemäß Verfahren (F) 2-(4-Brom-2,6-dimethylphenyl-4,5-(4-methyl)tetramethylen-3-hydroxy-2-cyclopenten-1-on und Methanthiophosphonsäurechlorid-(2,2,2-trifluorethylester) als Ausgangsprodukte, so kann der Reaktionsverlauf durch folgendes Reaktionsschema wiedergegeben werden:

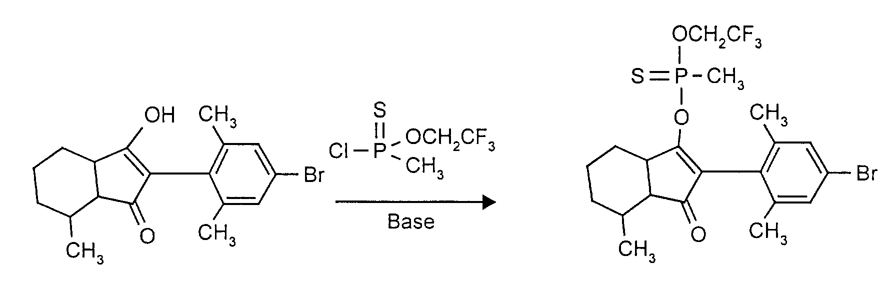

Verwendet man gemäß Verfahren (G) 2-(2-Brom-4,6-dimethylphenyl)-4,5-(3,3-ethylendioxy)-tetramethylen-3-hydroxy-2-cyclopenten-1-on und NaOH als Komponenten, so kann der Verlauf des erfindungsgemäßen Verfahrens durch folgendes Reaktionsschema wiedergegeben werden:

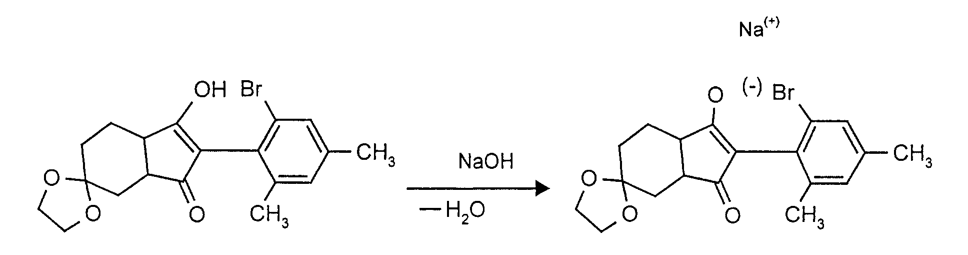

Verwendet man gemäß Verfahren (H_{α}) 2-(2-Methoxy-4,6-dimethylphenyl)-4,5-(3-methoxy)-tetramethylen-3-hydroxy-2-cyclopenten-1-on und Ethylisocyanat als Ausgangsprodukte, so kann der Reaktionsverlauf durch folgendes Schema wiedergegeben werden:

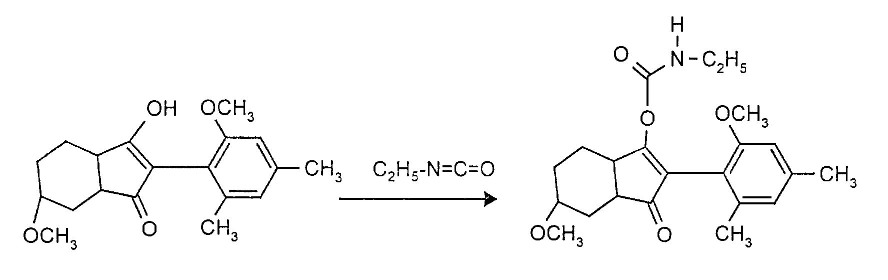

Verwendet man gemäß Verfahren (H_{β}) 2-(4-Methoxy-2,6-dimethylphenyl)-4,5-tetramethylen-3-hydroxy-2-cyclopenten-1-on und Dimethylcarbamidsäurechlorid als Ausgangsprodukte, so kann der Reaktionsverlauf durch folgendes Schema wiedergegeben werden:

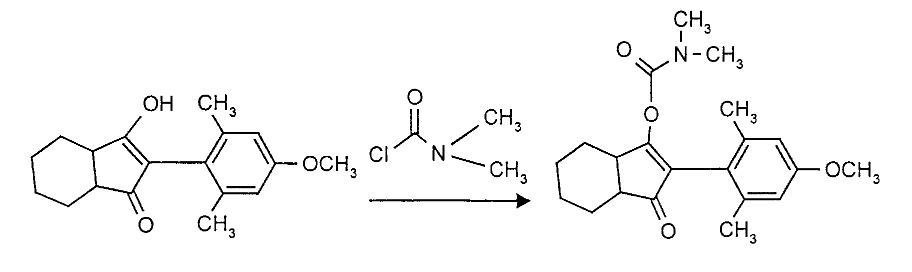

Die bei dem obigen Verfahren (A) als Ausgangsstoffe benötigten Verbindungen der Formel (II) 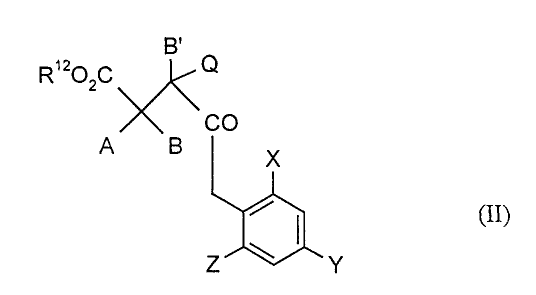 in welcher
- A, B, B', Q, X, Y, Z und R¹²: die oben angegebene Bedeutung haben,
sind neu. Sie lassen sich nach im Prinzip bekannten Methoden herstellen. Man erhält die 5-Aryl-4-ketocarbonsäureester der Formel (II) beispielsweise, wenn man 5-Aryl-4-ketocarbonsäuren der Formel (XIV) 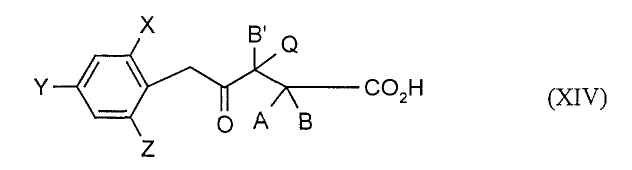 in welcher
- A, B, B', Q, X, Y und Z: die oben angegebene Bedeutung haben,
verestert, (vgl. z.B. Organikum, 15. Auflage, Berlin, 1977, Seite 499).

Die 5-Aryl-4-ketocarbonsäuren der Formel (XIV) 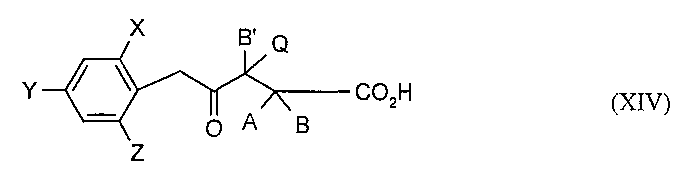 in welcher
- A, B, B', Q, X, Y und Z: die oben angegebene Bedeutung haben,
sind neu, lassen sich aber nach im Prinzip bekannten Methoden herstellen.

Man erhält die 5-Aryl-4-ketocarbonsäuren der Formel (XIV) beispielsweise, wenn man Carbonsäureanhydride der Formel (XV) 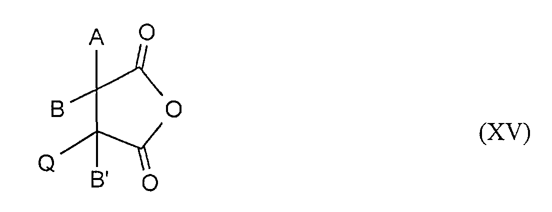 in welcher
- A, B, B' und Q: die oben angegebene Bedeutung haben,
mit metallorganischen Verbindungen der Formel (XVI) 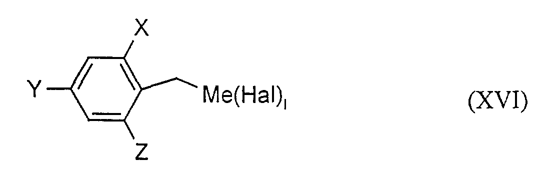 in welcher
- X, Y und Z: die oben angegebene Bedeutung haben,
- Me: für ein- oder zweiwertige Metallionen (beispielsweise des Lithiums oder Magnesiums),
- Hal: für Chlor oder Brom
und
- l: für eine Zahl 0 oder 1 steht,
in Gegenwart eines Verdünnungsmittels umsetzt (vgl. z.B. Organikum, 15. Auflage, Berlin, 1977, Seite 623).

Die Verbindungen (XV) und (XVI) sind teilweise bekannt und/oder lassen sich nach bekannten Verfahren in einfacher Weise darstellen (vgl. z.B. Organikum, 15. Auflage, Berlin, 1977, Seiten 525, 526 und 623).

Weiterhin erhält man 5-Aryl-4-ketocarbonsäuren der Formel (XIV) 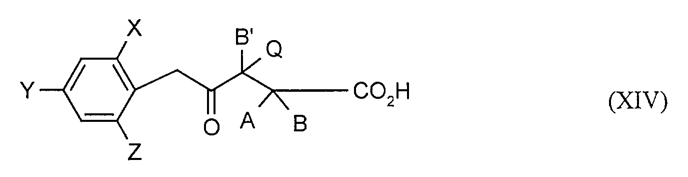 in welcher
- A, B, B', Q, X, Y und Z: die oben angegebene Bedeutung haben,
wenn man substituierte 2-Phenyl-3-oxo-adipinsäureester der Formel (XVII) 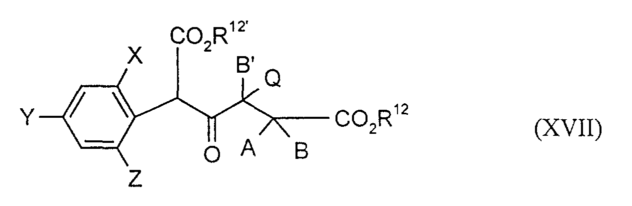 in welcher
- A, B, B', Q, X, Y und Z: die oben angegebene Bedeutung haben und
- R¹² und R¹²': für Alkyl (bevorzugt C₁-C₆-Alkyl), stehen,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XVII) 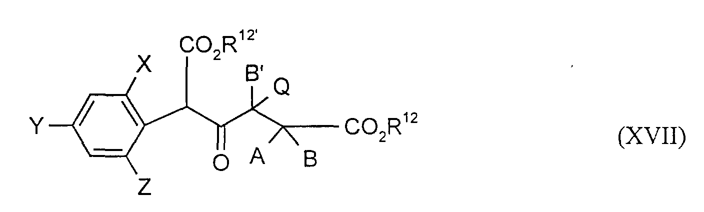 in welcher
- A, B, B', Q, X, Y, Z, R¹² und R^{12'}: die oben angegebene Bedeutung haben,
sind neu und erhältlich,
wenn man Dicarbonsäurehalbesterchloride der Formel (XVIII), 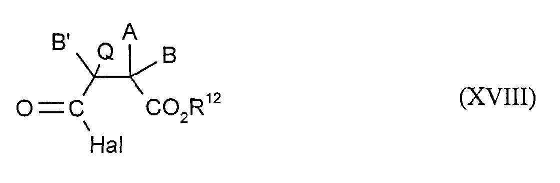 in welcher
- A, B, B', Q und R¹²: die oben angegebene Bedeutung haben und
- Hal: für Chlor oder Brom steht,
oder Carbonsäureanhydride der Formel (XV) 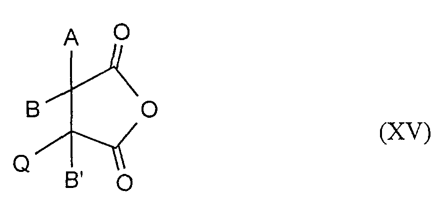 in welcher
- A, B, B' und Q: die oben angegebene Bedeutung haben,
mit einem substituierten Phenylessigsäureester der Formel (XIX) 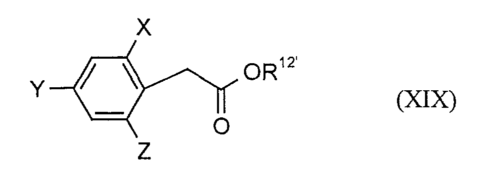 in welcher
- X, Y, Z und R^{12'}: die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228).

Die Verbindungen der Formel (XVIII) und (XIX) sind teilweise bekannt und/oder lassen sich nach bekannten Verfahren darstellen.

Die erfindungsgemäßen Wirkstoffe der Formeln (Ib) bis (Ig) werden jeweils ausgehend von den nach dem erfindungsgemäßen Verfahren (A) erhältlichen Verbindungen der Formel (Ia) hergestellt. Diese sind wichtige Zwischenprodukte für die Herstellung der Verbindungen der Formeln (Ib) bis (Ig).

Die zur Durchführung der erfindungsgemäßen Verbindungen (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Alkylhalogenide der Formel (VII), Sulfonsäurechloride der Formel (VIII), Phosphorverbindungen der Formel (IX) und Metall-verbindungen oder Amine der Formeln (X) und (XI) und Isocyanate der Formel (XII) und Carbamidsäurechloride oder Thiocarbamidsäurechloride der Formel (XIII) sind allgemein bekannte Verbindungen der organischen bzw. anorganischen Chemie.

Das Verfahren (A) ist dadurch gekennzeichnet, daß man Verbindungen der Formel (II), in welcher A, B, B', Q, X, Y, Z und R¹² die oben angegebene Bedeutung haben, in Gegenwart von Basen einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (A) alle üblichen Protonenakzeptoren eingesetzt werden.

Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecan (DBU), Diazabicyclononen (DBN), Hünig-Base und N,N-Dimethylanilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Bei dem erfindungsgemäßen Verfahren (Bβ) können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethylanilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat.

Als Verdünnungsmittel können bei dem erfindungsgemäßen Verfahren (C) alle gegenüber den Ausgangsstoffen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Methyl-tert.-butylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethioester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (Dα) setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten organischen Lösungsmittel in Frage, wie halogenierte Kohlenwasserstoffe, Ether, Amide, Alkohole, Nitrile, Sulfone und Sulfoxide.

Vorzugsweise werden Acetonitril, Dimethylsulfoxid, Methyl-tert.-butylether, Tetrahydrofuran, Dimethylformamid, Ethylacetat oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Dβ) setzt man pro Mol Ausgangsverbindung der Formel (II) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Als Basen können beim Verfahren (Dβ) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise verwendbar sind aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Oft ist es zweckmäßig, zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung der Formel (Ia) so lange mit Schwefelkohlenstoff um bis die Bildung der Zwischenverbindung abgeschlossen ist, z.B. nach mehrstündigem Rühren bei Raumtemperatur.

Die weitere Umsetzung mit dem Alkylhalogenid der Formel (VII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (E) setzt man pro Mol Ausgangsverbindung der Formel (Ia) ca. 1 Mol Sulfonsäurechlorid der Formel (VIII) bei 0 bis 150°C, vorzugsweise bei 20 bis 70°C um.

Als gegebenenfalls zugesetzte Verdünnungsmittel kommen alle inerten organischen Lösungsmittel in Frage wie halogenierte Kohlenwasserstoffe, Ether, Amide, Carbonsäureester, Nitrile, Sulfone oder Sulfoxide.

Vorzugsweise werden Acetonitril, Dimethylsulfoxid, Ethylacetat, Methyl-tert.-butyl-ether, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat und Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (E) kann gegebenenfalls unter Phasen-Transfer-Bedingungen gearbeitet werden (W.J. Spillane et. al.; J. Chem. Soc., Perkin Trans I, (3) 677-9 (1982)). In diesem Fall setzt man pro Mol Ausgangsverbindung der Formel (Ia) 0,3 bis 5 Mol Sulfonsäurechlorid der Formel (VIII), bevorzugt 1 Mol bei 0° bis 150°C, vorzugsweise bei 20 bis 70°C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol oder Toluol eingesetzt.

Beim Herstellungsverfahren (F) setzt man zum Erhalt von Verbindungen der Formel (Ie) auf 1 Mol der Verbindung der Formel (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C, ein.

Als gegebenenfalls zugesetzte Verdünnungsmittel kommen alle inerten organischen Lösungsmittel in Frage wie halogenierte Kohlenwasserstoffe, Ether, Amide, Nitrile, Carbonsäureester, Sulfone, Sulfoxide etc.

Vorzugsweise werden Acetonitril, Dimethylsulfoxid, Ethylacetat, Methyl-tert.-butylether, Tetrahydrofuran, Dimethylformamid oder Methylenchlorid eingesetzt.

Als gegebenenfalls zugesetzte Säurebindemittel kommen übliche anorganische oder organische Basen in Frage wie Hydroxide, Amine, Carbonate. Beispielhaft seien Natriumhydroxid, Natriumcarbonat, Kaliumcarbonat, Pyridin, Triethylamin oder DABCO aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, daß man Verbindungen der Formel (Ia) mit Metallverbindungen der Formel (X) oder Aminen der Formel (XI) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgeführt. Die Reaktionstemperaturen liegen im allgemeinen zwischen -20°C und 100°C, vorzugsweise zwischen 0°C und 50°C.

Bei der Durchführung des erfindungsgemäßen Verfahrens (H) werden die Ausgangsstoffe der Formeln (Ia) bzw. (XII) oder (XIII) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittel einengt.

Beim Herstellungsverfahren (Hα) setzt man pro Mol Ausgangsverbindung der Formel (Ia) ca. 1 Mol Isocyanat bzw. Isothiocyanat der Formel (XII) bei 0 bis 100°C, vorzugsweise bei 20 bis 50°C um.

Als gegebenenfalls zugesetzte Verdünnungsmittel kommen alle inerten organischen Lösungsmittel in Frage, wie Kohlenwasserstoffe, halogenierte Kohlenwasserstoffe, Ether, Carbonsäureester, Amide, Nitrile, Sulfone, Sulfoxide.

Vorzugsweise werden Toluol, Methylenchlorid, Tetrahydrofuran, Ethylacetat, Dimethylformamid oder Dimethylsulfoxid eingesetzt.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Hß) setzt man pro Mol Ausgangsverbindung der Formel (Ia) ca. 1 Mol Carbamidsäurechlorid bzw. Thiocarbamidsäurechlorid der Formel (XIII) bei 0 bis 150°C, vorzugsweise bei 20 bis 70°C um.

Als gegebenenfalls zugesetzte Verdünnungsmittel kommen aller inerten polaren organischen Lösungsmittel in Frage wie halogenierte Kohlenwasserstoffe, Carbonsäureester, Ether, Amide, Nitrile, Sulfone oder Sulfoxide.

Vorzugsweise werden Acetonitril, Ethylacetat, Dimethylsulfoxid, Methyl-tert.-butyl-ether, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin oder DABCO aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (I) zeichnen sich insbesondere durch hervorragende insektizide Wirksamkeit aus. Sie zeigen starke Wirkung beispielsweise gegen Meerettichblattkäfer-Larven (Phaedon cochleariae) und Raupen der Kohlschabe (Plutella maculipennis).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindunngen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat;Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

### Herstellungsbeispiele

### Beispiel Ia-1

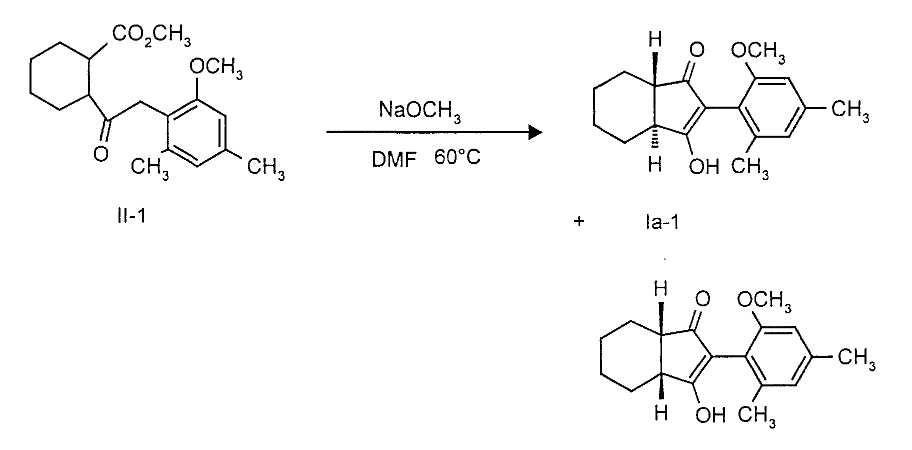

Zu einer Lösung der Verbindung gemäß Beispiel II-1 (21 g; 68,6 mmol) in DMF (30 ml) gibt man 10,5 g Natriummethylat und rührt 2 Stunden bei 60°C. Man gibt 5 ml Essigsäure zu, engt ein und chromatographiert an Kieselgel (1:1 Ethylacetat:Hexan). Man isoliert 5 g (25 %) der oben gezeigten Verbindungen als cis-trans Gemisch.
Farbloser Feststoff, Fp.: 128°C.

Analog zu Beispiel Ia-1 bzw. gemäß den allgemeinen Angaben zur Herstellung wurden die in der folgenden Tabelle aufgeführten Verbindungen der Formel (Ia) hergestellt:

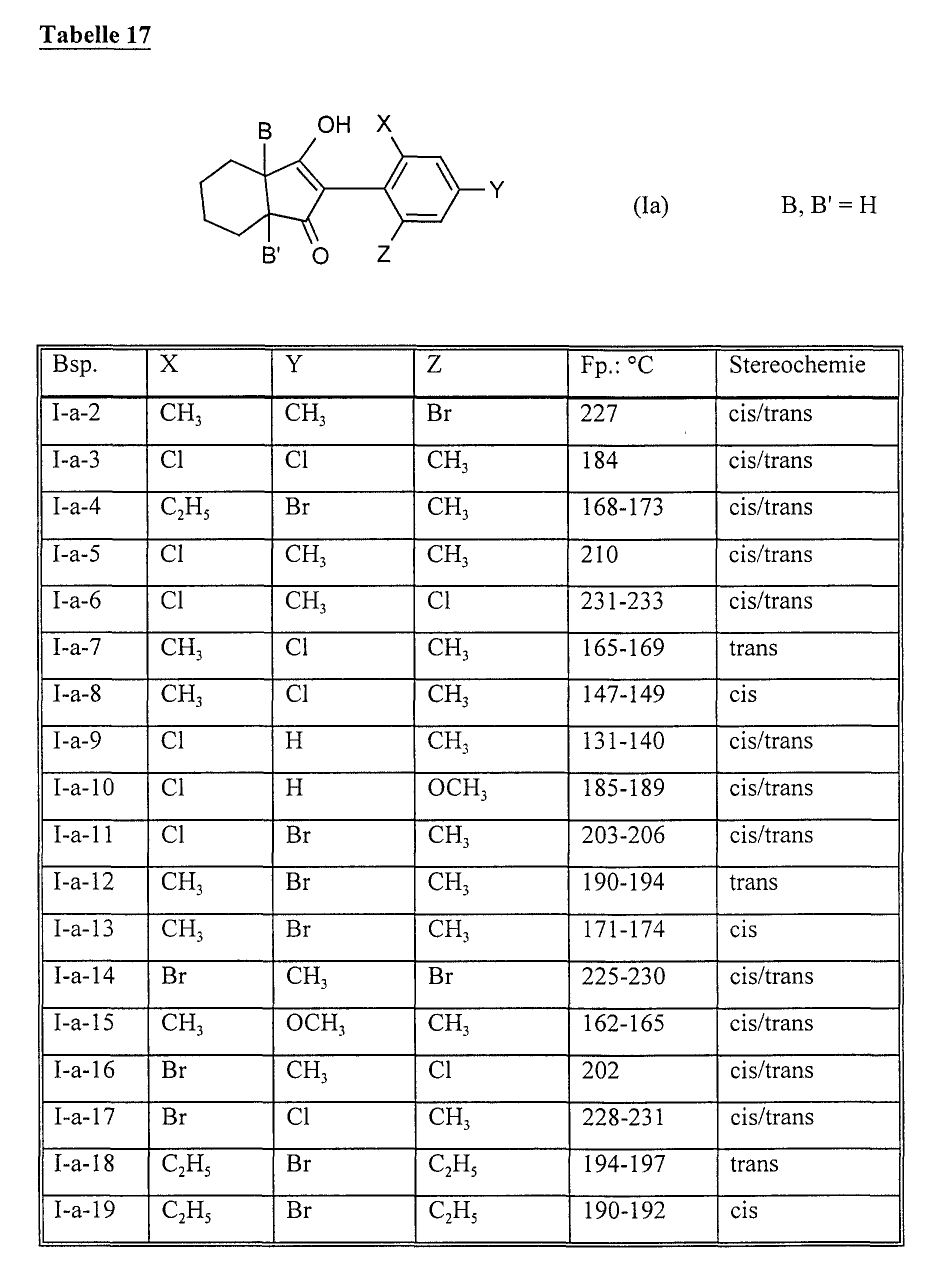

### Beispiel Ib-1

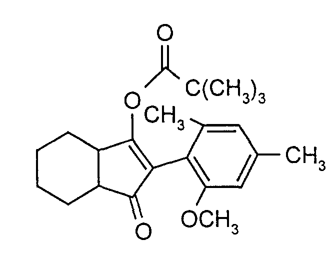

Zu 1,0 g (3,5 mmol) der Verbindung gemäß Beispiel (Ia-1) und 0,73 ml Triethylamin in 15 ml trockenem Methylenchlorid tropft man unter Eiskühlung eine Lösung von 0,56 ml (4,55 mmol) Pivaloylchlorid in 30 ml trockenem Methylenchlorid. Man rührt 2 Stunden bei Raumtemperatur, wäscht 2 mal mit 10 %iger wäßriger Citronensäure und extrahiert die wäßrigen sauren Phasen mit Methylenchlorid. Die vereinigten organischen Phasen werden 2 mal mit IN NaOH gewaschen, die wäßrigen alkalischen Phasen mit Methylenchlorid extrahiert und schließlich die vereinigten organischen Phasen getrocknet und eingeengt.

Man erhält 1,32 g (100% der Theorie) der oben gezeigten Verbindung als Öl (Isomerengemisch).
¹H-NMR (CDCl₃, 500 MHz)
δ = 1.12-1.18 (9H); 2.02-2.30 (6H); 3.63-3.73 (3H); 6.48-6.65 (2H).

Analog bzw. gemäß den allgemeinen Angaben zur Herstellung erhält man die folgenden Verbindungen der Formel (I-b):

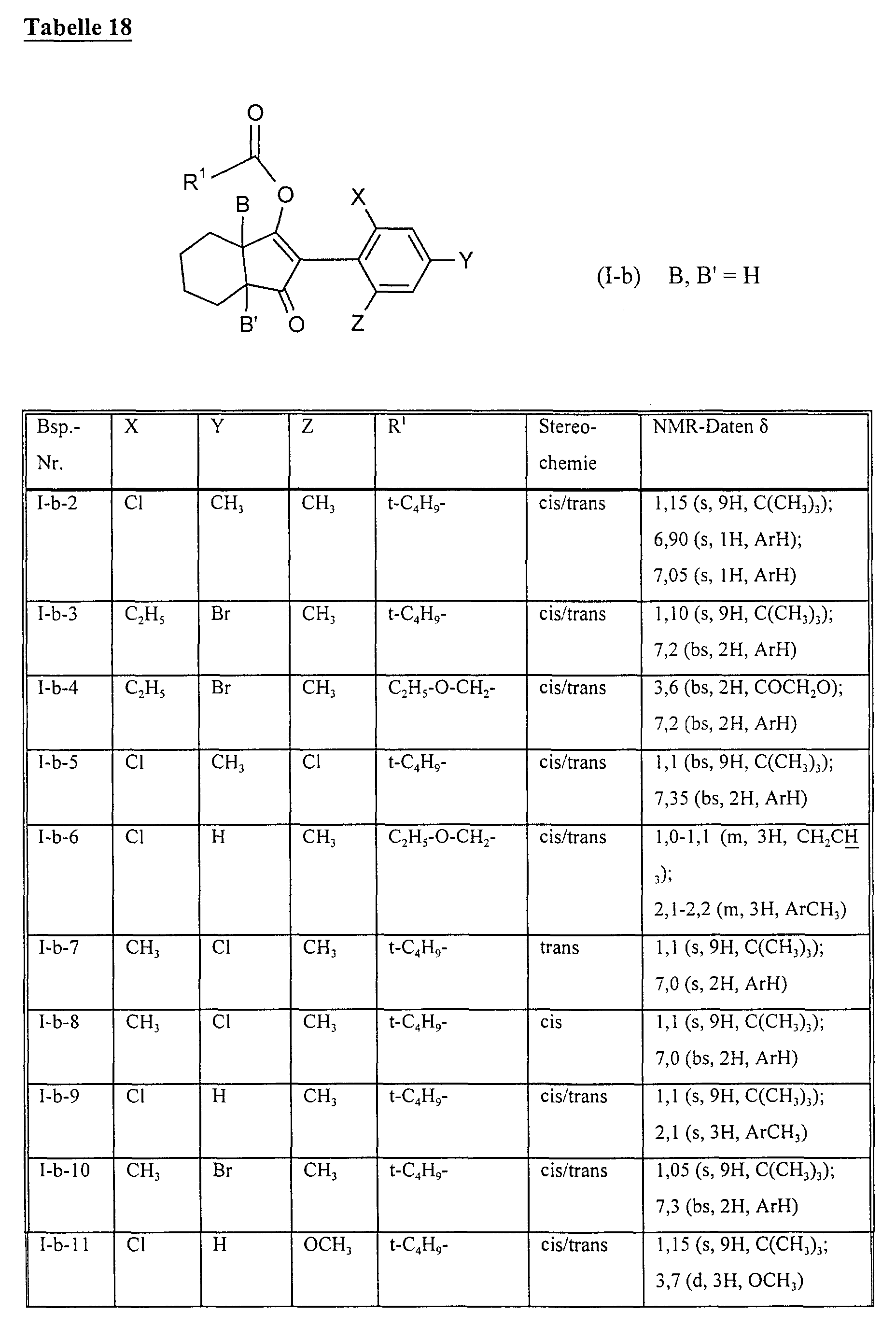

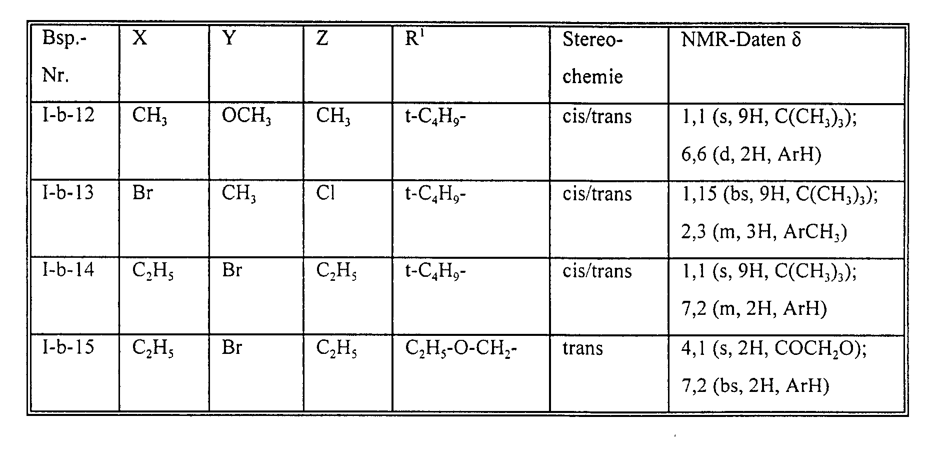

### Beispiel Ic-1

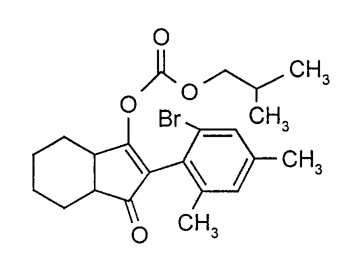

Zu 1,17 g (3,5 mmol) der Verbindung gemäß Beispiel (Ia-2) und 0,73 ml Triethylamin in 15 ml trockenem Methylenchlorid tropft man unter Eiskühlung eine Lösung von 0,59 ml (4,55 mmol) Chlorameisensäureisobutylester in 3 ml trockenem Methylenchlorid. Man rührt 2 Stunden bei Raumtemperatur und arbeitet dann wie bei Beispiel (Ib-1) beschrieben auf.

Man erhält 1,55 g (100% der Theorie) der oben gezeigten Verbindung als Öl (Isomerengemisch).
¹H-NMR (CDCl₃, 500 MHz)
δ = 0.85-0.90 (6H); 2.10-2.30 (6H); 3.85-3.95 (2H); 6.97-7.24 (2H).

Analog bzw. gemäß den allgemeinen Angaben zur Herstellung erhält man die folgenden Verbindungen der Formel (I-c):

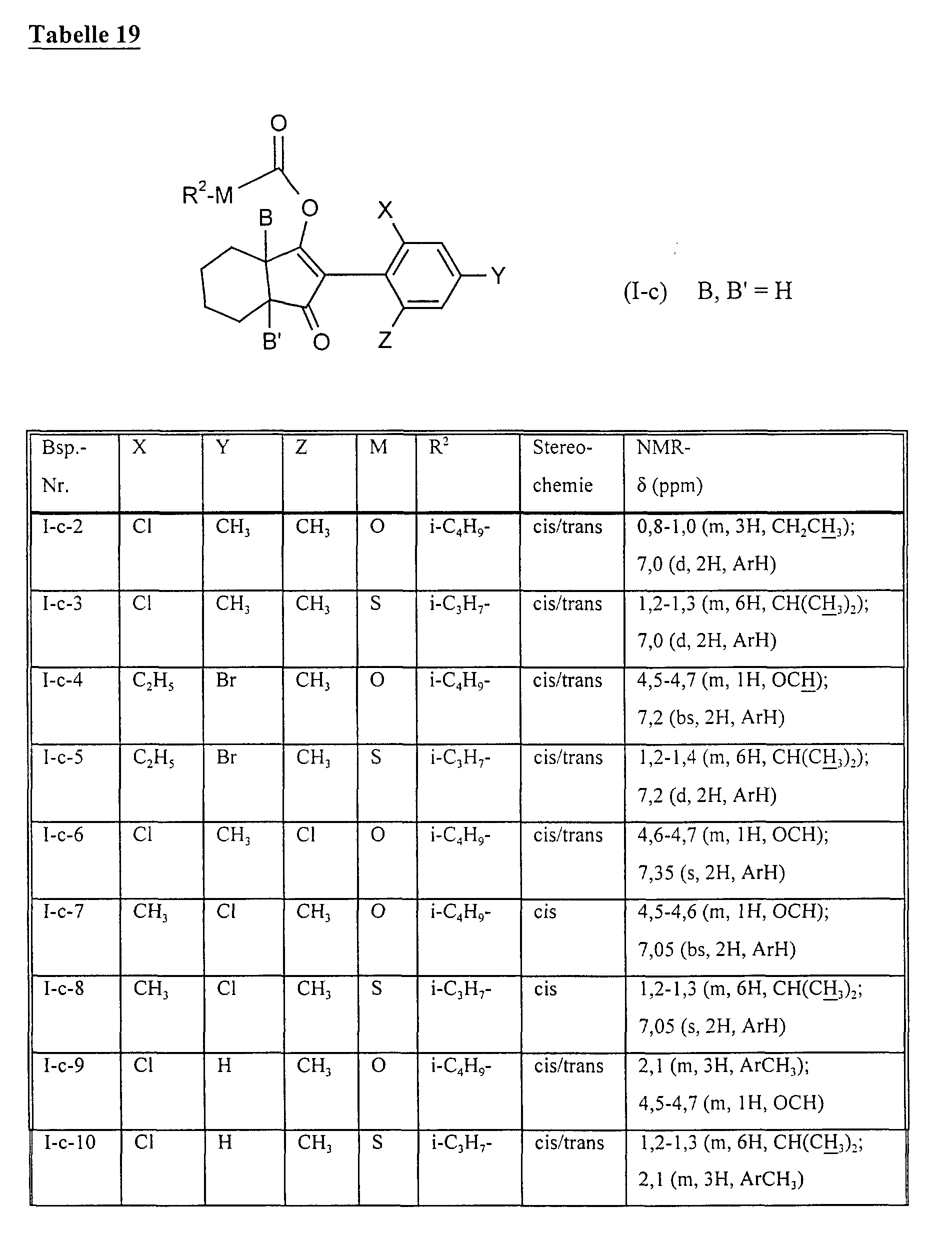

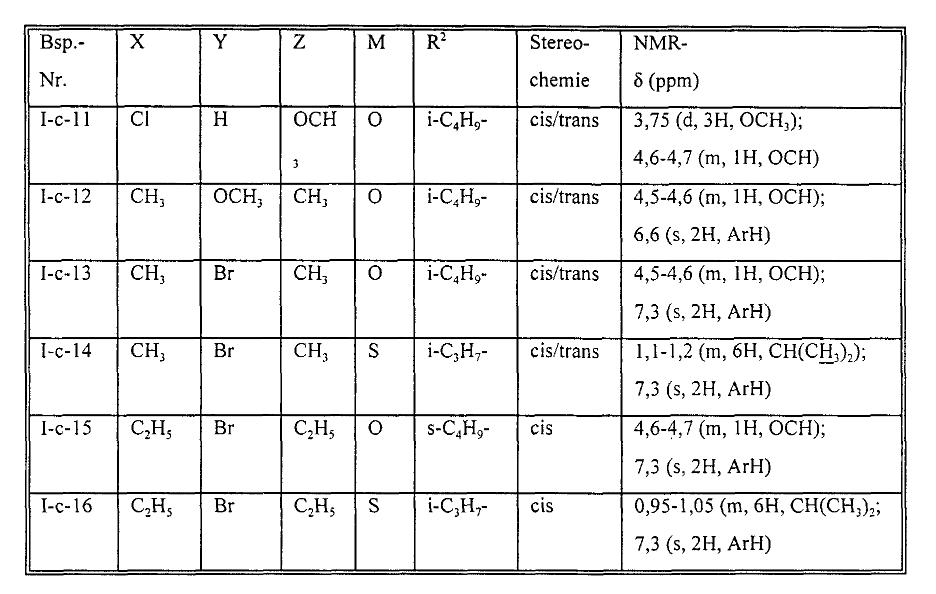

### Beispiel Id-1

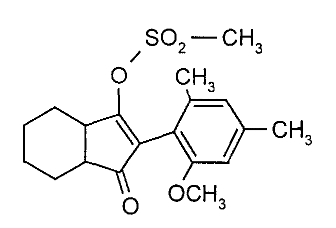

Zu 1,0 g (3,5 mmol) der Verbindung gemäß Beispiel (Ia-1) und 0,73 ml Triethylamin in 15 ml trockenem Methylenchlorid tropft man unter Eiskühlung eine Lösung von 0,35 ml (4,55 mmol) Methansulfonsäurechlorid in 3 ml trockenem Methylenchlorid. Man rührt 2 Stunden bei Raumtemperatur und arbeitet dann wie bei Beispiel (Ib-1) beschrieben auf. Der nach dem Einengen der organischen Phasen verbleibende Rückstand wird mit Cyclohexan/Essigester 3/1 verrührt und der Farbstoff abgesaugt und getrocknet.
Ausbeute: 0,50 g (39 % der Theorie); Fp.: 132°C.

### Herstellung der Ausgangsverbindungen

### Beispiel II-1

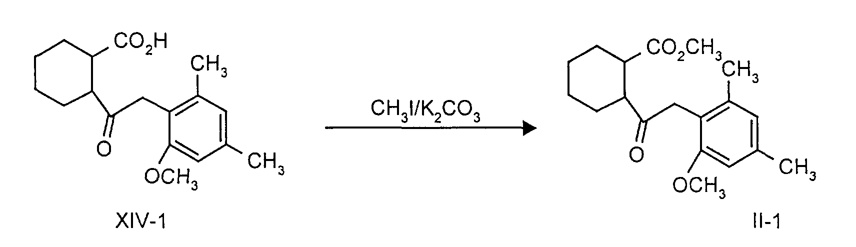

Eine Mischung der Verbindung gemäß Beispiel XIV-1 (33 g, 113 mmol), Kaliumcarbonat (46 g), Aceton (460 ml) und Iodmethan (46 ml) wird 5 Stunden unter Rückfluß gekocht, mit Ethylether (100 ml) verdünnt, über Kieselgel filtriert und eingeengt. Der Rückstand wird chromatographiert (Kieselgel, CH₂Cl₂:Petrolether 1:1). Man erhält 21 g farbloses Öl (60 %).
¹H-NMR (CDCl₃,δ ppm): 6.62 (bs, 1H); 6.53 (bs, 1H); 3.74 (s, 3H); 3.60 (s, 3H).

Analog zu Beispiel II-1 bzw. gemäß den allgemeinen Angaben zur Herstellung wurden die in der Tabelle 20 aufgeführten Verbindungen der Formel (II) hergestellt:

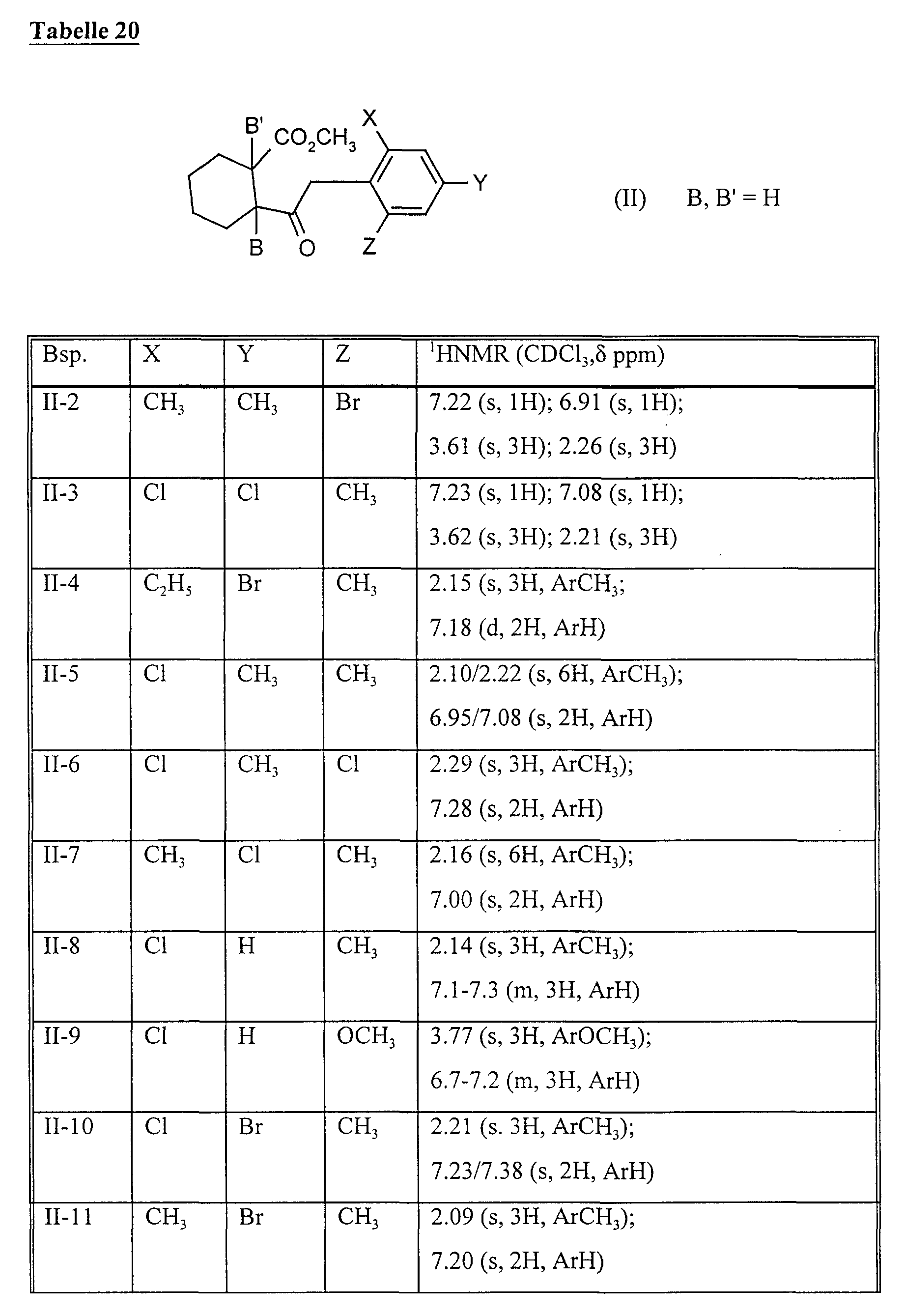

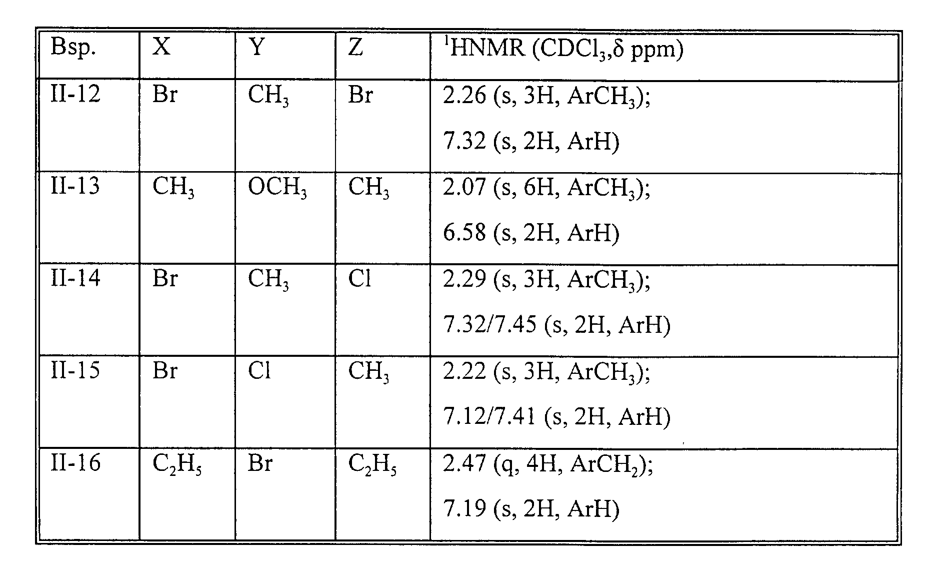

### Beispiel XIV-1

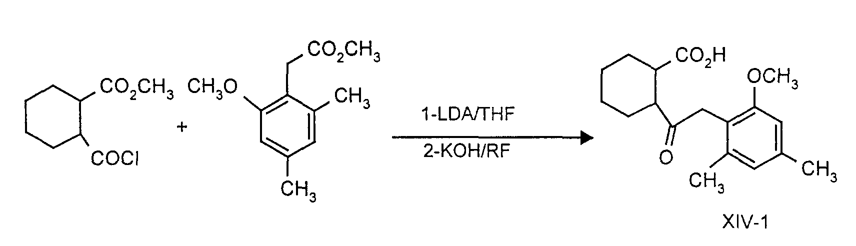

Zu einer Lösung von Lithiumdiisopropylamid (130 mmol) in Tetrahydrofuran (THF) (100 ml) gibt man 24,8 g (119 mmol) 2,4-Dimethyl-6-methoxyphenylessigsäuremethylester. Nach 30 Minuten bei Raumtemperatur gibt man 17g 3,4-Tetramethylenbernsteinsäuremethylesterchlorid zu und rührt bei Raumtemperatur (1 Stunde). Dann gibt man 100 ml Wasser und 30 g Ammoniumchlorid zu. Das Zwischenprodukt wird mit Ether extrahiert und über Kieselgel filtriert. Nach Einengen wird der Rückstand (Öl, 44 g) mit 88 g Kaliumhydroxid und 250 ml Wasser unter Rückfluß gekocht (2 Tage). Man kühlt ab, säuert an (konz. HCl), filtriert den Feststoff ab und erhält 33 g der oben gezeigten Verbindung XIV-1.
Farbloser Feststoff, Fp.: 128°C.

Analog zu Beispiel XIV-1 bzw. gemäß den allgemeinen Angaben zur Herstellung wurden die in der Tabelle 21 aufgeführten Verbindungen der Formel (XIV) hergestellt:

**Tabelle 21**

| Bsp. | X | Y | Z | ¹HNMR (CDCl₃,δ ppm) |
|---|---|---|---|---|
| XIV-3 | Cl | Cl | CH₃ | 3.85 (s, 2H); 2.80 (m, 2H) |
| XIV-2 | CH₃ | CH₃ | Br | 6.93 (s, 1H); 7.25 (s, 1H); 2.80 (m, 2H) |

Teilweise wurden die Verbindungen der Formel (XIV) 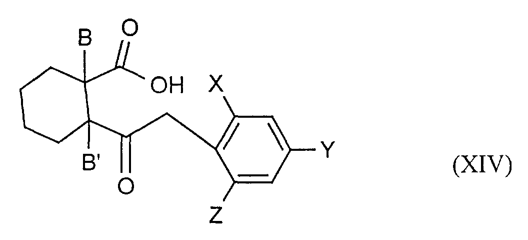 in welcher
- B, B', X, Y und Z: die oben angegebene Bedeutung haben,
als Rohprodukte in die Synthese zu den Verbindungen der Formel (II) eingesetzt.

### Anwendungsbeispiele

### Beispiel A

### Tetranychus-Test (resistent)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test bewirkte z.B. die Verbindung gemäß Herstellungsbeispiel Ia-1 bei einer beispielhaften Wirkstoffkonzentration von 0,01 % eine Abtötung von 100 % nach 13 Tagen.

### Beispiel B

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß Herstellungsbeispielen Ia-1, Ia-2 und Ia-3 bei einer beispielhaften Wirkstoffkonzentration von 0,1 % eine Abtötung von 100 % nach 7 Tagen.

### Beispiel C

### Plutella-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

ln diesem Test bewirkten z.B. die Verbindungen gemäß Herstellungsbeispielen Ia-1, Ia-2 und Ia-3 bei einer beispielhaften Wirkstoffkonzentration von 0,1 % eine Abtötung von 100 % nach 7 Tagen.

### Beispiel D

### Nephotettix-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß Herstellungsbeispielen Ia-1, Ia-2 und Ia-3 bei einer beispielhaften Wirkstoffkonzentration von 0,1 % eine Abtötung von 100 % nach 6 Tagen.

### Beispiel E

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

Bei einer beispielhaften Aufwandmenge von 60 g/ha zeigte in diesem Test beispielsweise die Verbindung gemäß Herstellungsbeispiel Ia-1 bei sehr guter Verträglichkeit durch Gerste und Baumwolle mindestens 90 % Wirkung gegenüber Alopecurus, Bromus, Sorghum und Matricaria.

Bei einer beispielhaften Aufwandmenge von 250 g/ha zeigte in diesem Test beispielsweise die Verbindung gemäß Herstellungsbeispiel Ia-2 100 % Wirkung gegenüber Alopecurus, Setaria und Sinapis.

Bei einer beispielhaften Aufwandmenge von 250 g/ha zeigte in diesem Test beispielsweise die Verbindung gemäß Herstellungsbeispiel Ia-3 bei sehr guter Verträglichkeit durch Zuckerrüben eine Wirkung von 80 % gegenüber Alopecurus.

### Beispiel F

### Myzus-Test

| | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen Ia-1, Ia-2 und Ia-3 bei einer beispielhaften Wirkstoffkonzentration von 0,1 % eine Abtötung von mindestens 90 % nach 6 Tagen.

## Patentansprüche

1. Verbindungen der Formel (I) 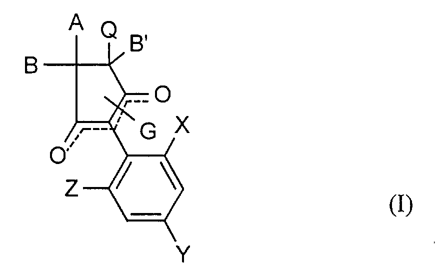 in welcher
X für Halogen, C₁-C₆-Alkyl, C₂-C₆-Alkenyl, C₁-C₆-Alkoxy, C₂-C₆-Alkenyloxy, C₁-C₆-Alkylthio, C₁-C₆-Alkylsulfinyl, C₁-C₆-Alkylsulfonyl, C₁-C₆-Halogenalkyl, C₁-C₆-Halogenalkoxy, C₂-C₆-Halogenalkenyloxy, Nitro, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Benzyloxy oder Benzylthio steht,
Y für Wasserstoff, Halogen, C₁-C₆-Alkyl, C₂-C₆-Alkenyl, C₁-C₆-Alkoxy, C₂-C₆-Alkenyloxy, C₁-C₆-Alkylthio, C₁-C₆-Alkylsulfinyl, C₁-C₆-Alkylsulfonyl, C₁-C₆-Halogenalkyl, C₁-C₆-Halogenalkoxy, C₂-C₆-Halogenalkenyloxy, Nitro oder Cyano steht,
Z für Halogen, C₁-C₆-Alkyl, C₂-C₆-Alkenyl, C₁-C₆-Alkoxy, C₂-C₆-Alkenyloxy, C₁-C₆-Halogenalkyl, C₁-C₆-Halogenalkoxy, C₂-C₆-Halogenalkenyloxy, Nitro oder Cyano steht, wobei X, Y und Z nicht gleichzeitig für Methyl stehen,
A und Q gemeinsam für jeweils gegebenenfalls einfach bis dreifach, gleich oder verschieden durch Halogen, Hydroxy, Mercapto, durch jeweils gegebenenfalls einfach bis neunfach, gleich oder verschieden durch Halogen substituiertes C₁-C₁₀-Alkyl, C₁-C₆-Alkoxy, C₁-C₆-Alkylthio, C₃-C₇-Cycloalkyl oder durch jeweils gegebenenfalls einfach bis fünffach, gleich oder verschieden durch Halogen, C₁-C₆-Alkyl oder C₁-C₆-Alkoxy substituiertes Benzyloxy oder Phenyl substituiertes C₁-C₆-Alkandiyl oder C₂-C₆-Alkendiyl stehen, welches außerdem gegebenenfalls eine der nachstehenden Gruppen 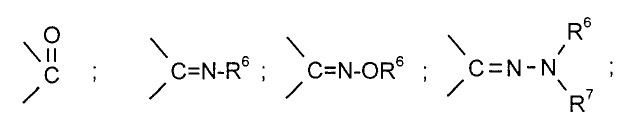 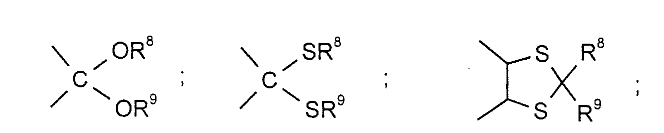 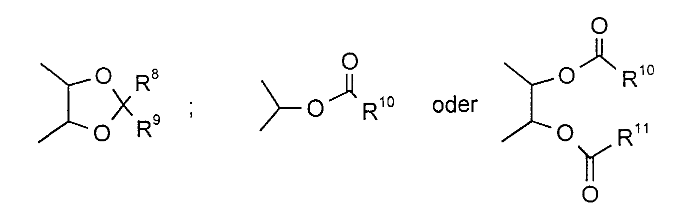 enthält oder durch eine C₁-C₂-Alkandiylgruppe überbrückt ist,
B und B' unabhängig voneinander bevorzugt für Wasserstoff, Halogen oder C₁-C₆-Alkyl oder gemeinsam für jeweils gegebenenfalls durch C₁-C₆-Alkyl substituiertes C₁-C₆-Alkandiyl oder C₂-C₄-Alkendiyl stehen,
G für Wasserstoff (a) oder für eine der Gruppen 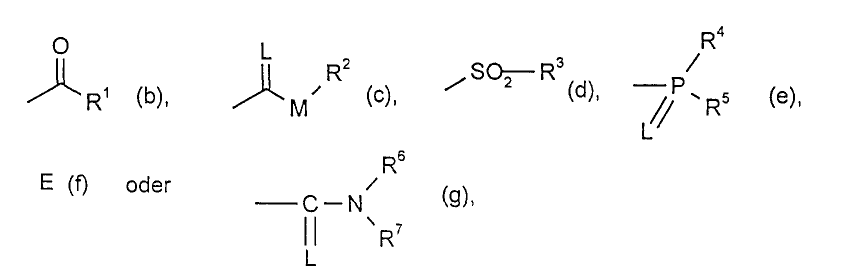 steht, in welchen
E für ein Metallion oder ein Ammoniumion steht,
L für Sauerstoff oder Schwefel steht und
M für Sauerstoff oder Schwefel steht,
R¹ für jeweils gegebenenfalls einfach oder mehrfach, gleich oder verschieden durch Halogen substituiertes C₁-C₂₀-Alkyl, C₂-C₁₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder für gegebenenfalls einfach oder mehrfach, gleich oder verschieden durch Halogen, C₁-C₆-Alkyl oder C₁-C₆-Alkoxy substituiertes C₃-C₈-Cycloalkyl, in welchem mindestens eine Methylengruppe durch ein Sauerstoff- und/oder Schwefelatom ersetzt sein kann,
für gegebenenfalls einfach oder mehrfach, gleich oder verschieden durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl,
für gegebenenfalls einfach oder mehrfach, gleich oder verschieden durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls einfach oder mehrfach, gleich oder verschieden durch Halogen oder C₁-C₆-Alkyl substituiertes Hetaryl mit 5 oder 6 Ringatomen und ein bis drei Heteroatomen aus der Reihe Sauerstoff, Schwefel und Stickstoff,
für gegebenenfalls einfach oder mehrfach, gleich oder verschieden durch Halogen oder C₁-C₆-Alkyl substituiertes Phenoxy-C₁-C₆-alkyl oder
für gegebenenfalls einfach oder mehrfach, gleich oder verschieden durch Halogen, Amino oder C₁-C₆-Alkyl substituiertes Hetaryloxy-C₁-C₆-alkyl mit 5 oder 6 Ringatomen und ein bis drei Heteroatomen aus der Reihe Sauerstoff, Schwefel und Stickstoff steht,
R² für jeweils gegebenenfalls einfach oder mehrfach, gleich oder verschieden durch Halogen substituiertes C₁-C₂₀-Alkyl, C₂-C₁₀-Alkenyl, C₁-C₈-Alkoxy-C₂-C₈-alkyl oder Poly-C₁-C₈-alkoxy-C₂-C₈-alkyl,
für gegebenenfalls einfach oder mehrfach, gleich oder verschieden durch Halogen, C₁-C₆-Alkyl oder C₁-C₆-Alkoxy substituiertes C₃-C₆-Cycloalkyl oder
für jeweils gegebenenfalls einfach oder mehrfach, gleich oder verschieden durch Halogen, Nitro, Cyano, C₁-C₆-Alkyl, C₁-C₆-Alkoxy, C₁-C₃-Halogenalkoxy oder C₁-C₃-Halogenalkyl substituiertes Phenyl oder Benzyl steht,
R³ für jeweils gegebenenfalls einfach oder mehrfach, gleich oder verschieden durch Halogen substituiertes C₁-C₁₂-Alkyl oder für jeweils gegebenenfalls einfach oder mehrfach, gleich oder verschieden durch Halogen, C₁-C₆-Alkyl, C₁-C₆-Alkoxy, C₁-C₃-Halogenalkyl, C₁-C₃-Halogenalkoxy, Cyano oder Nitro substituiertes Phenyl oder Phenyl-C₁-C₄-alkyl steht,
R⁴ und R⁵ unabhängig voneinander für jeweils gegebenenfalls einfach oder mehrfach, gleich oder verschieden durch Halogen substituiertes C₁-C₈-Alkyl, C₁-C₈-Alkoxy, C₁-C₈-Alkylamino, Di-(C₁-C₈-alkyl)-amino, C₁-C₈-Alkylthio, C₃-C₅-Alkenylthio, C₃-C₇-Cycloalkylthio oder für jeweils gegebenenfalls einfach oder mehrfach, gleich oder verschieden durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ für Wasserstoff, jeweils gegebenenfalls einfach oder mehrfach, gleich oder verschieden durch Halogen substituiertes C₁-C₁₀-Alkyl, C₃-C₈-Alkenyl, C₁-C₈-Alkoxy-C₂-C₈-alkyl, für gegebenenfalls einfach oder mehrfach, gleich oder verschieden durch Halogen, C₁-C₆-Alkyl, C₁-C₆-Alkoxy, C₁-C₃-Halogenalkyl oder C₁-C₃-Halogenalkoxy substituiertes C₃-C₁₀-Cycloalkyl, für gegebenenfalls einfach oder mehrfach, gleich oder verschieden durch Halogen, C₁-C₃-Halogenalkyl, C₁-C₈-Alkyl, C₁-C₃-Halogenalkoxy oder C₁-C₈-Alkoxy substituiertes Phenyl oder für gegebenenfalls einfach oder mehrfach, gleich oder verschieden durch Halogen, C₁-C₈-Alkyl, C₁-C₃-Halogenalkyl, C₁-C₃-Halogenalkoxy oder C₁-C₈-Alkoxy substituiertes Benzyl steht,
R⁷ für Wasserstoff oder für jeweils gegebenenfalls einfach oder mehrfach, gleich oder verschieden durch Halogen substituiertes C₁-C₁₀-Alkyl oder C₃-C₁₀-Alkenyl steht oder
R⁶ und R⁷ gemeinsam mit dem N-Atom, an das sie gebunden sind, für einen gegebenenfalls Sauerstoff oder Schwefel enthaltenden und gegebenenfalls durch C₁-C₆-Alkyl substituierten 3- bis 7-gliedrigen Ring stehen,
R⁸ und R⁹ unabhängig voneinander für Wasserstoff, gegebenenfalls einfach oder mehrfach, gleich oder verschieden durch Halogen substituiertes C₁-C₆-Alkyl oder für jeweils gegebenenfalls einfach oder mehrfach, gleich oder verschieden durch Halogen, C₁-C₆-Alkyl, C₁-C₆-Alkoxy, C₁-C₆-Halogenalkyl, C₁-C₆-Halogenalkoxy, Nitro oder Cyano substituiertes Phenyl oder Phenyl-C₁-C₄-alkyl,
oder zusammen für gegebenenfalls einfach oder mehrfach, gleich oder verschieden durch Halogen, C₁-C₆-Alkyl, C₁-C₆-Alkoxy oder C₁-C₃-Halogenalkyl substituiertes C₂-C₆-Alkandiyl stehen und
R¹⁰ und R¹¹ unabhängig voneinander für jeweils gegebenenfalls einfach oder mehrfach, gleich oder verschieden durch Halogen substituiertes C₁-C₁₀-Alkyl, C₂-C₁₀-Alkenyl, C₁-C₁₀-Alkoxy, C₁-C₁₀-Alkylamino, Di-(C₁-C₁₀-alkyl)-amino, C₃-C₁₀-Alkenylamino, Di-(C₃-C₁₀-alkenyl)-amino oder jeweils einfach oder mehrfach, gleich oder verschieden durch Halogen, C₁-C₄-Alkyl, C₁-C₄-Alkoxy, C₁-C₄-Halogenalkyl, Nitro oder Cyano substituiertes Phenyl oder Benzyl stehen.

2. Verbindungen der Formel (I) gemäß Anspruch 1, in welcher
X für Fluor, Chlor, Brom, C₁-C₄-Alkyl, C₂-C₄-Alkenyl, C₁-C₄-Alkoxy, C₂-C₄-Alkenyloxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₂-C₄-Halogenalkenyloxy, Nitro, Cyano oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Phenylthio, Benzyloxy oder Benzylthio steht,
Y für Wasserstoff, Fluor, Chlor, Brom, C₁-C₄-Alkyl, C₂-C₄-Alkenyl, C₁-C₄-Alkoxy, C₂-C₄-Alkenyloxy, C₁-C₄-Alkylthio, C₁-C-Alkylsulfinyl, C₁-C₄-Alkylsulfonyl, C₁-C₄-Halogenalkyl, C₁-C₄-Halogenalkoxy, C₂-C₄-Halogenalkenyloxy, Nitro oder Cyano steht,
Z für Fluor, Chlor, Brom, C₁-C₄-Alkyl, C₂-C₄-Alkenyl, C₁-C₄-Alkoxy, C₂-C₄-Alkenyloxy, C₁-C₄-Halogenalkyl, C₁-C₄-Halogenalkoxy, C₂-C₄-Halogenalkenyloxy, Nitro oder Cyano steht,
wobei X, Y und Z nicht gleichzeitig für Methyl stehen,
A und Q gemeinsam für jeweils gegebenenfalls einfach oder zweifach, gleich oder verschieden durch Fluor, Chlor, Brom, Hydroxy, Mercapto, durch jeweils gegebenenfalls einfach bis fünffach, gleich oder verschieden durch Fluor oder Chlor substituiertes C₁-C₈-Alkyl, C₁-C₄-Alkoxy, C₁-C₄-Alkylthio, C₅-C₇-Cycloalkyl oder Phenyl substituiertes C₁-C₅-Alkandiyl oder C₂-C₅-Alkendiyl stehen, welches außerdem gegebenenfalls eine der nachstehenden Gruppierungen 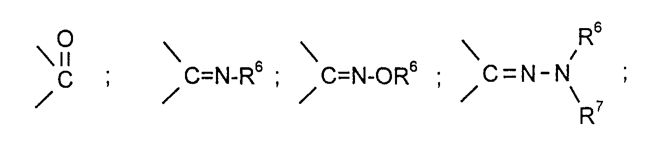 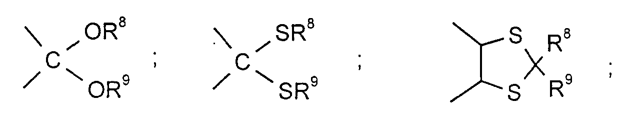 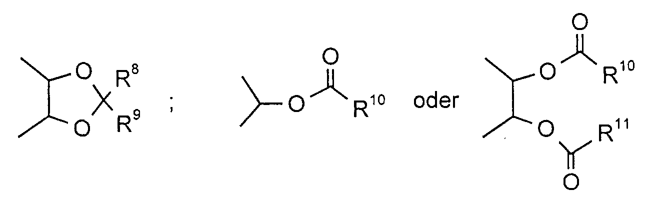 enthält oder durch eine C₁-C₂-Alkandiylgruppe überbrückt ist,
B und B' unabhängig voneinander für Wasserstoff, Fluor, Chlor oder C₁-C₄-Alkyl oder gemeinsam für jeweils gegebenenfalls durch C₁-C₄-Alkyl substituiertes C₁-C₅-Alkandiyl oder C₂-C₄-Alkendiyl stehen,
G für Wasserstoff (a) oder für eine der Gruppen 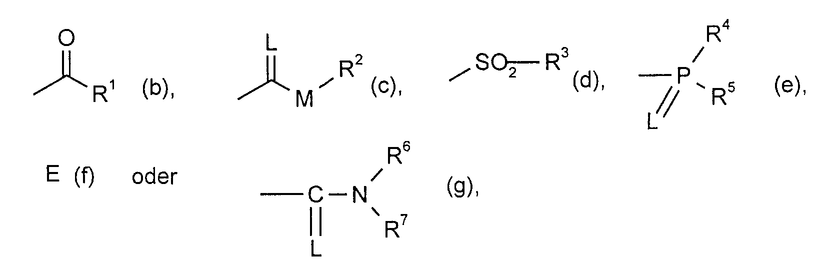 steht, in welchen
E für ein Metallion oder ein Ammoniumion steht,
L für Sauerstoff oder Schwefel steht und
M für Sauerstoff oder Schwefel steht.
R¹ für jeweils gegebenenfalls einfach bis neunfach, gleich oder verschieden durch Fluor oder Chlor substituiertes C₁-C₁₆-Alkyl, C₂-C₈-Alkenyl, C₁-C₆-Alkoxy-C₁-C₆-alkyl, C₁-C₆-Alkylthio-C₁-C₆-alkyl, Poly-C₁-C₆-alkoxy-C₁-C₆-alkyl oder für gegebenenfalls einfach bis dreifach, gleich oder verschieden durch Fluor, Chlor, C₁-C₄-Alkyl oder C₁-C₄-Alkoxy substituiertes C₃-C₇-Cycloalkyl, in welchem eine oder zwei nicht direkt benachbarte Methylengruppen durch Sauerstoff- und/oder Schwefelatome ersetzt sein können,
für gegebenenfalls einfach bis dreifach, gleich oder verschieden durch Fluor, Chlor, Brom, Cyano, Nitro, C₁-C₄-Alkyl, C₁-C₄-Alkoxy, C₁-C₃-Halogenalkyl oder C₁-C₃-Halogenalkoxy substituiertes Phenyl,
für gegebenenfalls einfach bis dreifach, gleich oder verschieden durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach oder zweifach, gleich oder verschieden durch Fluor, Chlor, Brom oder C₁-C₄-Alkyl substituiertes Furanyl, Thienyl, Pyridyl, Pyrimidyl, Thiazolyl oder Pyrazolyl,
für gegebenenfalls einfach bis dreifach, gleich oder verschieden durch Fluor, Chlor oder C₁-C₄-Alkyl substituiertes Phenoxy-C₁-C₅-alkyl oder
für jeweils gegebenenfalls einfach oder zweifach, gleich oder verschieden durch Fluor, Chlor, Amino oder C₁-C₄-Alkyl substituiertes Pyridyloxy-C₁-C₆-alkyl, Pyrimidinyloxy-C₁-C₆-alkyl oder Thiazolyloxy-C₁-C₆-alkyl steht,
R² für jeweils gegebenenfalls einfach bis siebenfach, gleich oder verschieden durch Fluor oder Chlor substituiertes C₁-C₁₆-Alkyl, C₂-C₈-Alkenyl, C₁-C₆-Alkoxy-C₂-C₆-alkyl oder Poly-C₁-C₆-alkoxy-C₂-C₆-alkyl,
für gegebenenfalls einfach bis dreifach, gleich oder verschieden durch Fluor, Chlor, C₁-C₄-Alkyl oder C₁-C₄-Alkoxy substituiertes C₃-C₆-Cycloalkyl oder
für jeweils gegebenenfalls einfach bis dreifach, gleich oder verschieden durch Fluor, Chlor, Brom, Nitro, Cyano, C₁-C₄-Alkyl, C₁-C₄-Alkoxy, C₁-C₂-Halogenalkoxy oder C₁-C₂-Halogenalkyl substituiertes Phenyl oder Benzyl steht,
R³ für gegebenenfalls einfach bis fünffach, gleich oder verschieden durch Fluor oder Chlor substituiertes C₁-C₉-Alkyl oder für jeweils gegebenenfalls einfach bis dreifach, gleich oder verschieden durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl oder Phenyl-C₁-C₂-alkyl steht,
R⁴ und R⁵ unabhängig voneinander für gegebenenfalls einfach bis fünffach, gleich oder verschieden durch Fluor oder Chlor substituiertes C₁-C₆-Alkyl, C₁-C₆-Alkoxy, C₁-C₆-Alkylamino, Di-(C₁-C₆-alkyl)-amino, C₁-C₆-Alkylthio, C₃-C₄-Alkenylthio, C₃-C₆-Cycloalkylthio oder für jeweils gegebenenfalls einfach bis dreifach, gleich oder verschieden durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Al-kyl oder C₁-C₃-Halogenalkyl substituiertes Phenyl, Phenoxy oder Phenylthio stehen,
R⁶ für Wasserstoff, jeweils gegebenenfalls einfach bis fünffach, gleich oder verschieden durch Fluor oder Chlor substituiertes C₁-C₈-Alkyl, C₃-C₆-Alkenyl, C₁-C₆-Alkoxy-C₂-C₆-alkyl, für gegebenenfalls einfach bis dreifach, gleich oder verschieden durch Fluor, Chlor, C₁-C₄-Alkyl, C₁-C₄-Alkoxy, C₁-C₂-Halogenalkyl oder C₁-C₂-Halogenalkoxy substituiertes C₃-C₈-Cycloalkyl, für gegebenenfalls einfach bis dreifach, gleich oder verschieden durch Fluor, Chlor, Brom, C₁-C₂-Halogenalkyl, C₁-C₅-Alkyl, C₁-C₂-Halogenalkoxy oder C₁-C₅-Alkoxy substituiertes Phenyl oder für gegebenenfalls einfach bis dreifach, gleich oder verschieden durch Fluor, Chlor, Brom, C₁-C₅-Alkyl, C₁-C₂-Halogenalkyl, C₁-C₂-Halogenalkoxy oder C₁-C₅-Alkoxy substituiertes Benzyl steht,
R⁷ für Wasserstoff oder für jeweils gegebenenfalls einfach bis fünffach, gleich oder verschieden durch Fluor oder Chlor substituiertes C₁-C₈-Alkyl oder C₃-C₈-Alkenyl steht oder
R⁶ und R⁷ gemeinsam mit dem N-Atom, an das sie gebunden sind, für einen gegebenenfalls Sauerstoff oder Schwefel enthaltenden und gegebenenfalls durch C₁-C₄-Alkyl substituierten 4- bis 7-gliedrigen Ring stehen,
R⁸ und R⁹ unabhängig voneinander für Wasserstoff, gegebenenfalls einfach bis fünffach, gleich oder verschieden durch Fluor oder Chlor substituiertes C₁-C₄-Alkyl oder für gegebenenfalls einfach bis dreifach, gleich oder verschieden durch Fluor, Chlor, C₁-C₄-Alkyl, C₁-C₄-Alkoxy, C₁-C₂-Halogenalkyl, C₁-C₂-Halogenalkoxy, Nitro oder Cyano substituiertes Phenyl oder zusammen für gegebenenfalls einfach bis dreifach, gleich oder verschieden durch Fluor, Chlor, C₁-C₄-Alkyl, C₁-C₄-Alkoxy oder C₁-C₂-Halogenalkyl substituiertes C₂-C₅-Alkandiyl stehen und
R¹⁰ und R¹¹ unabhängig voneinander für jeweils gegebenenfalls einfach bis fünffach, gleich oder verschieden durch Fluor oder Chlor substituiertes C₁-C₈-Alkyl, C₂-C₈-Alkenyl, C₁-C₈-Alkoxy, C₁-C₈-Alkylamino, C₃-C₈-Alkenylamino, Di-(C₁-C₈-alkyl)-amino oder Di-(C₃-C₈-alkenyl)-amino stehen.

3. Verbindungen der Formel (I) gemäß Anspruch 1, in welcher
X für Fluor, Chlor, Brom, Methyl, Ethyl, Propyl, iso-Propyl, Ethenyl, 1-Propenyl, Methoxy, Ethoxy, Propoxy, iso-Propoxy, Allyloxy, Methallyloxy, Trifluormethyl, Difluormethoxy, Trifluormethoxy, Trifluorethoxy, Methylthio, Methylsulfinyl, Methylsulfonyl, Nitro, Cyano, oder jeweils gegebenenfalls durch Fluor, Chlor, Brom, Methyl, Ethyl, Propyl, iso-Propyl, tert.-Butyl, Methoxy, Ethoxy, Propoxy, tert.-Butoxy, Trifluormethyl, Trifluormethoxy, Nitro oder Cyano substituiertes Phenyl, Phenoxy, Phenylthio, Benzyloxy oder Benzylthio steht,
Y für Wasserstoff, Fluor, Chlor, Brom, Methyl, Ethyl, n-Propyl, i-Propyl, n-Butyl, i-Butyl, tert.-Butyl, Ethenyl, 1-Propenyl, Methoxy, Ethoxy, Propoxy, iso-Propoxy, Allyloxy, Methallyloxy, Trifluormethyl, Methylthio, Methylsulfinyl, Methylsulfonyl, Difluormethoxy, Trifluormethoxy, Trifluorethoxy, Nitro oder Cyano steht,
Z für Fluor, Chlor, Brom, Methyl, Ethyl, n-Propyl, i-Propyl, n-Butyl, i-Butyl, tert.-Butyl, Ethenyl, 1-Propenyl, Methoxy, Ethoxy, Propoxy, iso-Propoxy, Allyloxy, Methallyloxy, Trifluormethyl, Difluormethoxy, Trifluormethoxy, Trifluorethoxy, Nitro oder Cyano steht,
wobei X, Y und Z nicht gleichzeitig für Methyl stehen,
A und Q gemeinsam für jeweils gegebenenfalls einfach oder zweifach, gleich oder verschieden durch Fluor, Chlor, Hydroxy, durch jeweils gegebenenfalls einfach bis dreifach, gleich oder verschieden durch Fluor oder Chlor substituiertes C₁-C₆-Alkyl oder C₁-C₂-Alkoxy substituiertes C₁-C₄-Alkandiyl oder C₂-C₄-Alkendiyl stehen,
B und B' unabhängig voneinander für Wasserstoff, Methyl oder Ethyl stehen,
G für Wasserstoff (a) oder für eine der Gruppen 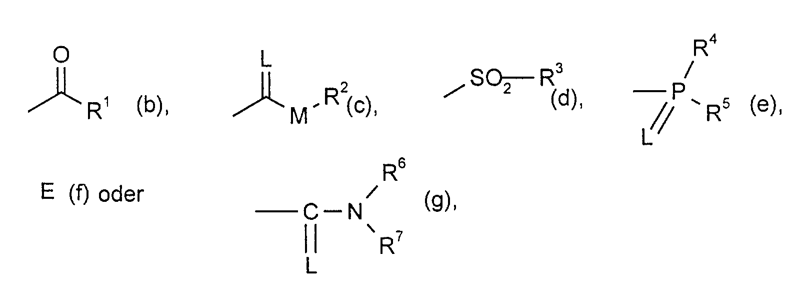 steht, in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis fünffach, gleich oder verschieden durch Fluor oder Chlor substituiertes C₁-C₁₄-Alkyl, C₂-C₆-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls einfach oder zweifach, gleich oder verschieden durch Fluor, Chlor, Methyl, Ethyl, Methoxy oder Ethoxy substituiertes C₃-C₆-Cycloalkyl, in welchem eine oder zwei nicht direkt benachbarte Methylengruppen durch Sauerstoff- und/oder Schwefelatome ersetzt sein können,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl, Trifluormethoxy, Cyano oder Nitro substituiertes Phenyl,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl oder Trifluormethoxy substituiertes Benzyl,
für jeweils gegebenenfalls einfach oder zweifach, gleich oder verschieden durch Fluor, Chlor, Brom, Methyl oder Ethyl substituiertes Thienyl, Furanyl oder Pyridyl,
für gegebenenfalls einfach oder zweifach, gleich oder verschieden durch Fluor, Chlor, Methyl oder Ethyl substituiertes Phenoxy-C₁-C₄-alkyl oder
für jeweils gegebenenfalls einfach oder zweifach, gleich oder verschieden durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl steht,
R² für jeweils gegebenenfalls einfach bis fünffach, gleich oder verschieden durch Fluor oder Chlor substituiertes C₁-C₁₄-Alkyl, C₂-C₆-Alkenyl, C₁-C₄-Alkoxy-C₂-C₆-alkyl oder Poly-C₁-C₄-alkoxy-C₂-C₆-alkyl, für gegebenenfalls einfach bis dreifach, gleich oder verschieden durch Fluor, Chlor, Methyl, Ethyl, Methoxy oder Ethoxy substituiertes C₃-C₆-Cycloalkyl
oder für jeweils gegebenenfalls einfach oder zweifach, gleich oder verschieden durch Fluor, Chlor, Nitro, Cyano, Methyl, Ethyl, Propyl, i-Propyl, Methoxy, Ethoxy, Trifluormethoxy oder Trifluormethyl substituiertes Phenyl oder Benzyl steht,
R³ für gegebenenfalls einfach bis dreifach, gleich oder verschieden durch Fluor oder Chlor substituiertes C₁-C₆-Alkyl oder für jeweils gegebenenfalls einfach oder zweifach durch Fluor, Chlor, Brom, Methyl, Ethyl, Methoxy, Ethoxy, Trifluormethyl, Trifluormethoxy, Cyano oder Nitro substituiertes Phenyl oder Benzyl steht,
R⁴ und R⁵ unabhängig voneinander für jeweils gegebenenfalls einfach bis dreifach, gleich oder verschieden durch Fluor oder Chlor substituiertes C₁-C₄-Alkyl, C₁-C₄-Alkoxy, C₁-C₄-Alkylamino, Di-(C₁-C₄-alkyl)-amino, C₁-C₄-Alkylthio oder für jeweils gegebenenfalls einfach oder zweifach, gleich oder verschieden durch Fluor, Chlor, Brom, Nitro, Cyano, C₁-C₂-Alkoxy, Trifluormethoxy, C₁-C₂-Alkylthio, Trifluormethyl oder C₁-C₃-Alkyl substituiertes Phenyl, Phenoxy oder Phenylthio stehen,
R⁶ für Wasserstoff, jeweils gegebenenfalls einfach bis dreifach, gleich oder verschieden durch Fluor oder Chlor substituiertes C₁-C₆-Alkyl, C₁-C₆-Alkoxy-C₂-C₄-alkyl, für gegebenenfalls einfach oder zweifach, gleich oder verschieden durch Fluor, Chlor, Methyl, Methoxy, Trifluormethyl oder Trifluormethoxy substituiertes C₃-C₆-Cycloalkyl, für gegebenenfalls einfach oder zweifach, gleich oder verschieden durch Fluor, Chlor, Trifluormethyl, C₁-C₄-Alkyl, Trifluormethoxy oder C₁-C₄-Alkoxy substituiertes Phenyl oder für gegebenenfalls einfach oder zweifach, gleich oder verschieden durch Fluor, Chlor, C₁-C₄-Alkyl, Trifluormethyl, Trifluormethoxy oder C₁-C₄-Alkoxy substituiertes Benzyl steht,
R⁷ für Wasserstoff, jeweils gegebenenfalls einfach bis dreifach, gleich oder verschieden durch Fluor oder Chlor substituiertes C₁-C₆-Alkyl oder C₃-C₆-Alkenyl steht oder
R⁶ und R⁷ gemeinsam mit dem N-Atom, an das sie gebunden sind, für einen gegebenenfalls Sauerstoff oder Schwefel enthaltenden, gegebenenfalls durch Methyl substituierten 5- bis 7-gliedrigen Ring stehen.

4. Verfahren zur Herstellung von Verbindungen der Formel (I) gemäß Anspruch 1, **dadurch gekennzeichnet, daß** man
(A) Verbindungen der Formel (Ia) 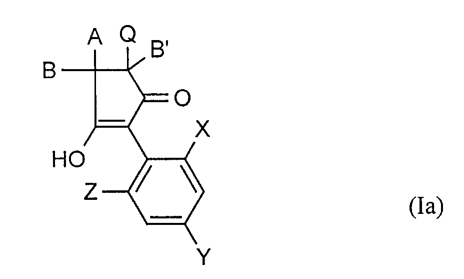 in welcher
A, B, B', Q, X, Y und Z die in Anspruch 1 angegebene Bedeutung haben,
erhält,
wenn man Verbindungen der Formel (II) 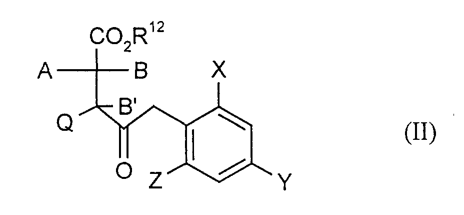 in welcher
A, B, B' Q, X, Y und Z die oben angegebene Bedeutung haben, und
R¹² für Alkyl steht,
in Gegenwart eines Verdünnungsmittels und in Gegenwart einer Base intramolekular kondensiert;
(B) Verbindungen der Formel (Ib) 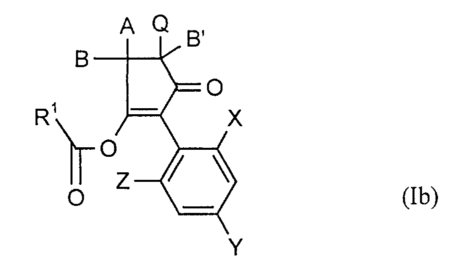 in welcher
A, B, B', Q, X, Y, Z und R¹ die in Anspruch 1 angegebene Bedeutung haben,
erhält,
wenn man Verbindungen der Formel (Ia), 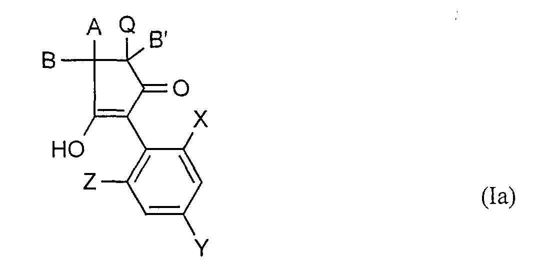 in welcher
A, B, B', X, Y, Z und Q die oben angegebene Bedeutung haben,
α) mit Säurehalogeniden der Formel (III) 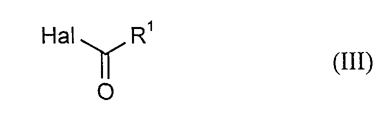 in welcher
R¹ die oben angegebene Bedeutung hat und
Hal für Halogen steht,
gegebenenfalls in Gegenwart eines Verdünnungsmittels und gegebenenfalls in Gegenwart eines Säurebindemittels umsetzt
oder
β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels umsetzt;
(C) Verbindungen der Formel (Ic-1) 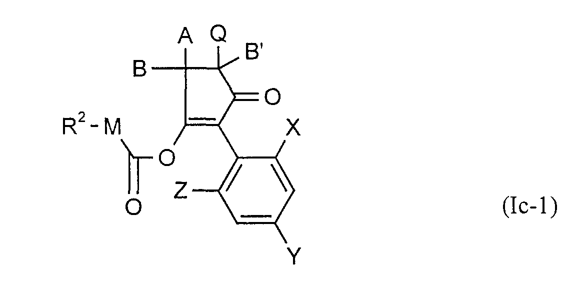 in welcher
A, B, B', Q, X, Y, Z und R² die in Anspruch 1 angegebene Bedeutung haben,
und
M für Sauerstoff oder Schwefel steht,
erhält,
wenn man Verbindungen der Formel (Ia) 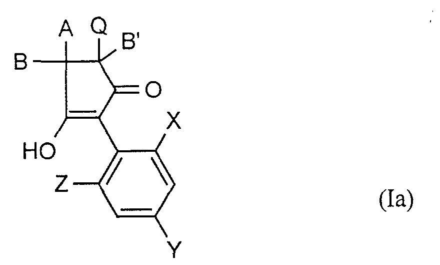 in welcher
A, B, B', Q, X, Y und Z die oben angegebene Bedeutung haben,
mit Chlorameisensäureestern oder Chlorameisensäurethiolestern der Formel (V)
R²-M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt;
(D) Verbindungen der Formel (Ic-2) 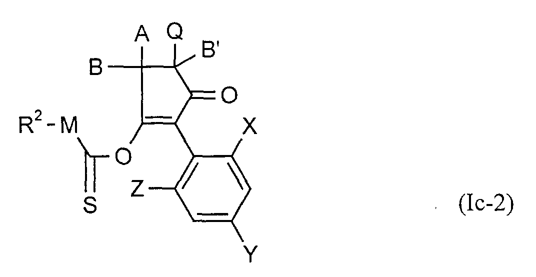 in welcher
A, B, B', Q, X, Y, Z und R² die in Anspruch 1 angegebene Bedeutung haben
und
M für Sauerstoff oder Schwefel steht,
erhält,
wenn man Verbindungen der Formel (Ia) 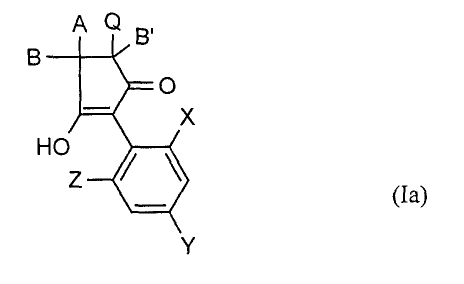 in welcher
A, B, B', Q, X, Y und Z die oben angegebene Bedeutung haben,
α) mit Chlormonothioameisensäureestern oder Chlordithioameisensäureestern der Formel (VI) 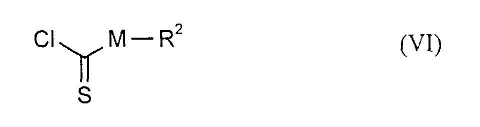 in welcher
M und R² die oben angegebene Bedeutung haben,
gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der allgemeinen Formel (VII)
R²-Hal (VII)
in welcher
R² die oben angegebene Bedeutung hat
und
Hal für Chlor, Brom oder Iod steht,
gegebenenfalls in Gegenwart eines Verdünnungsmittels und gegebenenfalls in Gegenwart einer Base umsetzt;
(E) Verbindungen der Formel (Id) 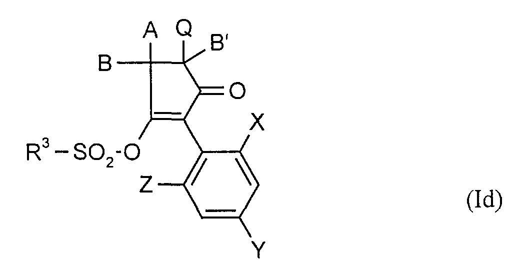 in welcher
A, B, B', Q, X, Y, Z und R³ die in Anspruch 1 angegebene Bedeutung haben,
erhält,
wenn man Verbindungen der Formel (Ia) 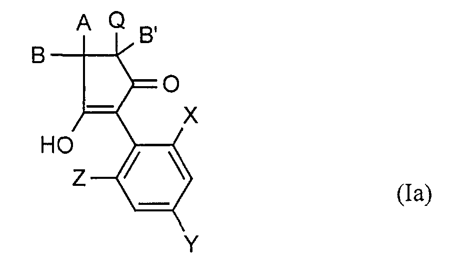 in welcher
A, B, B', Q, X, Y und Z die oben angegebene Bedeutung haben,
mit Sulfonsäurechloriden der Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels umsetzt;
(F) Verbindungen der Formel (Ie) 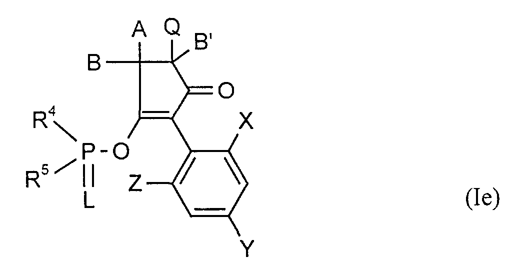 in welcher
A, B, L, B', Q, X, Y, Z, R⁴ und R⁵ die in Anspruch 1 angegebene Bedeutung haben,
erhält,
wenn man Verbindungen der Formel (Ia) bzw. deren Enole 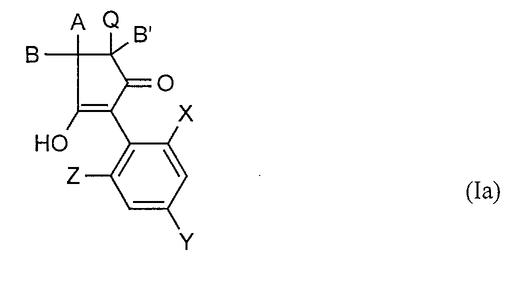 in welcher
A, B, B' Q, X, Y und Z die oben angegebene Bedeutung haben,
mit Phosphorverbindungen der Formel (IX) 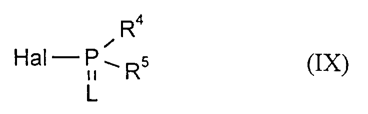 in welcher
L, R⁴ und R⁵ die oben angegebene Bedeutung haben
und
Hal für Halogen steht,
gegebenenfalls in Gegenwart eines Verdünnungsmittels und gegebenenfalls in Gegenwart eines Säurebindemittels umsetzt;
(G) Verbindungen der Formel (If) 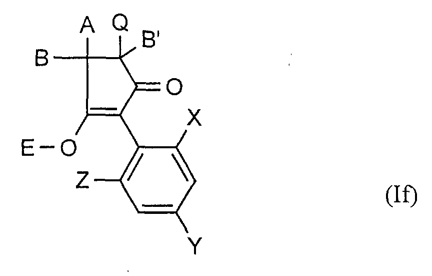 in welcher
A, B, B' Q, X, Y und Z die in Anspruch 1 angegebene Bedeutung haben,
und
E für ein Metallionäquivalent oder für ein Ammoniumion steht,
erhält,
wenn man Verbindungen der Formel (Ia) 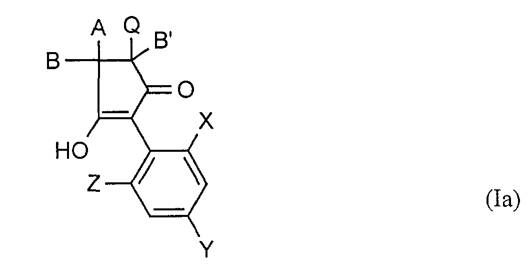 in welcher
A, B, B', Q, X, Y und Z die oben angegebene Bedeutung haben,
mit Metall-Verbindungen oder Aminen der Formeln (X) bzw. (XI)
Me Rₜ ¹⁶ (X) 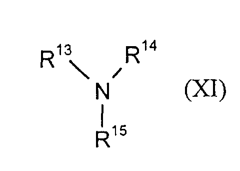
in welchen
Me für ein- oder zweiwertige Metallionen steht,
t für die Zahl 1 oder 2 steht,
R¹³, R¹⁴ und R¹⁵ unabhängig voneinander für Wasserstoff oder Alkyl stehen und
R¹⁶ für Wasserstoff, Hydroxy oder C₁-C₄-Alkoxy steht,
gegebenenfalls in Gegenwart eines Verdünnungsmittels umsetzt;
(H) Verbindungen der Formel (Ig) 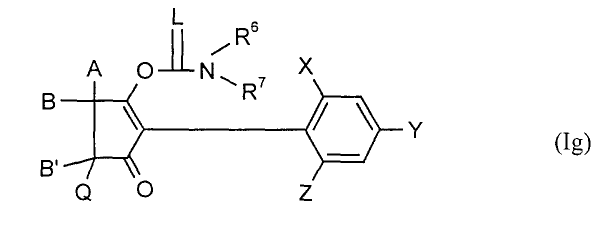 in welcher
A, B, L, B', Q, X, Y, Z, R⁶ und R⁷ die in Anspruch 1 angegebene Bedeutung haben,
erhält, wenn man Verbindungen der Formel (Ia) 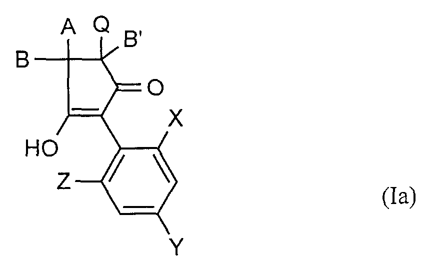 in welcher
A, B, B', Q, X, Y und Z die oben angegebene Bedeutung haben,
α) mit Verbindungen der Formel (XII)
R⁶-N=C=L (XII)
in welcher
L und R⁶ die oben angegebene Bedeutung haben,
gegebenenfalls in Gegenwart eines Verdünnungsmittels und gegebenenfalls in Gegenwart eines Katalysators
oder
β) mit Carbamidsäurechloriden oder Thiocarbamidsäurechloriden der Formel (XIII) 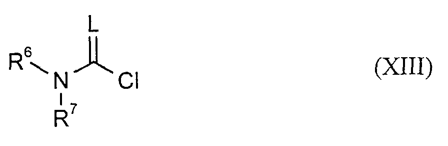 in welcher
L, R⁶ und R⁷ die oben angegebene Bedeutung haben
gegebenenfalls in Gegenwart eines Verdünnungsmittels und
gegebenenfalls in Gegenwart eines Säurebindemittels umsetzt.

5. Verbindungen der Formel (II) 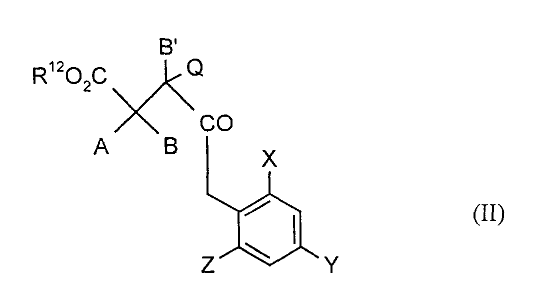 in welcher
A, B, B', Q, X, Y und Z die in Anspruch 1 angegebene Bedeutung haben
und
R¹² für Alkyl steht.

6. Verbindungen der Formel (XIV) 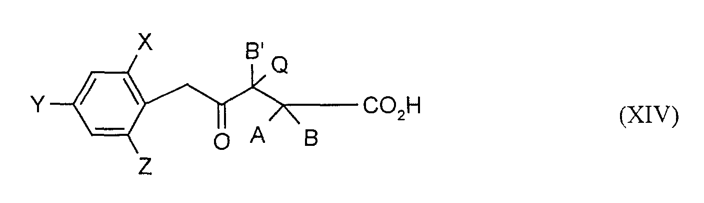 in welcher
A, B, B', Q, X, Y und Z die in Anspruch 1 angegebene Bedeutung haben.

7. Verbindungen der Formel (XVII) 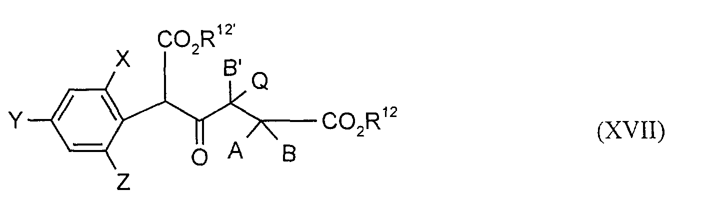 in welcher
A, B, B', Q, X, Y und Z die in Anspruch 1 angegebene Bedeutung haben
und
R¹² und R¹²' für Alkyl stehen.

8. Schädlingsbekämpfungsmittel und herbizide Mittel, **gekennzeichnet durch** einen Gehalt an mindestens einer Verbindung der Formel (I) gemäß Anspruch 1.

9. Verwendung von Verbindungen der Formel (I) gemäß Anspruch 1 zur Bekämpfung von Schädlingen und Unkräutern.

10. Verfahren zur Bekämpfung von Schädlingen und Unkräutern, **dadurch gekennzeichnet, daß** man Verbindungen der Formel (I) gemäß Anspruch 1 auf Schädlinge, Unkräuter und/oder ihren Lebensraum einwirken läßt.

11. Verfahren zur Herstellung von Schädlingsbekämpfungsmitteln und herbiziden Mitteln, **dadurch gekennzeichnet, daß** man Verbindungen der Formel (I) gemäß Anspruch 1 mit Streckmitteln und/oder oberflächenaktiven Mitteln vermischt.

12. Verwendung von Verbindungen der Formel (I) gemäß Anspruch 1 zur Herstellung von Schädlingsbekämpfungsmitteln und herbiziden Mitteln.

## Claims

1. Compounds of the formula (I) 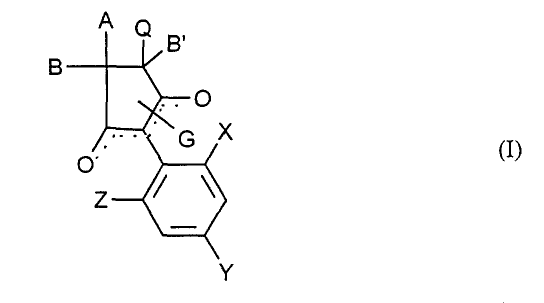 in which
X represents halogen, C₁-C₆-alkyl, C₂-C₆-alkenyl, C₁-C₆-alkoxy, C₂-C₆-alkenyloxy, C₁-C₆-alkylthio, C₁-C₆-alkylsulphinyl, C₁-C₆-alkylsulphonyl, C₁-C₆-halogenoalkyl, C₁-C₆-halogenoalkoxy, C₂-C₆-halogenoalkenyloxy, nitro, cyano or respectively optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, phenoxy, phenylthio, benzyloxy or benzylthio,
Y represents hydrogen, halogen, C₁-C₆-alkyl, C₂-C₆-alkenyl, C₁-C₆-alkoxy, C₂-C₆-alkenyloxy, C₁-C₆-alkylthio, C₁-C₆-alkylsulphinyl, C₁-C₆-alkylsulphonyl, C₁-C₆-halogenoalkyl, C₁-C₆-halogenoalkoxy, C₂-C₆-halogenoalkenyloxy, nitro or cyano,
Z represents halogen, C₁-C₆-alkyl, C₂-C₆-alkenyl, C₁-C₆-alkoxy, C₂-C₆-alkenyloxy, C₁-C₆-halogenoalkyl, C₁-C₆-halogenoalkoxy, C₂-C₆-halogenoalkenyloxy, nitro or cyano,
where X, Y and Z do not simultaneously represent methyl,
A and Q together represent C₁-C₆-alkanediyl or C₂-C₆-alkenediyl, each of which is optionally mono- to trisubstituted by identical or different substituents from the group consisting of halogen, hydroxyl, mercapto, and of C₁-C₁₀-alkyl, C₁-C₆-alkoxy, C₁-C₆-alkylthio, C₃-C₇-cycloalkyl, each of which is optionally mono- to nonasubstituted by identical or different halogens, and of benzyloxy and phenyl, each of which is optionally mono- to pentasubstituted by identical or different substituents from the group consisting of halogen, C₁-C₆-alkyl and C₁-C₆-alkoxy, it being additionally possible for the C₁-C₆-alkanediyl or the C₂-C₆-alkenediyl to contain one of the groups below 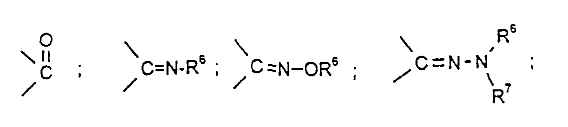 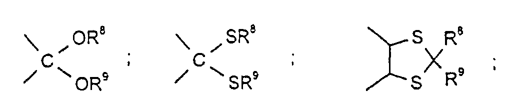 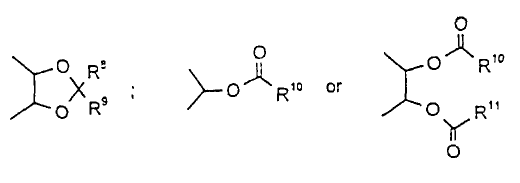 or to be bridged by a C₁-C₂-alkanediyl group,
B and B' independently of one another each represent hydrogen, halogen or C₁-C₆-alkyl or together represent respectively optionally C₁-C₆-alkyl-substituted C₁-C₆-alkanediyl or C₂-C₄-alkenediyl,
G represents hydrogen (a) or represents one of the groups 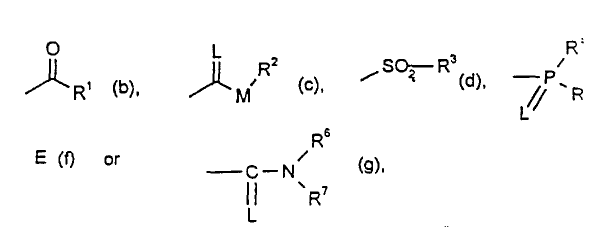 in which
E represents a metal ion or an ammonium ion,
L represents oxygen or sulphur and
M represents oxygen or sulphur,
R¹ represents C₁-C₂₀-alkyl, C₂-C₁₀-alkenyl, C₁-C₈-alkoxy-C₁-C₈-alkyl, C₁-C₈-alkylthio-C₁-C₈-alkyl, poly-C₁-C₈-alkoxy-C₁-C₈-alkyl, each of which is optionally mono- or polysubstituted by identical or different halogens, or represents C₃-C₈-cycloalkyl which is optionally mono- or polysubstituted by identical or different substituents from the group consisting of halogen, C₁-C₆-alkyl and C₁-C₆-alkoxy and in which at least one methylene group may be replaced by an oxygen and/or sulphur atom,
represents phenyl which is optionally mono- or polysubstituted by identical or different substituents from the group consisting of halogen, nitro, cyano, C₁-C₆-alkyl, C₁-C₆-alkoxy, C₁-C₆-halogenoalkyl and C₁-C₆-halogenoalkoxy,
represents phenyl-C₁-C₆-alkyl which is optionally mono- or polysubstituted by identical or different substituents from the group consisting of halogen, C₁-C₆-alkyl, C₁-C₆-alkoxy, C₁-C₆-halogenoalkyl and C₁-C₆-halogenoalkoxy,
represents hetaryl which is optionally mono- or polysubstituted by identical or different substituents from the group consisting of halogen and C₁-C₆-alkyl and has 5 or 6 ring atoms and one to three hetero atoms from the group consisting of oxygen, sulphur and nitrogen,
represents phenoxy-C₁-C₆-alkyl which is optionally mono- or polysubstituted by identical or different substituents from the group consisting of halogen and C₁-C₆-alkyl, or
represents hetaryloxy-C₁-C₆-alkyl which is optionally mono- or polysubstituted by identical or different substituents from the group consisting of halogen, amino and C₁-C₆-alkyl and has 5 or 6 ring atoms and one to three hetero atoms from the group consisting of oxygen, sulphur and nitrogen,
R² represents C₁-C₂₀-alkyl, C₂-C₁₀-alkenyl, C₁-C₈-alkoxy-C₂-C₈-alkyl or poly-C₁-C₈-alkoxy-C₂-C₈-alkyl, each of which is optionally mono- or polysubstituted by identical or different halogens,
represents C₃-C₆-cycloalkyl which is optionally mono- or polysubstituted by identical or different substituents from the group consisting of halogen, C₁-C₆-alkyl and C₁-C₆-alkoxy, or
represents phenyl or benzyl, each of which is optionally mono- or polysubstituted by identical or different substituents from the group consisting of halogen, nitro, cyano, C₁-C₆-alkyl, C₁-C₆-alkoxy, C₁-C₃-halogenoalkoxy and C₁-C₃-halogenoalkyl,
R³ represents C₁-C₁₂-alkyl, which is optionally mono- or polysubstituted by identical or different halogens, or represents phenyl or phenyl-C₁-C₄-alkyl, each of which is optionally mono- or polysubstituted by identical or different substituents from the group consisting of halogen, C₁-C₆-alkyl, C₁-C₆-alkoxy, C₁-C₃-halogenoalkyl, C₁-C₃-halogenoalkoxy, cyano and nitro,
R⁴ and R⁵ independently of one another each represent C₁-C₈-alkyl, C₁-C₈-alkoxy, C₁-C₈-alkylamino, di-(C₁-C₈-alkyl)-amino, C₁-C₈-alkylthio, C₃-C₅-alkenylthio, C₃-C₇-cycloalkylthio, each of which is optionally mono- or polysubstituted by identical or different halogens, or represents phenyl, phenoxy or phenylthio, each of which is optionally mono- or polysubstituted by identical or different substituents from the group consisting of halogen, nitro, cyano, C₁-C₄-alkoxy, C₁-C₄-halogenoalkoxy, C₁-C₄-alkylthio, C₁-C₄-halogenoalkylthio, C₁-C₄-alkyl and C₁-C₄-halogenoalkyl,
R⁶ represents hydrogen, represents C₁-C₁₀-alkyl, C₃-C₈-alkenyl, C₁-C₈-alkoxy-C₂-C₈-alkyl, each of which is optionally mono- or polysubstituted by identical or different halogens, represents C₃-C₁₀-cycloalkyl which is optionally mono- or polysubstituted by identical or different substituents from the group consisting of halogen, C₁-C₆-alkyl, C₁-C₆-alkoxy, C₁-C₃-halogenoalkyl and C₁-C₃-halogenoalkoxy, represents phenyl which is optionally mono- or polysubstituted by identical or different substituents from the group consisting of halogen, C₁-C₃-halogenoalkyl, C₁-C₈-alkyl, C₁-C₃-halogenoalkoxy and C₁-C₈-alkoxy, or represents benzyl which is optionally mono- or polysubstituted by identical or different substituents from the group consisting of halogen, C₁-C₈-alkyl, C₁-C₃-halogenoalkyl, C₁-C₃-halogenoalkoxy and C₁-C₈-alkoxy,
R⁷ represents hydrogen or represents C₁-C₁₀-alkyl or C₃-C₁₀-alkenyl, each of which is optionally mono- or polysubstituted by identical or different halogens, or
R⁶ and R⁷ combine with the linking N-atom to form an optionally oxygen- or sulphur-containing and optionally C₁-C₆-alkyl-substituted 3- to 7-membered ring,
R⁸ and R⁹ independently of one another each represent hydrogen, represent C₁-C₆-alkyl which is optionally mono- or polysubstituted by identical or different halogens or represent phenyl or phenyl-C₁-C₄-alkyl, each of which is optionally mono- or polysubstituted by identical or different substituents from the group consisting of halogen, C₁-C₆-alkyl, C₁-C₆-alkoxy, C₁-C₆-halogenoalkyl, C₁-C₆-halogenoalkoxy, nitro and cyano,
or together represent C₂-C₆-alkanediyl which is optionally mono- or polysubstituted by identical or different substituents from the group consisting of halogen, C₁-C₆-alkyl, C₁-C₆-alkoxy and C₁-C₃-halogenoalkyl, and
R¹⁰ and R¹¹ independently of one another each represent C₁-C₁₀-alkyl, C₂-C₁₀-alkenyl, C₁-C₁₀-alkoxy, C₁-C₁₀-alkylamino, di-(C₁-C₁₀-alkyl)-amino, C₃-C₁₀-alkenylamino, di-(C₃-C₁₀-alkenyl)-amino, each of which is optionally mono- or polysubstituted by identical or different halogens, or represent phenyl or benzyl, each of which is mono- or polysubstituted by identical or different substituents from the group consisting of halogen, C₁-C₄-alkyl, C₁-C₄-alkoxy, C₁-C₄-halogenoalkyl, nitro and cyano.

2. Compounds of the formula (I) according to Claim 1, in which
X represents fluorine, chlorine, bromine, C₁-C₄-alkyl, C₂-C₄-alkenyl, C₁-C₄-alkoxy, C₂-C₄-alkenyloxy, C₁-C₄-alkylthio, C₁-C₄-alkylsulphinyl, C₁-C₄-alkylsulphonyl, C₁-C₄-halogenoalkyl, C₁-C₄-halogenoalkoxy, C₂-C₄-halogenoalkenyloxy, nitro, cyano or respectively optionally fluorine-, chlorine-, bromine-, C₁-C₄-alkyl-, C₁-C₄-alkoxy-, C₁-C₂-halogenoalkyl-, C₁-C₂-halogenoalkoxy-, nitro- or cyano-substituted phenyl, phenoxy, phenylthio, benzyloxy or benzylthio,
Y represents hydrogen, fluorine, chlorine, bromine, C₁-C₄-alkyl, C₂-C₄-alkenyl, C₁-C₄-alkoxy, C₂-C₄-alkenyloxy, C₁-C₄-alkylthio, C₁-C₄-alkylsulphinyl, C₁-C₄-alkylsulphonyl, C₁-C₄-halogenoalkyl, C₁-C₄-halogenoalkoxy, C₂-C₄-halogenoalkenyloxy, nitro or cyano,
Z represents fluorine, chlorine, bromine, C₁-C₄-alkyl, C₂-C₄-alkenyl, C₁-C₄-alkoxy, C₂-C₄-alkenyloxy, C₁-C₄-halogenoalkyl, C₁-C₄-halogenoalkoxy, C₂-C₄-halogenoalkenyloxy, nitro or cyano,
where X, Y and Z do not simultaneously represent methyl,
A and Q together represent C₁-C₅-alkanediyl or C₂-C₅-alkenediyl, each of
which is optionally mono- or disubstituted by identical or different substituents from the group consisting of fluorine, chlorine, bromine, hydroxyl, mercapto, and of C₁-C₈-alkyl, C₁-C₄-alkoxy, C₁-C₄-alkylthio, C₅-C₇-cycloalkyl or phenyl, each of which is optionally mono- to pentasubstituted by identical or different substituents from the group consisting of fluorine and chlorine, it being additionally possible for the C₁-C₅-alkanediyl or the C₂-C₅-alkenediyl to contain one of the groupings below 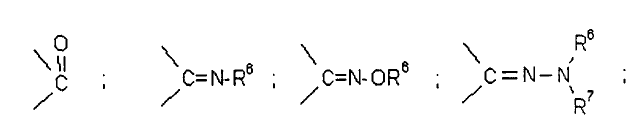 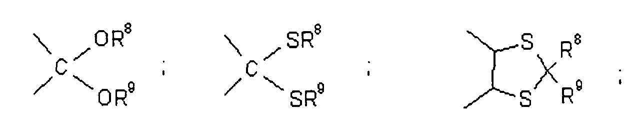 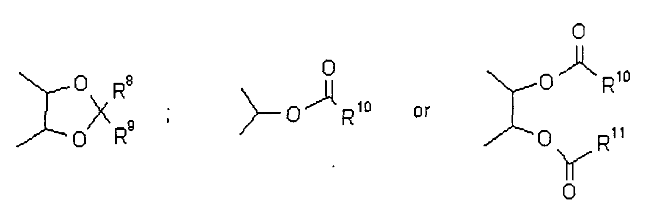 or to be bridged by a C₁-C₂-alkanediyl group,
B and B' independently of one another each represent hydrogen, fluorine, chlorine or C₁-C₄-alkyl or together represent respectively optionally C₁-C₄-alkyl-substituted C₁-C₅-alkanediyl or C₂-C₄-alkenediyl,
G represents hydrogen (a) or one of the groups 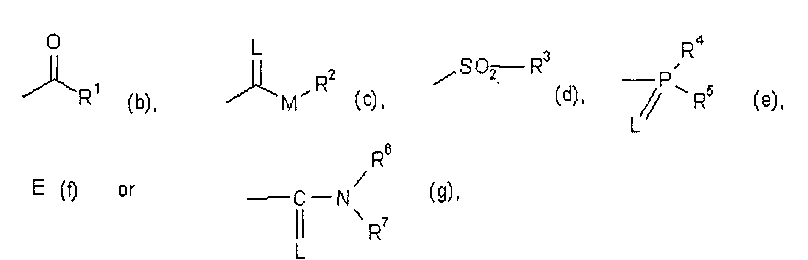 in which
E represents a metal ion or an ammonium ion,
L represents oxygen or sulphur and
M represents oxygen or sulphur,
R¹ represents C₁-C₁₆-alkyl, C₂-C₈-alkenyl, C₁-C₆-alkoxy-C₁-C₆-alkyl, C₁-C₆-alkylthio-C₁-C₆-alkyl, poly-C₁-C₆-alkoxy-C₁-C₆-alkyl, each of which is optionally mono- to nonasubstituted by identical or different substituents from the group consisting of fluorine and chlorine, or represents C₃-C₇-cycloalkyl which is optionally mono- to trisubstituted by identical or different substituents from the group consisting of fluorine, chlorine, C₁-C₄-alkyl and C₁-C₄-alkoxy and in which one or two not directly adjacent methylene groups may be replaced by oxygen and/or sulphur atoms,
represents phenyl which is optionally mono- to trisubstituted by identical or different substituents from the group consisting of fluorine, chlorine, bromine, cyano, nitro, C₁-C₄-alkyl, C₁-C₄-alkoxy, C₁-C₃-halogenoalkyl and C₁-C₃-halogenoalkoxy,
represents phenyl-C₁-C₄-alkyl which is optionally mono- to trisubstituted by identical or different substituents from the group consisting of fluorine, chlorine, bromine, C₁-C₄-alkyl, C₁-C₄-alkoxy, C₁-C₃-halogenoalkyl and C₁-C₃-halogenoalkoxy,
represents furanyl, thienyl, pyridyl, pyrimidyl, thiazolyl or pyrazolyl, each of which is optionally mono- or disubstituted by identical or different substituents from the group consisting of fluorine, chlorine, bromine and C₁-C₄-alkyl,
represents phenoxy-C₁-C₅-alkyl which is optionally mono- to trisubstituted by identical or different substituents from the group consisting of fluorine, chlorine and C₁-C₄-alkyl, or
represents pyridyloxy-C₁-C₆-alkyl, pyrimidinyloxy-C₁-C₆-alkyl or thiazolyloxy-C₁-C₆-alkyl, each of which is optionally mono- or disubstituted by identical or different substituents from the group consisting of fluorine, chlorine, amino and C₁-C₄-alkyl,
R² represents C₁-C₁₆-alkyl, C₂-C₈-alkenyl, C₁-C₆-alkoxy-C₂-C₆-alkyl or poly-C₁-C₆-alkoxy-C₂-C₆-alkyl, each of which is optionally mono- to heptasubstituted by identical or different substituents from the group consisting of fluorine and chlorine,
represents C₃-C₆-cycloalkyl which is optionally mono- to trisubstituted by identical or different substituents from the group consisting of fluorine, chlorine, C₁-C₄-alkyl and C₁-C₄-alkoxy, or
represents phenyl or benzyl, each of which is optionally mono- to trisubstituted by identical or different substituents from the group consisting of fluorine, chlorine, bromine, nitro, cyano, C₁-C₄-alkyl, C₁-C₄-alkoxy, C₁-C₂-halogenoalkoxy and C₁-C₂-halogenoalkyl,
R³ represents C₁-C₉-alkyl, which is optionally mono- to pentasubstituted by identical or different substituents from the group consisting of fluorine and chlorine, or represents phenyl or phenyl-C₁-C₂-alkyl, each of which is optionally mono- to trisubstituted by identical or different substituents from the group consisting of fluorine, chlorine, bromine, C₁-C₄-alkyl, C₁-C₄-alkoxy, C₁-C₂-halogenoalkyl, C₁-C₂-halogenoalkoxy, cyano and nitro,
R⁴ and R⁵ independently of one another each represent C₁-C₆-alkyl, C₁-C₆-alkoxy, C₁-C₆-alkylamino, di-(C₁-C₆-alkyl)-amino, C₁-C₆-alkylthio, C₃-C₄-alkenylthio, C₃-C₆-cycloalkylthio, each of which is optionally mono- to pentasubstituted by identical or different substituents from the group consisting of fluorine and chlorine, or represents phenyl, phenoxy or phenylthio, each of which is optionally mono- to trisubstituted by identical or different substituents from the group consisting of fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl and C₁-C₃-halogenoalkyl,
R⁶ represents hydrogen, represents C₁-C₈-alkyl, C₃-C₆-alkenyl, C₁-C₆-alkoxy-C₂-C₆-alkyl, each of which is optionally mono- to pentasubstituted by identical or different substituents from the group consisting of fluorine and chlorine, represents C₃-C₈-cycloalkyl which is optionally mono- to trisubstituted by identical or different substituents from the group consisting of fluorine, chlorine, C₁-C₄-alkyl, C₁-C₄-alkoxy, C₁-C₂-halogenoalkyl and C₁-C₂-halogenalkoxy, represents phenyl which is optionally mono- to trisubstituted by identical or different substituents from the group consisting of fluorine, chlorine, bromine, C₁-C₂-halogenoalkyl, C₁-C₅-alkyl, C₁-C₂-halogenoalkoxy and C₁-C₅-alkoxy, or represents benzyl which is optionally mono- to trisubstituted by identical or different substituents from the group consisting of fluorine, chlorine, bromine, C₁-C₅-alkyl, C₁-C₂-halogenoalkyl, C₁-C₂-halogenoalkoxy and C₁-C₅-alkoxy,
R⁷ represents hydrogen or represents C₁-C₈-alkyl or C₃-C₈-alkenyl, each of which is optionally mono- to pentasubstituted by identical or different substituents from the group consisting of fluorine and chlorine, or
R⁶ and R⁷ combine with the linking N-atom to form an optionally oxygen- or sulphur-containing and optionally C₁-C₄-alkyl-substituted 4- to 7-membered ring,
R⁸ and R⁹ independently of one another each represent hydrogen, C₁-C₄-alkyl which is optionally mono- to pentasubstituted by identical or different substituents from the group consisting of fluorine and chlorine, or represent phenyl which is optionally mono- to trisubstituted by identical or different substituents from the group consisting of fluorine, chlorine, C₁-C₄-alkyl, C₁-C₄-alkoxy, C₁-C₂-halogenoalkyl, C₁-C₂-halogenoalkoxy, nitro and cyano,
or together represent C₂-C₅-alkanediyl which is optionally mono- to trisubstituted by identical or different substituents from the group consisting of fluorine, chlorine, C₁-C₄-alkyl, C₁-C₄-alkoxy and C₁-C₂-halogenoalkyl, and
R¹⁰ and R¹¹ independently of one another each represent C₁-C₈-alkyl, C₂-C₈-alkenyl, C₁-C₈-alkoxy, C₁-C₈-alkylamino, C₃-C₈-alkenylamino, di-(C₁-C₈-alkyl)-amino or di-(C₃-C₈-alkenyl)-amino, each of which is optionally mono- to pentasubstituted by identical or different substituents from the group consisting of fluorine and chlorine.

3. Compounds of the formula (I) according to Claim 1, in which
X represents fluorine, chlorine, bromine, methyl, ethyl, propyl, isopropyl, ethenyl, 1-propenyl, methoxy, ethoxy, propoxy, isopropoxy, allyloxy, methallyloxy, trifluoromethyl, difluoromethoxy, trifluoromethoxy, trifluoroethoxy, methylthio, methylsulphinyl, methylsulphonyl, nitro, cyano, or respectively optionally fluorine-, chlorine-, bromine-, methyl-, ethyl-, propyl-, isopropyl-, tert-butyl-, methoxy-, ethoxy-, propoxy-, tert-butoxy-, trifluoromethyl-, trifluoromethoxy-, nitro- or cyano-substituted phenyl, phenoxy, phenylthio, benzyloxy or benzylthio,
Y represents hydrogen, fluorine, chlorine, bromine, methyl, ethyl, n-propyl, i-propyl, n-butyl, i-butyl, tert-butyl, ethenyl, 1-propenyl, methoxy, ethoxy, propoxy, isopropoxy, allyloxy, methallyloxy, trifluoromethyl, methylthio, methylsulphinyl, methylsulphonyl, difluoromethoxy, trifluoromethoxy, trifluoroethoxy, nitro or cyano,
Z represents fluorine, chlorine, bromine, methyl, ethyl, n-propyl, i-propyl, n-butyl, i-butyl, tent-butyl, ethenyl, 1-propenyl, methoxy, ethoxy, propoxy, isopropoxy, allyloxy, methallyloxy, trifluoromethyl, difluoromethoxy, trifluoromethoxy, trifluoroethoxy, nitro or cyano,
where X, Y and Z do not simultaneously represent methyl,
A and Q together represent C₁-C₄-alkanediyl or C₂-C₄-alkenediyl, each of which is optionally mono- or disubstituted by identical or different substituents from the group consisting of fluorine, chlorine, hydroxyl, and of C₁-C₆-alkyl and C₁-C₂-alkoxy, each of which is optionally mono- to trisubstituted by identical or different substituents from the group consisting of fluorine and chlorine,
B and B' independently of one another each represent hydrogen, methyl or ethyl,
G represents hydrogen (a) or represents one of the groups 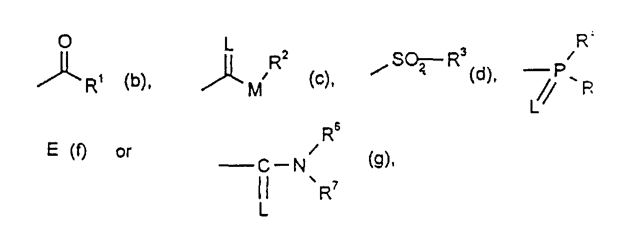 in which
E represents a metal ion or an ammonium ion,
L represents oxygen or sulphur and
M represents oxygen or sulphur,
R¹ represents C₁-C₁₄-alkyl, C₂-C₆-alkenyl, C₁-C₄-alkoxy-C₁-C₆-alkyl, C₁-C₄-alkylthio-C₁-C₆-alkyl, poly-C₁-C₄-alkoxy-C₁-C₄-alkyl, each of which is optionally mono- to pentasubstituted by identical or different substituents from the group consisting of fluorine and chlorine, or represents C₃-C₆-cycloalkyl which is optionally mono- or di substituted by identical or different substituents from the group consisting of fluorine, chlorine, methyl, ethyl, methoxy and ethoxy and in which one or two not directly adjacent methylene groups may be replaced by oxygen and/or sulphur atoms,
represents phenyl which is optionally mono- or disubstituted by identical or different substituents from the group consisting of fluorine, chlorine, bromine, methyl, ethyl, propyl, i-propyl, methoxy, ethoxy, trifluoromethyl, trifluoromethoxy, cyano and nitro,
represents benzyl which is optionally mono- or disubstituted by identical or different substituents from the group consisting of fluorine, chlorine, bromine, methyl, ethyl, propyl, i-propyl, methoxy, ethoxy, trifluoromethyl and trifluoromethoxy,
represents thienyl, furanyl or pyridyl, each of which is optionally mono- or disubstituted by identical or different substituents from the group consisting of fluorine, chlorine, bromine, methyl and ethyl,
represents phenoxy-C₁-C₄-alkyl which is optionally mono- or disubstituted by identical or different substituents from the group consisting of fluorine, chlorine, methyl and ethyl, or
represents pyridyloxy-C₁-C₄-alkyl, pyrimidyloxy-C₁-C₄-alkyl or thiazolyloxy-C₁-C₄-alkyl, each of which is optionally mono- or disubstituted by identical or different substituents from the group consisting of fluorine, chlorine, amino, methyl and ethyl,
R² represents C₁-C₁₄-alkyl, C₂-C₆-alkenyl, C₁-C₄-alkoxy-C₂-C₆-alkyl or poly-C₁-C₄-alkoxy-C₂-C₆-alkyl, each of which is optionally mono- to pentasubstituted by identical or different substituents from the group consisting of fluorine and chlorine, represents C₃-C₆-cycloalkyl which is optionally mono- to trisubstituted by identical or different substituents from the group consisting of fluorine, chlorine, methyl, ethyl, methoxy and ethoxy,
or represents phenyl or benzyl, each of which is optionally mono- or disubstituted by identical or different substituents from the group consisting of fluorine, chlorine, nitro, cyano, methyl, ethyl, propyl, i-propyl, methoxy, ethoxy, trifluoromethoxy and trifluoromethyl,
R³ represents C₁-C₆-alkyl, which is optionally mono- to trisubstituted by identical or different substituents from the group consisting of fluorine and chlorine, or represents phenyl or benzyl, each of which is optionally mono- or disubstituted by fluorine, chlorine, bromine, methyl, ethyl, methoxy, ethoxy, trifluoromethyl, trifluoromethoxy, cyano or nitro,
R⁴ and R⁵ independently of one another each represent C₁-C₄-alkyl, C₁-C₄-alkoxy, C₁-C₄-alkylamino, di-(C₁-C₄-alkyl)-amino, C₁-C₄-alkylthio, each of which is optionally mono- to trisubstituted by identical or different substituents from the group consisting of fluorine and chlorine, or represents phenyl, phenoxy or phenylthio, each of which is optionally mono- or disubstituted by identical or different substituents from the group consisting of fluorine, chlorine, bromine, nitro, cyano, C₁-C₂-alkoxy, trifluoromethoxy, C₁-C₂-alkylthio, trifluoromethyl or C₁-C₃-alkyl,
R⁶ represents hydrogen, represents C₁-C₆-alkyl, C₁-C₆-alkoxy-C₂-C₄-alkyl, each of which is optionally mono- to trisubstituted by identical or different substituents from the group consisting of fluorine and chlorine, represents C₃-C₆-cycloalkyl which is optionally mono- or disubstituted by identical or different substituents from the group consisting of fluorine, chlorine, methyl, methoxy, trifluoromethyl and trifluoromethoxy, represents phenyl which is optionally mono- or disubstituted by identical or different substituents from the group consisting of fluorine, chlorine, trifluoromethyl, C₁-C₄-alkyl, trifluoromethoxy and C₁-C₄-alkoxy, or represents benzyl which is optionally mono- or disubstituted by identical or different substituents from the group consisting of fluorine, chlorine, C₁-C₄-alkyl, trifluoromethyl, trifluoromethoxy and C₁-C₄-alkoxy,
R⁷ represents hydrogen, or represents C₁-C₆-alkyl or C₃-C₆-alkenyl, each of which is optionally mono- to trisubstituted by identical or different substituents from the group consisting of fluorine and chlorine, or
R⁶ and R⁷ combine with the linking N-atom to form an optionally oxygen- or sulphur-containing, optionally methyl-substituted 5- to 7-membered ring.

4. Process for preparing compounds of the forrnula (I) according to Claim 1,
**characterized in that**
(A) compounds of the formula (Ia) 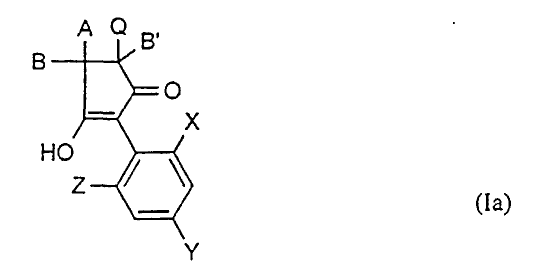 in which
A, B, B', Q, X, Y and Z are each as defined in Claim 1
are obtained
when compounds of the formula (II) 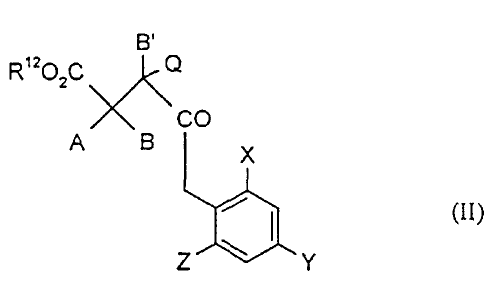 in which
A, B, B', Q, X, Y and Z are each as defined above, and
R¹² represents alkyl
are condensed intramolecularly in the presence of a diluent and in the
presence of a base;
(B) compounds of the formula (Ib) 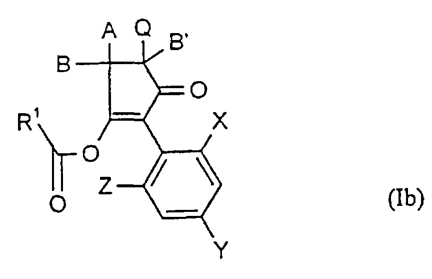 in which
A, B, B', Q, X, Y, Z and R¹ are each as defined in Claim 1
are obtained
when compounds of the formula (Ia) 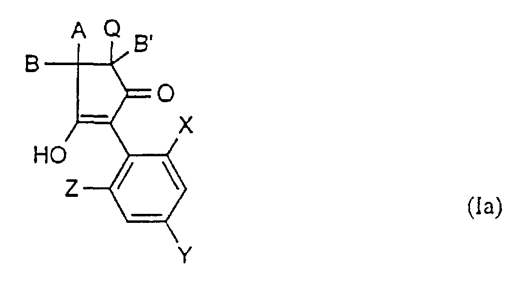 in which
A, B, B', X, Y, Z and Q are each as defined above
are reacted
α) with acyl halides of the formula (III) 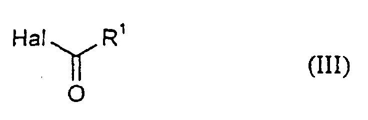 in which
R¹ is as defined above and
Hal represents halogen,
if appropriate in the presence of a diluent and if appropriate in the presence of an acid binder
or
β) with carboxylic anhydrides of the formula (IV)
R¹-CO-O-CO-R¹ (IV)
in which
R¹ is as defined above,
if appropriate in the presence of a diluent and if appropriate in the presence of an acid binder;
(C) compounds of the formula (Ic-1) 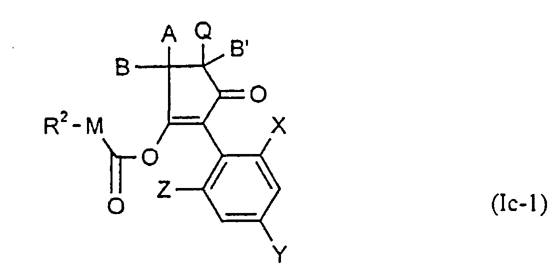 in which
A, B, B', Q, X, Y, Z and R² are as defined in Claim 1
and
M represents oxygen or sulphur
are obtained
when compounds of the formula (Ia) 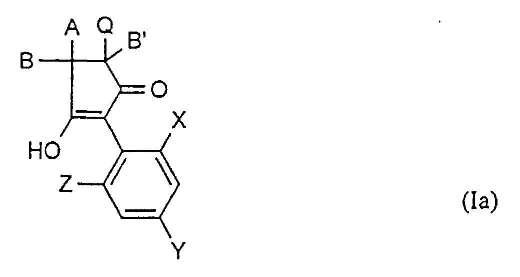 in which
A, B, B', Q, X, Y and Z are each as defined above
are reacted with chloroformic esters or chloroformic thioesters of the formula (V)
R²-M-CO-Cl (V)
in which
R² and M are each as defined above,
if appropriate in the presence of a diluent and if appropriate in the presence of an acid binder;
(D) compounds of the formula (Ic-2) 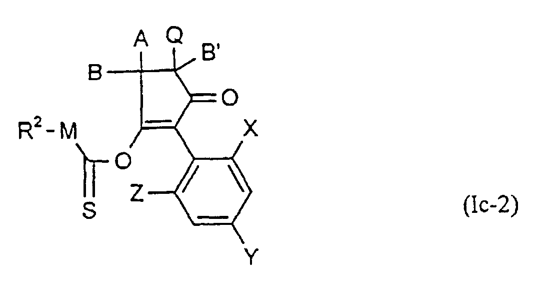 in which
A, B, B', Q, X, Y, Z and R² are each as defined in Claim 1,
and
M represents oxygen or sulphur,
are obtained,
when compounds of the formula (Ia) 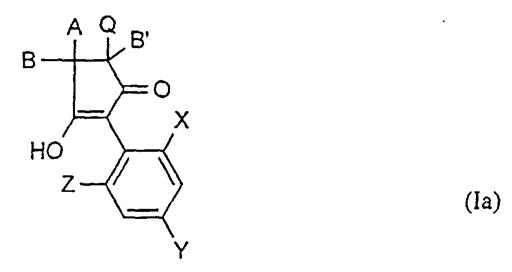 in which
A, B, B', Q, X, Y and Z are each as defined above
are reacted
α) with chloromonothioformic esters or chlorodithioformic ester of the formula (VI) 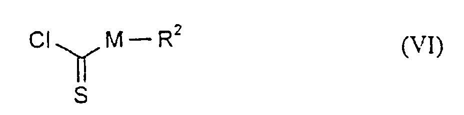 in which
M and R² are each as defined above,
if appropriate in the presence of a diluent and if appropriate in the presence of an acid binder,
or
β) with carbon disulphide and then with alkyl halides of the general formula (VII)
R²-Hal (VII)
in which
R² is as defined above
and
Hal represents chlorine, bromine or iodine,
if appropriate in the presence of a diluent and if appropriate in the presence of a base;
(E) compounds of the formula (Id) 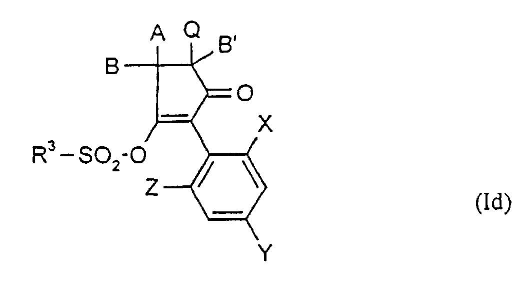 in which
A, B, B', Q, X, Y, Z and R³ are each as defined in Claim 1
are obtained
when compounds of the formula (Ia) 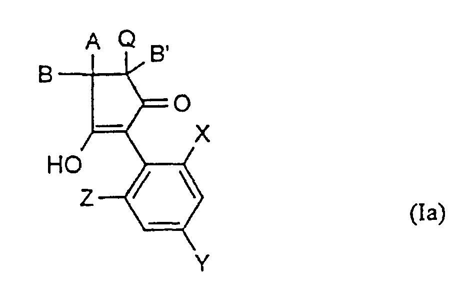 in which
A, B, B', Q, X, Y and Z are each as defined above
are reacted with sulphonyl chlorides of the formula (VIII)
R³-SO₂-Cl (VIII)
in which
R³ is as defined above,
if appropriate in the presence of a diluent and if appropriate in the presence of an acid binder;
(F) compounds of the formula (Ie) 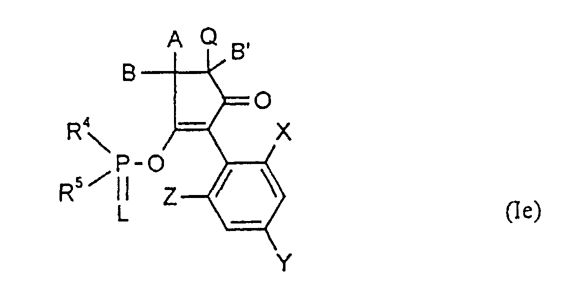 in which
A, B, L, B', Q, X, Y, Z, R⁴ and R⁵ are each as defined in Claim 1
are obtained
when compounds of the formula (Ia) or enols thereof 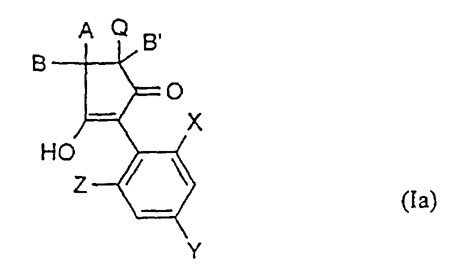 in which
A, B, B', Q, X, Y and Z are each as defined above
are reacted with phosphorus compounds of the formula (IX) 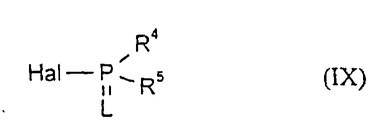 in which
L, R⁴ and R⁵ are each as defined above
and
Hal represents halogen,
if appropriate in the presence of a diluent and if appropriate in the presence of an acid binder;
(G) compounds of the formula (If) 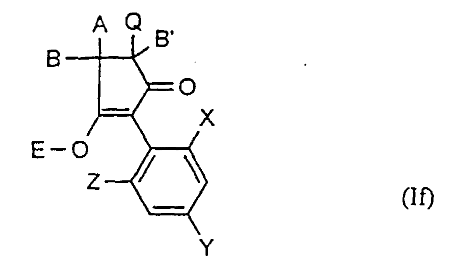 in which
A, B, B', Q, X, Y and Z are each as defined in Claim 1
and
E represents a metal ion or represents an ammonium ion
are obtained
when compounds of the formula (Ia) 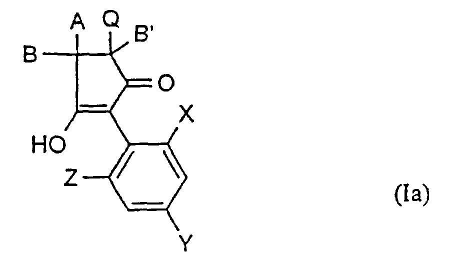 in which
A, B, B', Q, X, Y and Z are each as defined above
are reacted with metal compounds or amines of the formulae (X) and (XI), respectively
Me Rₜ ¹⁶ (X) 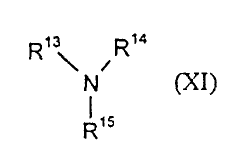
in which
Me represents mono- or bivalent metal ions,
t represents the number 1 or 2,
R¹³, R¹⁴ and R¹⁵ independently of one another each represent hydrogen or alkyl and
R¹⁶ represents hydrogen, hydroxyl or C₁-C₄-alkoxy,
if appropriate in the presence of a diluent;
(H) compounds of the formula (Ig) 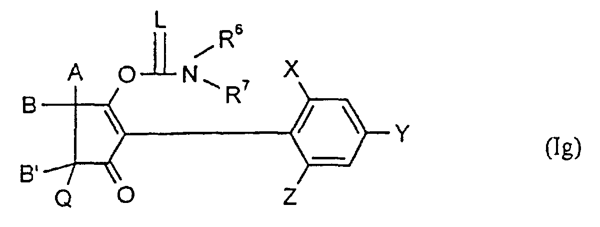 in which
A, B, L, B', Q, X, Y, Z, R⁶ and R⁷ are each as defined in Claim 1
are obtained when compounds of the formula (Ia) 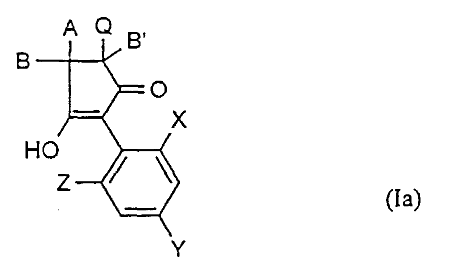 in which
A, B, B', Q, X, Y and Z are each as defined above
are reacted
α) with compounds of the formula (XII)
R⁶-N=C=L (XII)
in which
L and R⁶ are each as defined above,
if appropriate in the presence of a diluent and if appropriate in the presence of a catalyst
or
β) with carbamoyl chlorides or thiocarbamoyl chlorides of the formula (XIII) 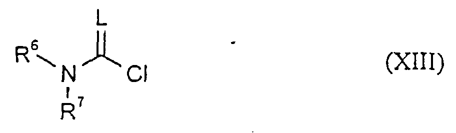 in which
L, R⁶ and R⁷ are each as defined above
if appropriate in the presence of a diluent and if appropriate in the presence of an acid binder.

5. Compounds of the formula (II) 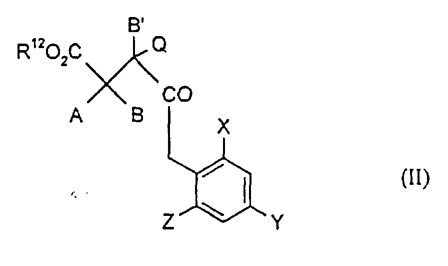 in which
A, B, B', Q, X, Y and Z are each as defined in Claim 1
and
R¹² represents alkyl.

6. Compounds of the formula (XIV) 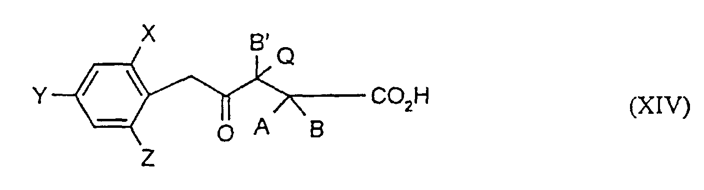 in which
A, B, B', Q, X, Y and Z are each as defined in Claim 1.

7. Compounds of the formula (XVII) 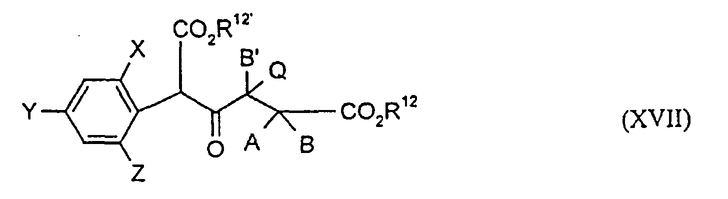 in which
A, B, B', Q, X, Y and Z are each as defined in Claim 1
and
R¹² and R^{12'} each represent alkyl.

8. Pesticides and herbicides, **characterized in that** they comprise at least one compound of the formula (I) according to Claim 1.

9. Use of compounds of the formula (I) according to Claim 1 for controlling pests and weeds.

10. Method for controlling pests and weeds, **characterized in that** compounds of the formula (I) according to Claim 1 are allowed to act on pests, weeds and/or their habitat.

11. Process for preparing pesticides and herbicides, **characterized in that** compounds of the formula (I) according to Claim 1 are mixed with extenders and/or surfactants.

12. Use of compounds of the formula (I) according to Claim 1 for preparing pesticides and herbicides.

## Revendications

1. Comoosés de formule (I) 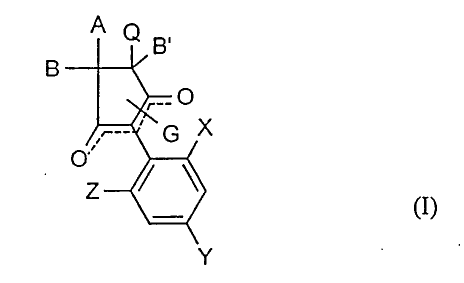 dans laquelle
X représente un halogène, un groupe alkyle en C₁ à C₆, alcényle en C₂ à C₆, alkoxy en C₁ à C₆, alcényloxy en C₂ à C₆, alkylthio en C₁ à C₆, alkylsulfinyle en C₁ à C₆, alkylsulfonyle en C₁ à C₆, halogénalkyle en C₁ à C₆, halogénalkoxy en C₁ à C₆, halogénalcényloxy en C₂ à C₆, nitro, cyano, ou un groupe phényle, phénoxy, phénylthio, benzyloxy ou benzylthio portant éventuellement dans chaque cas un substituant halogéno, alkyle en C₁ à C₆, alkoxy en C₁ à C₆, halogénalkyle en C₁ à C₄, halogénalkoxy en C₁ à C₄, nitro ou cyano,
Y représente l'hydrogène, un halogène, un groupe alkyle en C₁ à C₆, alcényle en C₂ à C₆, alkoxy en C₁ à C₆, alcényloxy en C₂ à C₆, alkylthio en C₁ à C₆, alkylsulfinyle en C₁ à C₆, alkoxysulfonyle en C₁ à C₆, halogénalkyle en C₁ à C₆, halogénalkoxy en C₁ à C₆, haloqénalcényloxy en C₂ à C₆, nitro ou cyano,
Z représente un halogène, un groupe alkyle en C₁ à C₆, alcényle en C₂ à C₆, alkoxy en C₁ à C₆, alcényloxy en C₂ à C₆, halogénalkyle en C₁ à C₆, halogénalkoxy en C₁ à C₆, halogénalcényloxy en C₂ à C₆, nitro ou cyano,
X, Y et Z ne représentant pas simultanément un groupe méthyle,
A et Q forment ensemble un groupe alcanediyle en C₁ à C₆ ou alcènediyle en C₂ à C₆ portant chacun, le cas échéant, un à trois substituants, identiques ou différents, halogéno, hydroxy, mercapto, alkyle en C₁ à C₁₀, alkoxy en C₁ à C₆, alkylthio en C₁ à C₆, cycloalkyle en C₃ à C₇ portant chacun, le cas échéant, un à neuf substituants halogéno identiques ou différents, ou benzyloxy ou phényle portant chacun, le cas échéant, un à cinq substituants, identiques ou différents, halogéno, alkyle en C₁ à C₆ ou alkoxy en C₁ à C₆, qui contient en outre, le cas échéant, l'un des groupes suivants 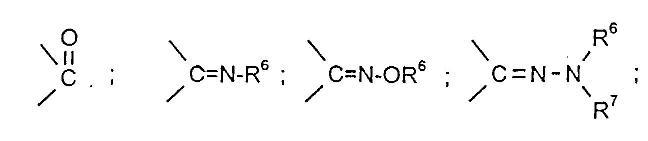 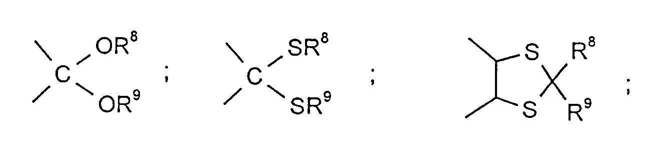 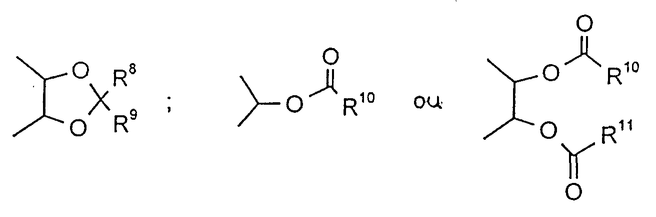 ou qui est ponté par un groupe alcanediyle en C₁ ou C₂,
B et B' représentent, indépendamment l'un de l'autre, avantageusement l'hydrogène, un halogène ou un groupe alkyle en C₁ à C₆ ou forment ensemble un groupe alcanediyle en C₁ à C₆ ou alcènediyle en C₂ à C₄ portant chacun, le cas échéant, un substituant alkyle en C₁ à C₆,
G représente l'hydrogène (a) ou l'un des groupes 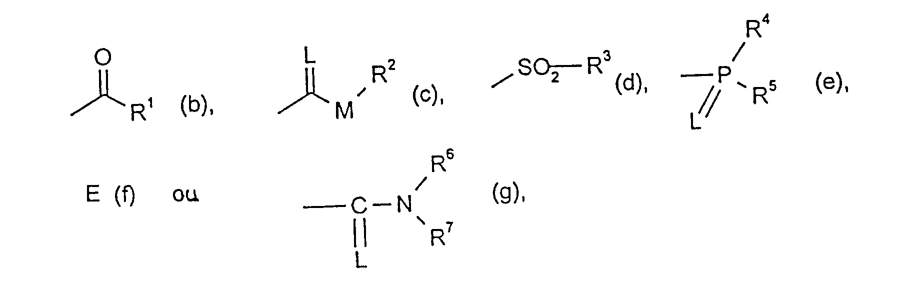 dans lesquels
E est un ion de métal ou un ion ammonium,
L représente l'oxygène ou le soufre et
M représente l'oxygène ou le soufre,
R¹ représente un groupe alkyle en C₁ à C₂₀, alcényle en C₂ à C₁₀, (alkoxy en C₁ à C₈)-(alkyle en C₁ à C₈), (alkylthio en C₁ à C₈)-(alkyle en C₁ à C₈), poly(alkoxy en C₁ à C₈)-(alkyle en C₁ à C₈) portant chacun, le cas échéant, un ou plusieurs substituants halogéno identiques ou différents, ou un groupe cycloalkyle en C₃ à C₈, portant éventuellement un ou plusieurs substituants halogéno, alkyle en C₁ à C₆ ou alkoxy en C₁ à C₆ identiques ou différents, dans lequel au moins un groupe méthylène peut être remplacé par un atome d'oxygène et/ou un atome de soufre,
un groupe phényle portant, le cas échéant, un ou plusieurs substituants halogéno, nitro, cyano, alkyle en C₁ à C₆, alkoxy en C₁ à C₆, halogénalkyle en C₁ à C₆ ou halogénalkoxy en C₁ à C₆ identiques ou différents,
un groupe phényl-(alkyle en C₁ à C₆) portant éventuellement un ou plusieurs substituants halogéno, alkyle en C₁ à C₆, alkoxy en C₁ à C₆, halogénalkyle en C₁ à C₆ ou halogénalkoxy en C₁ à C₆ identiques ou différents,
un groupe hétaryle à noyau de 5 ou 6 atomes ayant un à trois hétéroatomes de la série oxygène, soufre et azote, portant, le cas échéant, un ou plusieurs substituants halogéno ou alkyle en C₁ à C₆ identiques ou différents,
un groupe phénoxy-(alkyle en C₁ à C₆) portant éventuellement un ou plusieurs substituants halogéno ou alkyle en C₁ à C6 identiques ou différents, ou bien un groupe hétaryloxy-(alkyle en C₁ à C₆) à noyau de 5 ou 6 atomes ayant un à trois hétéroatomes de la série oxygène, soufre et azote, portant éventuellement un ou plusieurs substituants halogéno, amino ou alkyle en C₁ à C₆ identiques ou différents,
R² est un groupe alkyle en C₁ à C₂₀, alcényle en C₂ à C₁₀, (alkoxy en C₁ à C₈)-(alkyle en C₂ à C₈) ou poly(alkoxy en C₁ à C₈)-(alkyle en C₂ à C₈) portant chacun, le cas échéant, un ou plusieurs substituants halogéno identiques ou différents,
un groupe cycloalkyle en C₃ à C₆ portant éventuellement un ou plusieurs substituants halogéno, alkyle en C₁ à C₆ ou alkoxy en C₁ à C₆ identiques ou différents,
un groupe phényle ou benzyle portant chacun éventuellement un ou plusieurs substituants halogéno, nitro, cyano, alkyle en C₁ à C₆, alkoxy en C₁ à C₆, halogénalkoxy en C₁ à C₃ ou halogénalkyle en C₁ à C₃ identiques ou différents,
R³ est un groupe alkyle en C₁ à C₁₂ éventuellement substitué une ou plusieurs fois identiques ou différentes par un halogène, ou un groupe phényle ou phényl-(alkyle en C₁ à C₄) portant chacun, le cas échéant, un ou plusieurs substituants, identiques ou différents, halogéno, alkyle en C₁ à C₆, alkoxy en C₁ à C₆, halogénalkyle en C₁ à C₃, halogénalkoxy en C₁ à C₃, cyano ou nitro,
R⁴ et R⁵ représentent, indépendamment l'un de l'autre, un groupe alkyle en C₁ à C₈, alkoxy en C₁ à C₈, alkylamino en C₁ à C₈, di-(alkyle en C₁ à C₈)-amino, alkylthio en C₁ à C₈, alcénylthio en C₃ à C₅, cycloalkylthio en C₃ à C₇ portant chacun, le cas échéant, un ou plusieurs substituants halogéno identiques ou différents, ou un groupe phényle, phénoxy ou phénylthio portant chacun, le cas échéant, un ou plusieurs substituants, identiques ou différents, halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄,
R⁶ représente l'hydrogène, un groupe alkyle en C₁ à C₁₀, alcényle en C₃ à C₈, (alkoxy en C₁ à C₈)-(alkyle en C₂ à C₈) portant chacun, le cas échéant, un ou plusieurs substituants halogéno identiques ou différents, un groupe cycloalkyle en C₃ à C₁₀ portant éventuellement un ou plusieurs substituants halogéno, alkyle en C₁ à C₆, alkoxy en C₁ à C₆, halogénalkyle en C₁ à C₃ ou halogénalkoxy en C₁ à C₃ identiques ou différents, un groupe phényle éventuellement substitué une ou plusieurs fois identiques ou différentes par un radical halogéno, halogénalkyle en C₁ à C₃, alkyle en C₁ à C₈, halogénalkoxy en C₁ à C₃ ou alkoxy en C₁ à C₈ ou un groupe benzyle portant éventuellement un ou plusieurs substituants, identiques ou différents, halogéno, alkyle en C₁ à C₈, halogénalkyle en C₁ à C₃, halogénalkoxy en C₁ à C₃ ou alkoxy en C₁ à C₈,
R⁷ représente l'hydrogène ou un groupe alkyle en C₁ à C₁₀ ou alcényle en C₃ à C₁₀ portant éventuellement dans chaque cas un ou plusieurs substituants halogéno identiques ou différents, ou bien
R⁶ et R⁷ forment conjointement avec l'atome d'azote auquel ils sont liés un noyau de 3 à 7 atomes contenant éventuellement de l'oxygène ou du soufre et éventuellement substitué par un radical alkyle en C₁ à C₆,
R⁸ et R⁹ représentent, indépendamment l'un de l'autre, l'hydrogène, un groupe alkyle en C₁ à C₆ éventuellement substitué une ou plusieurs fois identiques ou différentes par un halogène, ou représentent un groupe phényle ou phényl-(alkyle en C₁ à C₄) dont chacun porte éventuellement un ou plusieurs substituants halogéno, alkyle en C₁ à C₆, alkoxy en C₁ à C₆, halogénalkyle en C₁ à C₆, halogénalkoxy en C₁ à C₆, nitro ou cyano identiques ou différents,
ou forment ensemble un groupe alcanediyle en C₂ à C₆ éventuellement substitué une ou plusieurs fois identiques ou différentes par un radical halogéno, alkyle en C₁ à C₆, alkoxy en C₁ à C₆ ou halogénalkyle en C₁ à C₃ et
R¹⁰ et R¹¹ représentent, indépendamment l'un de l'autre, un groupe alkyle en C₁ à C₁₀, alcényle en C₂ à C₁₀, alkoxy en C₁ à C₁₀, alkylamino en C₁ à C₁₀, di-(alkyle en C₁ à C₁₀)-amino, alcénylamino en C₃ à C₁₀, di-(alcényle en C₃ à C₁₀)-amino portant chacun, le cas échéant, un ou plusieurs substituants halogéno identiques ou différents, ou un groupe phényle ou benzyle portant chacun, le cas échéant, un ou plusieurs substituants halogéno, alkyle en C₁ à C₄, alkoxy en C₁ à C₄, halogénalkyle en C₁ à C₄, nitro ou cyano identiques ou différents.

2. Composés de formule (I) suivant la revendication 1, dans lesquels
X représente le fluor, le chlore, le brome, un groupe alkyle en C₁ à C₄, alcényle en C₂ à C₄, alkoxy en C₁ à C₄, alcényloxy en C₂ à C₄, alkylthio en C₁ à C₄, alkylsulfinyle en C₁ à C₄, alkylsulfonyle en C₁ à C₄, halogénalkyle en C₁ à C₄, halogénalkoxy en C₁ à C₄, halogénalcényloxy en C₂ à C₄, nitro, cyano, ou un groupe phényle, phénoxy, phénylthio, benzyloxy ou benzylthio portant chacun, le cas échéant, un substituant fluoro, chloro, bromo, alkyle en C₁ à C₄, alkoxy en C₁ à C₄, halogénalkyle en C₁ ou C₂, halogénalkoxy en C₁ ou C₂, nitro ou cyano,
Y reprsente l'hydrogène, le fluor, le chlore, le brome, un groupe alkyle en C₁ à C₄, alcényle en C₂ à C₄, alkoxy en C₁ à C₄, alcényloxy en C₂ à C₄, alkylthio en C₁ à C₄, alkylsulfinyle en C₁ à C , alkylsulfonyle en C₁ à C₄, halogénalkyle en C₁ à C₄, halogénalkoxy en C₁ à C₄, halogénalcényloxy en C₂ à C₄, nitro ou cyano,
Z représente le fluor, le chlore, le brome, un groupe alkyle en C₁ à C₄, alcényle en C₂ à C₄, alkoxy en C₁ à C₄, alcényloxy en C₂ à C₄, halogénalkyle en C₁ à C₄, halogénalkoxy en C₁ à C₄, halogénalcényloxy en C₂ à C₄, nitro ou cyano,
X, Y et Z ne représentant pas simultanément un qroupe méthyle,
A et Q forment ensemble un groupe alcanediyle en C₁ à C₅ ou alcènediyle en C₂ à C₅ portant chacun, le cas échéant, un ou deux substituants, identiques ou différents, fluoro, chloro, bromo, hydroxy, mercapto, alkyle en C₁ à C₈, alkoxy en C₁ à C₄, alkylthio en C₁ à C₄, cycloalkyle en C₅ à C₇ ou phényle portant chacun, le cas échéant, un à cinq substituants fluoro ou chloro identiques ou différents, qui contient en outre, le cas échéant, l'un des groupements suivants 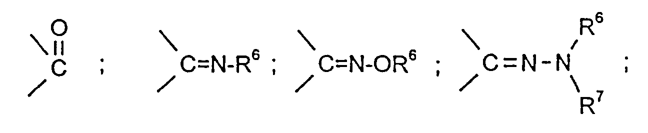 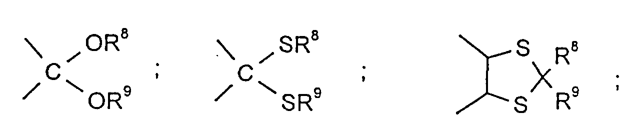 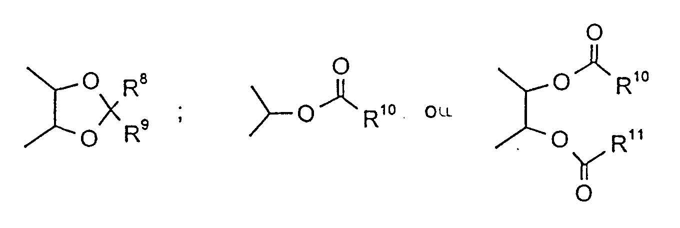 ou est ponté par un groupe alcanediyle en C₁ ou C₂,
B et B' représentent, indépendamment l'un de l'autre, l'hydrogène, le fluor, le chlore ou un groupe alkyle en C₁ à C₄ ou forment ensemble un groupe alcanediyle en C₁ à C₅ ou alcènediyle en C₂ à C₄ éventuellement substitué chacun par un radical alkyle en C₁ à C₄,
G représente l'hydrogène (a) ou l'un des groupes 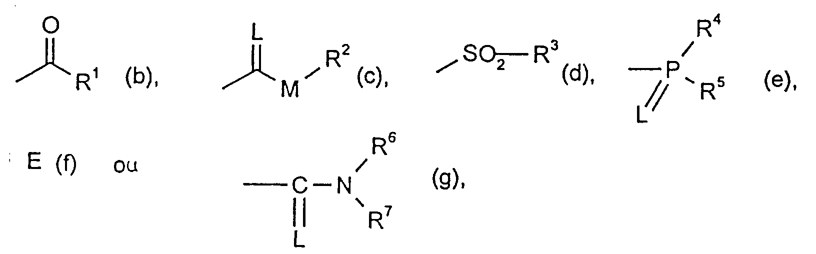 dans lesquels
E représente un ion de métal ou un ion ammonium,
L est l'oxygène ou le soufre et
M est l'oxygène ou le soufre,
R¹ représente un groupe alkyle en C₁ à C₁₆, alcényle en C₂ à C₈, (alkoxy en C₁ à C₆) - (alkyle en C₁ à C₆), (alkylthio en C₁ à C₆)-(alkyle en C₁ à C₆) , poly(alkoxy en C₁ à C₆)-(alkyle en C₁ à C₆) portant chacun, le cas échéant, un à neuf substituants fluoro ou chloro identiques ou différents, ou un groupe cycloalkyle en C₃ à C₇ éventuellement substitué une à trois fois identiques ou différentes par un radical fluoro, chloro, alkyle en C₁ à C₄ ou alkoxy en C₁ à C₄, dans lequel un ou deux groupes méthylène non contigus peuvent être remplacés par des atomes d'oxygène et/ou de soufre,
un groupe phényle éventuellement substitué une à trois fois identiques ou différentes par un radical fluoro, chloro, bromo, cyano, nitro, alkyle en C₁ à C₄, alkoxy en C₁ à C₄, halogénalkyle en C₁ à C₃ ou halogénalkoxy en C₁ à C₃,
un groupe phényl-(alkyle en C₁ à C₄) portant éventuellement un à trois substituants fluoro, chloro, bromo, alkyle en C₁ à C₄, alkoxy en C₁ à C₄, halogénalkyle en C₁ à C₃ ou halogénalkoxy en C₁ à C₃ identiques ou différents,
un groupe furannyle, thiényle, pyridyle, pyrimidyle, thiazolyle ou pyrazolyle portant chacun, le cas échéant, un ou deux substituants fluoro, chloro, bromo ou alkyle en C₁ à C₄ identiques ou différents,
un groupe phénoxy-(alkyle en C₁ à C₅) portant éventuellement un à trois substituants fluoro, chloro ou alkyle en C₁ à C₄ identiques ou différents ou bien
un groupe pyridyloxy-(alkyle en C₁ à C₆),pyrimidinyloxy-(alkyle en C₁ à C₆) ou thiazolyloxy-(alkyle en C₁ à C₆) portant chacun, le cas échéant, un ou deux substituants fluoro, chloro, amino ou alkyle en C₁ à C₄ identiques ou différents,
R² représente un groupe alkyle en C₁ à C₁₆, alcényle en C₂ à C₈, (alkoxy en C₁ à C₆) - (alkyle en C₂ à C₆) ou poly(alkoxy en C₁ à C₆)-(alkyle en C₂ à C₆) portant chacun, le cas échant, un à sept substituants fluoro ou chloro identiques ou différents,
un groupe cycloalkyle en C₃ à C₆ portant éventuellement un à trois substituants fluoro, chloro, alkyle en C₁ à C₄ ou alkoxy en C₁ à C₄ identiques ou différents,
un groupe phényle ou benzyle portant chacun, le cas échéant, un à trois substituants fluoro, chloro, bromo, nitro, cyano, alkyle en C₁ à C₄, alkoxy en C₁ à C₄, halogénalkoxy en C₁ ou C₂ ou halogénalkyle en C₁ ou C₂ identiques ou différents,
R³ représente un groupe alkyle en C₁ à C₉ portant éventuellement un à cinq substituants fluoro ou chloro identiques ou différents ou un groupe phényle ou phényl-(alkyle en C₁ ou C₂) portant chacun, le cas échéant, un à trois substituants fluoro, chloro, bromo, alkyle en C₁ à C₄, alkoxy en C₁ à C₄, halogénalkyle en C₁ ou C₂, halogénalkoxy en C₁ ou C₂, cyano ou nitro identiques ou différents,
R⁴ et R⁵ représentent, indépendamment l'un de l'autre, un groupe, portant éventuellement un à cinq substituants fluoro ou chloro identiques ou différents, alkyle en C₁ à C₆, alkoxy en C₁ à C₆, alkylamino en C₁ à C₆, di-(alkyle en C₁ à C₆)-amino, alkylthio en C₁ à C₆, alcénylthio en C₃ ou C₄, cycloalkylthio en C₃ à C₆ ou un groupe phényle, phénoxy ou phénylthio portant chacun, le cas échéant, un à trois substituants fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃ identiques ou différents,
R⁶ représente l'hydrogène, un groupe alkyle en C₁ à C₈, alcényle en C₃ à C₆, (alkoxy en C₁ à C₆) - (alkyle en C₂ à C₆) portant chacun, le cas échéant, un à cinq substituants fluoro ou chloro identiques ou différents, un groupe cycloalkyle en C₃ à C₈ éventuellement substitué une à trois fois identiques ou différentes par un radical fluoro, chloro, alkyle en C₁ à C₄, alkoxy en C₁ à C₄, haogénalkyle en C₁ ou C₂ ou halogénalkoxy en C₁ ou C₂, un groupe phényle portant éventuellement un à trois substituants fluoro, chloro, bromo, halogénalkyle en C₁ ou C₂, alkyle en C₁ à C₅, halogénalkoxy en C₁ ou C₂ ou alkoxy en C₁ à C₅ identiques ou différents ou un groupe benzyle portant éventuellement un à trois substituants fluoro, chloro, bromo, alkyle en C₁ à C₅, halogénalkyle en C₁ ou C₂, halogénalkoxy en C₁ ou C₂ ou alkoxy en C₁ à C₅ identiques ou différents,
R⁷ représente l'hydrogène ou un groupe alkyle en C₁ à C₈ ou alcényle en C₃ à C₈ portant chacun, le cas échéant, un à cinq substituants fluoro ou chloro identiques ou différents,
R⁶ et R⁷ forment conjointement avec l'atome d'azote auquel ils sont liés un noyau de 4 à 7 atomes contenant éventuellement de l'oxygène ou du soufre et éventuellement substitué par un radical alkyle en C₁ à C₄,
R⁸ et R⁹ représentent, indépendamment l'un de l'autre, l'hydrogène, un groupe alkyle en C₁ à C₄ éventuellement substitué une à cinq fois identiques ou différentes par du fluor ou du chlore, ou un groupe phényle portant éventuellement un à trois substituants fluoro, chloro, alkyle en C₁ à C₄, alkoxy en C₁ à C₄, halogénalkyle en C₁ ou C₂, halogénalkoxy en C₁ ou C₂, nitro ou cyano identiques ou différents, ou forment ensemble un groupe alcanediyle en C₂ à C₅ portant éventuellement un à trois substituants fluoro, chloro, alkyle en C₁ à C₄, alkoxy en C₁ à C₄ ou halogénalkyle en C₁ ou C₂ identiques ou différents et
R¹⁰ et R¹¹ représentent, indépendament l'un de l'autre, un groupe alkyle en C₁ à C₈, alcényle en C₂ à C₈, alkoxy en C₁ à C₈, alkylamino en C₁ à C₈, alcénylamino en C₃ à C₈, di-(alkyle en C₁ à C₈)-amino ou di-(alcényle en C₃ à C₈)-amino portant chacun, le cas échéant, un à cinq substituants fluoro ou chloro identiques ou différents.

3. Composés de formule (I) suivant la revendication 1, dans lesquels
X représente le fluor, le chlore, le brome, un groupe méthyle, éthyle, propyle, isopropyle, éthényle, 1-propényle, méthoxy, éthoxy, propoxy, isopropoxy, allyloxy, méthallyloxy, trifluorométhyle, difluorométhoxy, trifluorométhoxy, trifluoréthoxy, méthylthio, méthylsulfinyle, méthylsulfonyle, nitro, cyano, ou un groupe phényle, phénoxy, phénylthio, benzyloxy ou benzylthio portant éventuellement dans chaque cas un substituant fluoro, chloro, bromo, méthyle, éthyle, propyle, isopropyle, tertio-butyle, méthoxy, éthoxy,
L est l'oxygène ou le soufre et
M est l'oxygène ou le soufre,
R¹ représente un groupe alkyle en C₁ à C₁₄, alcényle en C₂ à C₆, (alkoxy en C₁ à C₄) - (alkyle en C₁ à C₆), (alkylthio en C₁ à C₄)-(alkyle en C₁ à C₆), poly(alkoxy en C₁ à C₄)-(alkyle en C₁ à C₄) portant chacun, le cas échéant, un à cinq substituants fluoro ou chloro identiques ou différents ou un groupe cycloalkyle en C₃ à C₆ portant éventuellement un ou deux substituants fluoro, chloro, méthyle, éthyle, méthoxy ou éthoxy identiques ou différents, dans lequel un ou deux groupes méthylène non contigus peuvent être remplacés par des atomes d'oxygène et/ou de soufre,
un groupe phényle portant éventuellement un ou deux substituants fluoro, chloro, bromo, méthyle, éthyle, propyle, isopropyle, méthoxy, éthoxy, trifluorométhyle, trifluorométhoxy, cyano ou nitro identiques ou différents,
un groupe benzyle portant éventuellement un ou deux substituants fluoro, chloro, bromo, méthyle, éthyle, propyle, isopropyle, méthoxy, éthoxy, trifluorométhyle ou trifluorométhoxy identiques ou différents,
un groupe thiényle, furannyle ou pyridyle portant chacun, le cas échéant, un ou deux substituants fluoro, chloro, bromo, méthyle ou éthyle identiques ou différents,
un groupe phénoxy-(alkyle en C₁ à C₄) portant, le cas échéant, un ou deux substituants fluoro, chloro, méthyle ou éthyle identiques ou différents, ou bien
un groupe pyridyloxy-(alkyle en C₁ à C₄), pyrimidyloxy-(alkyle en C₁ à C₄) ou thiazolyloxy-(alkyle en C₁ à C₄) portant chacun, le cas échéant, un ou deux substituants fluoro, chloro, amino, méthyle ou éthyle identiques ou différents,
R² représente un groupe alkyle en C₁ à C₁₄, alcényle en C₂ à C₆, (alkoxy en C₁ à C₄) - (alkyle en C₂ à C₆) ou poly(alkoxy en C₁ à C₄)-(alkyle en C₂ à C₆) portant chacun, le cas échéant, un à cinq substituants fluoro ou chloro identiques ou différents, un groupe cycloalkyle en C₃ à C₆ portant éventuellement un à trois substituants fluoro, chloro, méthyle, éthyle, méthoxy ou éthoxy identiques ou différents,
ou un groupe phényle ou benzyle portant chacun, le cas échéant, un ou deux substituants fluoro, chloro, nitro, cyano, méthyle, éthyle, propyle, isopropyle, méthoxy, éthoxy, trifluorométhoxy ou trifluorométhyle identiques ou différents,
R³ représente un groupe alkyle en C₁ à C₆ portant éventuellement un à trois substituants fluoro ou chloro identiques ou différents ou un groupe phényle ou benzyle portant chacun, le cas échéant, un ou deux substituants fluoro, chloro, bromo, méthyle, éthyle, méthoxy, éthoxy, trifluorométhyle, trifluorométhoxy, cyano ou nitro,
R⁴ et R⁵ représentent, indépendamment l'un de l'autre, un groupe alkyle en C₁ à C₄, alkoxy en C₁ à C₄, alkylamino en C₁ à C₄, di-(alkyle en C₁ à C₄)-amino, alkylthio en C₁ à C₄ portant chacun, le cas échéant, un à trois substituants fluoro ou chloro identiques ou différents ou un groupe phényle, phénoxy ou phénylthio portant chacun, le cas échéant, un ou deux substituants fluoro, chloro, bromo, nitro, cyano, alkoxy en C₁ ou C₂, trifluorométhoxy, alkylthio en C₁ ou C₂, trifluorométhyle ou alkyle en C₁ à C₃ identiques ou différents,
R⁶ représente l'hydrogène, un groupe alkyle en C₁ à C₆, (alkoxy en C₁ à C₆)-(alkyle en C₂ à C₄) portant chacun, le cas échéant, un à trois substituants fluoro ou chloro identiques ou différents, un groupe cycloalkyle en C₃ à C₆ portant, le cas échéant, un ou deux substituants fluoro, chloro, méthyle, méthoxy, trifluorométhyle ou trifluorométhoxy identiques ou différents, un groupe phényle portant, le cas échéant, un ou deux substituants fluoro, chloro, trifluorométhyle, alkyle en C₁ à C₄, trifluorométhoxy ou alkoxy en C₁ à C₄ identiques ou différents ou un groupe benzyle portant, le cas échéant, un ou deux substituants fluoro, chloro, alkyle en C₁ à C₄, trifluorométhyle, trifluorométhoxy ou alkoxy en C₁ à C₄ identiques ou différents,
R⁷ représente l'hydrogène, un groupe alkyle en C₁ à C₆ ou alcényle en C₃ à C₆ portant chacun, le cas échéant, un à trois substituants fluoro ou chloro identiques ou différents, ou bien
R⁶ et R⁷ forment conjointement avec l'atome d'azote auquel ils sont liés un noyau pentagonal à heptagonal contenant éventuellement de l'oxygène ou du soufre et éventuellement substitué par un radical méthyle.

4. Procédé de production de composés de formule (I) suivant la revendication 1, **caractérisé en ce que**
(A) on obtient des composés de formule (Ia) 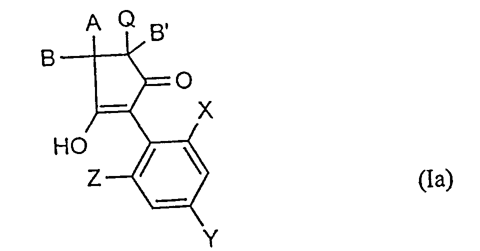 dans laquelle
A, B, B', Q, X, Y et Z ont la définition indiquée dans la revendication 1,
en effectuant la condensation intramoléculaire de composés de formule (II) 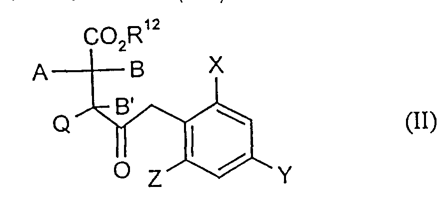 dans laquelle
A, B, B', Q, X, Y et Z ont la définition indiquée ci-dessus, et
R¹² est un groupe alkyle,
en présence d'un diluant et en présence d'une base ;
(B) on obtient des composés de formule (Ib) 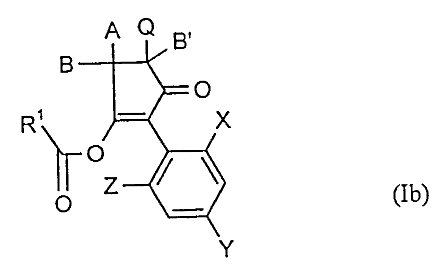 dans laquelle
A, B, B', Q, X, Y, Z et R¹ ont la définition indiquée dans la revendication 1,
en faisant réagir des composés de formule (Ia) 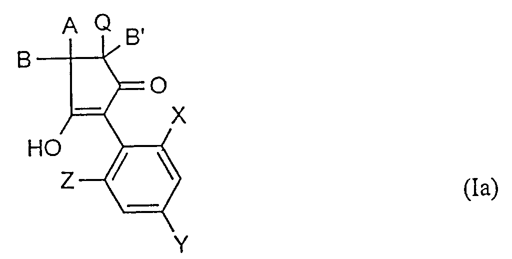 dans laquelle
A, B, B', X, Y, Z et Q ont la définition indiquée ci-dessus,
α) avec des halogénures d'acides de formule (III) 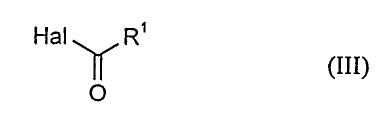 dans laquelle
R¹ a la définition indiquée ci-dessus et
Hal représente un halogène,
le cas échéant en présence d'un diluant et en présence éventuelle d'un accepteur d'acide,
ou bien
β) avec des anhydrides d'acides carboxyliques de formule (IV)
R¹-CO-O-CO-R¹ (IV)
dans laquelle
R¹ a la définition indiquée ci-dessus,
le cas échéant en présence d'un diluant et en présence éventuelle d'un accepteur d'acide ;
(C) on obtient des composés de formule (Ic-1) 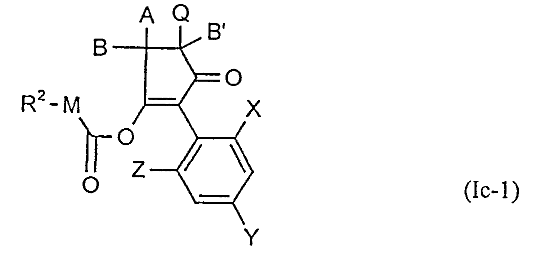 dans laquelle
A, B, B', Q, X, Y, Z et R² ont la définition indiquée dans la revendication 1,
et
M représente l'oxygène ou le soufre,
en faisant réagir des composés de formule (Ia) 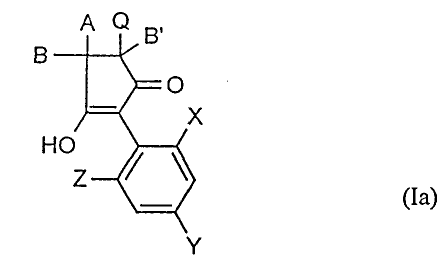 dans laquelle
A, B, B', Q, X, Y et Z ont la définition indiquée ci-dessus,
avec des esters d'acide chloroformique ou des thioesters d'acide chloroformique de formule (V)
R²-M-CO-Cl (V)
dans laquelle
R² et M ont la définition indiquée ci-dessus,
le cas échéant en présence d'un diluant et en présence éventuelle d'un accepteur d'acide ;
(D) on obtient des composés de formule (Ic-2) 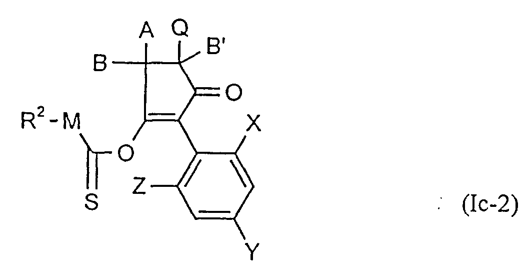 dans laquelle
A, B, B', Q, X, Y, Z et R² ont la définition indiquée dans la revendication 1,
et
M représente l'oxygène ou le soufre,
en faisant réagir des composés de formule (Ia) 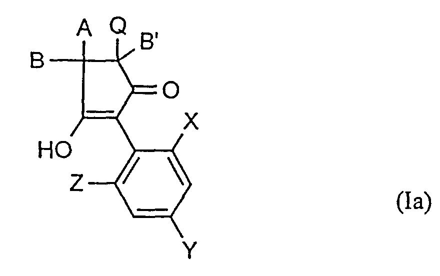 dans laquelle
A, B, B', Q, X, Y et Z ont la définition indiquée ci-dessus,
α) avec des esters d'acide chloromonothioformique ou des esters d'acide chlorodithioformique de formule (VI) 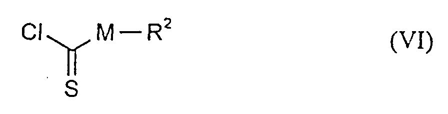 dans laquelle
M et R² ont la définition indiquée ci-dessus,
le cas échéant en présence d'un diluant et en présence éventuelle d'un accepteur d'acide,
ou bien
β) avec le sulfure de carbone, puis avec des halogénures d'alkyle de formule générale (VII)
R²-Hal (VII)
dans laquelle
R² a la définition indiquée ci-dessus
et
Hal représente le chlore, le brome ou l'iode,
le cas échéant en présence d'un diluant et en présence éventuelle d'une base ;
(E) on obtient des composés de formule (Id) 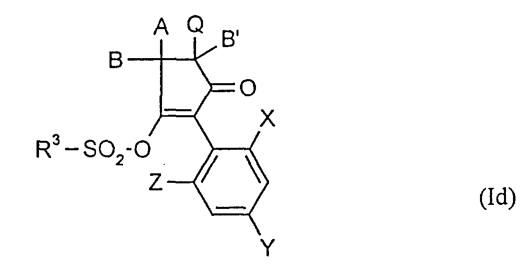 dans laquelle
A, B, B', Q, X, Y, Z et R³ ont la définition indiquée dans la revendication 1,
en faisant réagir des composés de formule (Ia) 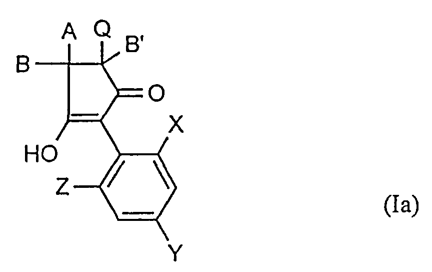 dans laquelle
A, B, B', Q, X, Y et Z ont la définition indiquée ci-dessus,
avec des chlorures d'acide sulfonique de formule (VIII)
R³-SO₂-Cl (VIII)
dans laquelle
R³ a la définition indiquée ci-dessus,
le cas échéant en présence d'un diluant et en présence éventuelle d'un accepteur d'acide ;
(F) on obtient des composés de formule (Ie) 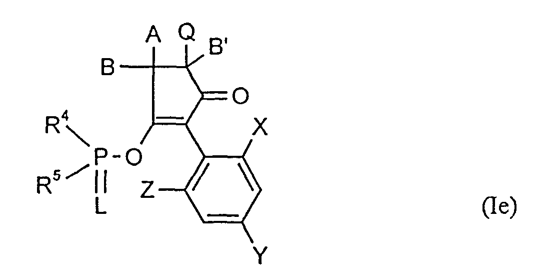 dans laquelle
A, B, L, B', Q, X, Y, Z, R⁴ et R⁵ ont la définition indiquée ci-dessus,
en faisant réagir des composés de formule (Ia) ou leurs énols 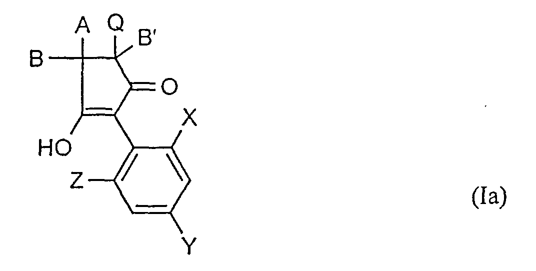 formule dans laquelle
A, B, B', Q, X, Y et Z ont la définition indiquée ci-dessus,
avec des composés de phosphore de formule (IX) 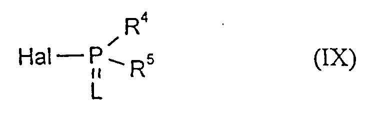 dans laquelle
L, R⁴ et R⁵ ont la définition indiquée ci-dessus
et
Hal représente un halogène,
le cas échéant en présence d'un diluant et en présence éventuelle d'un accepteur d'acide ;
(G) on obtient des composés de formule (If) 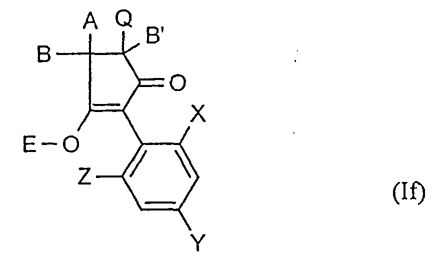 dans laquelle
A, B, B', Q, X, Y et Z ont la définition indiquée dans la revendication 1,
et
E représente un équivalent d'ion de métal ou un ion ammonium,
en faisant réagir des composés de formule (Ia) 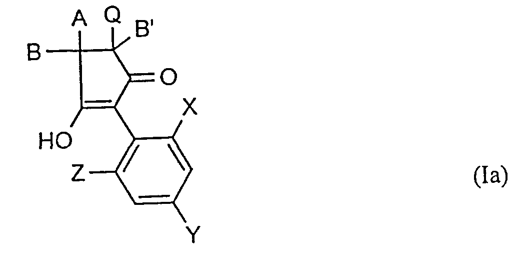 dans laquelle
A, B, B', Q, X, Y et Z ont la définition indiquée ci-dessus,
avec des composés métalliques de formule (X) ou des amines de formule (XI)
Me Rₜ ¹⁶ (X) 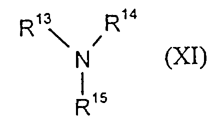
formules dans lesquelles
Me représente un ion de métal monovalent ou divalent,
t représente le nombre 1 ou 2,
R¹³, R¹⁴ et _{R}¹⁵ représentent, indépendamment l'un de l'autre, l'hydrogène ou un groupe alkyle et
R¹⁶ représente l'hydrogène, un groupe hydroxy ou un groupe alkoxy en C₁ à C₄,
le cas échéant en présence d'un diluant ;
(H) on obtient des composés de formule (Ig) 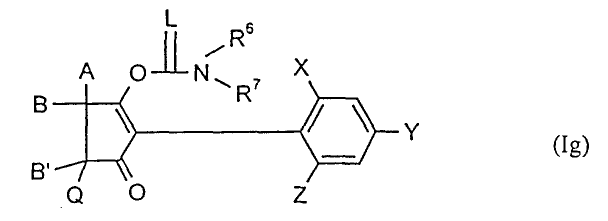 dans laquelle
A, B, L, B', Q, X, Y, Z, R⁶ et R⁷ ont la définition indiquée dans la revendication 1,
en faisant réagir des composés de formule (Ia) 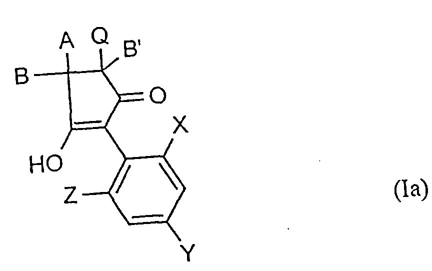 dans laquelle
A, B, B', Q, X, Y et Z ont la définition indiquée ci-dessus,
α) avec des composés de formule (XII)
R⁶-N=C=L (XII)
dans laquelle
L et R⁶ ont la définition indiquée ci-dessus,
le cas échéant en présence d'un diluant et en présence éventuelle d'un catalyseur
ou bien
β) avec des chlorures d'acide carbamique ou des chlorures d'acide thiocarbamique de formule (XIII) 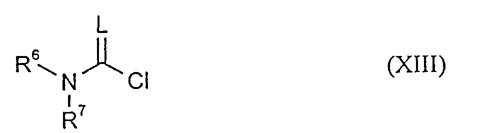 dans laquelle
L, R⁶ et R⁷ ont la définition indiquée ci-dessus,
le cas échéant en présence d'un diluant et en présence éventuelle d'un accepteur d'acide.

5. Composés de formule (II) 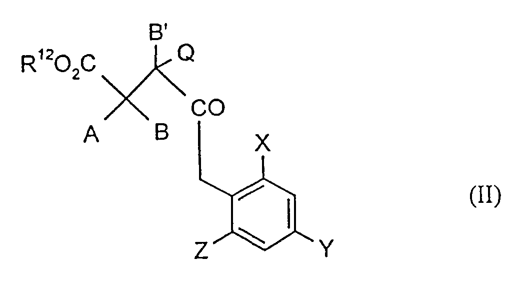 dans laquelle
A, B, B', Q, X, Y et Z ont la définition indiquée dans la revendication 1
et
R¹² représente un groupe alkyle.

6. Composés de formule (XIV) 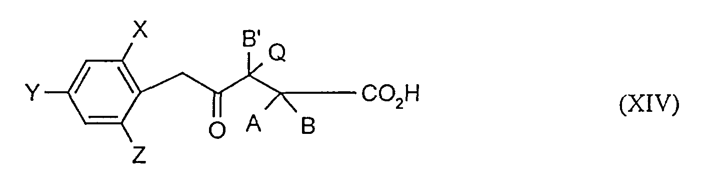 dans laquelle
A, B, B', Q, X, Y et Z ont la définition indiquée dans la revendication 1.

7. Composés de formule (XVII) 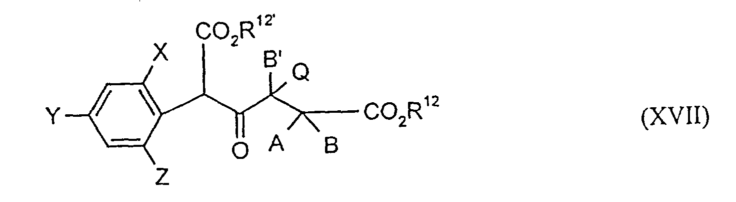 dans laquelle
A, B, B', Q, X, Y et Z ont la définition indiquée dans la revendication 1
et
R¹² et R^{12'} représentent un groupe alkyle.

8. Compositions pesticides et compositions herbicides, **caractérisées par** une teneur en au moins un composé de formule (I) suivant la revendication 1.

9. Utilisation de composés de formule (I) suivant la revendication 1 pour combattre des parasites et des mauvaises herbes.

10. Procédé pour combattre des parasites et des mauvaises herbes, **caractérisé en ce qu'**on fait agir des composés de formule (I) suivant la revendication 1 sur les parasites, les mauvaises herbes et/ou leur milieu.

11. Procédé de préparation de compositions pesticides et de compositions herbicides, **caractérisé en ce qu'**on mélange des composés de formule (I) suivant la revendication 1 avec des diluants et/ou des agents tensio-actifs.

12. Utilisation de composés de formule (I) suivant la revendication 1, pour la préparation de compositions pesticides et de compositions herbicides.
